# EUROPEAN PATENT SPECIFICATION

(11) **EP 2 007 729 B1**
(45) Date of publication and mention of the grant of the patent: **27.06.2012**
(21) Application number: 07723520.8
(22) Date of filing: 23.03.2007
(51) Int. Cl.: C07D 217/04, C07D 217/06, C07D 217/10, C07D 401/12, C07D 403/12, C07D 405/12, C07D 409/12, C07D 413/12, C07D 417/12, C07D 513/04, A61K 31/4725, A61P 3/00

(54) **SUBSTITUTED TETRAHYDROISOQUINOLINE COMPOUNDS, THEIR PREPARATION AND USE IN MEDICAMENTS**
SUBSTITUIERTE TETRAHYDROISOCHINOLINVERBINDUNGEN, IHRE HERSTELLUNG UND VERWENDUNG IN MEDIKAMENTEN
COMPOSÉS DE TÉTRAHYDROISOQUINOLÉINE SUBSTITUÉS, LEUR PRÉPARATION ET LEUR UTILISATION DANS DES MÉDICAMENTS

(30) Priority: 23.03.2006 EP 06380059
(43) Date of publication of application: 31.12.2008
(73) Proprietor: LABORATORIOS DEL DR. ESTEVE, S.A., 08026 Barcelona (ES)
(72) Inventor: TORRENS, JOVER, Antonio, E-08221-Terrassa, Barcelona (ES); MAS, PRIO, Josep, E-08191-Rubi, Barcelona (ES); PORT, CASAMITJANA, Adriana, E-08202-Sabadell Barcelona (ES); BUSCHMANN, Helmut, Heinrich, E-08960-Sant Just Desvern, Barcelona (ES)
(74) Representative: Carpintero Lopez, Francisco
(86) International application number: PCT/EP2007/002569
(87) International publication number: WO 2007/107373

(56) References cited:
- WO-A-01/32646
- WO-A-02/100822
- WO-A-03/068752
- WO-A-03/095428
- WO-A-2004/031181
- WO-A-2005/025576
- DE-A1- 10 053 799
- HOLENZ J ET AL: "Medicinal Chemistry Driven Approaches Toward Novel and Selective Serotonin 5-HT6 Receptor Ligands" JOURNAL OF MEDICINAL CHEMISTRY, AMERICAN CHEMICAL SOCIETY. WASHINGTON, US, vol. 48, no. 6, 2005, pages 1781-1795, XP002993846 ISSN: 0022-2623
- HOLENZ ET AL: "Medicinal chemistry strategies to 5-HT6 receptor ligands as potential cognitive enhancers and antiobesity agents" DRUG DISCOVERY TODAY, ELSEVIER, RAHWAY, NJ, US, vol. 11, no. 7-8, April 2006 (2006-04), pages 283-299, XP005362935 ISSN: 1359-6446

## Description

The present invention relates to substituted tetrahydroisoquinoline compounds of general formula I, 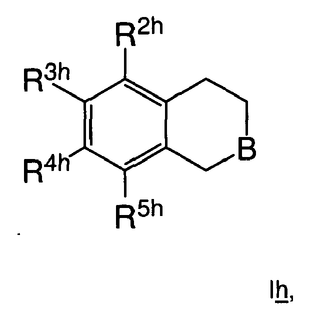 a process for their preparation, medicaments comprising said substituted tetrahydroisoquinoline compounds as well as the use of said substituted tetrahydroisoquinoline compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125, 1562; D.C. Rogers et al., Br. J. Pharmacol. Suppl., 1999, 127, 22P; A. Bourson et al., J. Pharmacol. Exp. Ther. , 1995, 274, 173; A.J. Sleight, et al., Behav. Brain Res. , 1996, 73, 245; T.A. Branchek et al., Annu. Rev. Pharmacol. Toxicol., 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther. , 1994, 268, 1403; C.E. Glatt et al., Mol. Med. 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gérard et al., Brain Research , 1997, 746, 207; M.R. Pranzatelli, Drugs of Today , 1997, 33, 379].

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

WO03095428, W003068752, W02004031181 and WO2005025576 are all related applications disclosing inter alia tetrahydrosioquinoline showing antipsychotic activity. However, in general terms these compounds are far removed from the ones of the present invention.

WO0132646 and W002100822 teaches some quinolines having affinity for 5HT6 receptors.

Some 1-(1-hydrosulfonylpiperidin-4-yl)-2,4-dihydro-1H-benzo[d][1,3]oxazines and indole sulphonamide were tested as good 5HT6 ligands by Holenz et al (J. Med. Chem. Am. Med. Soc., 2005, 48(6): 1781-1795).

DE10053799 describe tetrahydrosioquinoline sulphonamide but having the sulphonamide group inverted with respect to the compounds of the present invention.

Surprisingly, it has been found that the substituted tetrahydroisoquinoline compounds of general formula Ih given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one of its aspects the present invention relates to a medicament comprising a substituted tetrahydroisoquinoline compounds of general formula Ih.

A substituted tetrahydroisoquinoline compound of general formula Ih, 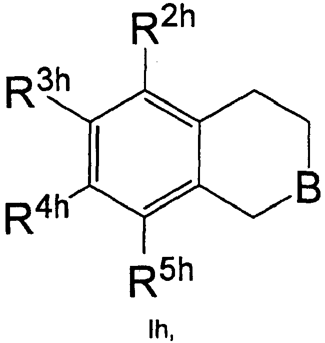 wherein
B represents a radical selected from the group consisting of 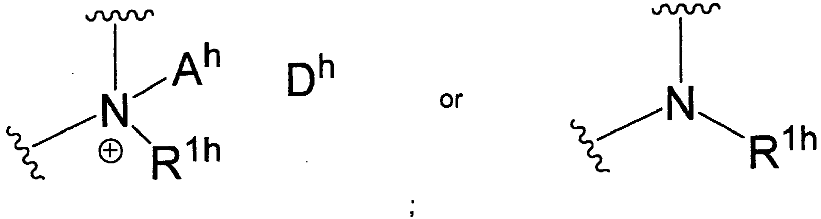
- A^{h}: represents a hydrogen atom or a radical selected from the group consisting of methyl, ethyl, n-propyl, isopropyl, n-butyl, isobutyl, 2-butyl and tert-butyl;
- D^{h}: represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate and benzenesulfonate;
- R^{1h}: represents a radical selected from the group consisting of H, methyl, ethyl, -C(=O)-cyclopropyl, -C(=O)-O-tert-butyl and -CH₂-cyclopropyl;
- R^{2h} , R^{3h}, R^{4h} and R^{5h},: independently of one another, each represents a hydrogen atom or a -N(R^{11h})-S(=O)₂-R^{12h} radical; with the proviso that at least one of the substituents R^{2h}, R^{3h}, R^{4h} and R^{5h} represents a -N(R^{11h})-S(=O)₂-R^{12h} moiety; R^{11h} represents a radical selected from the group consisting of H, methyl and ethyl;
- R^{12h}: represents a radical selected from the group consisting of 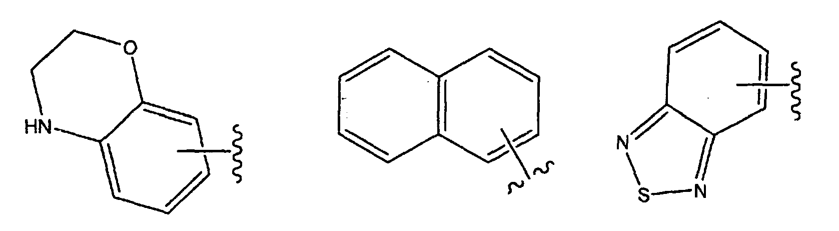 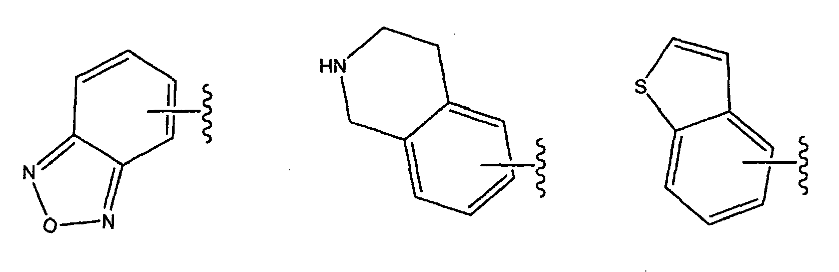 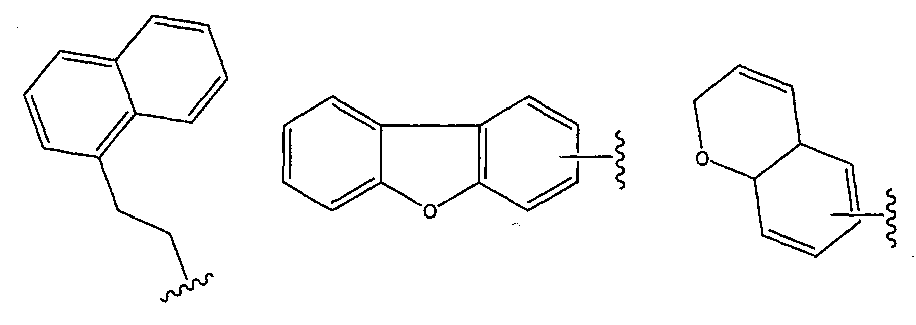 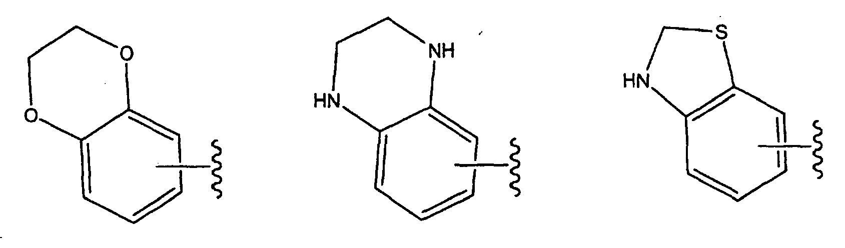 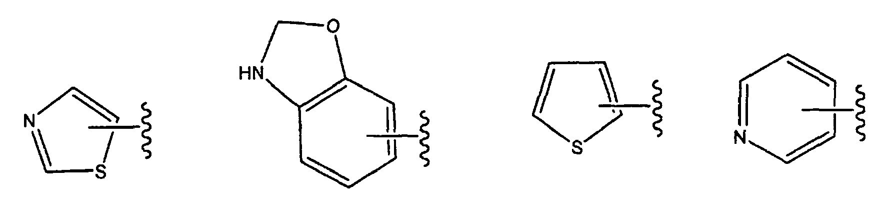 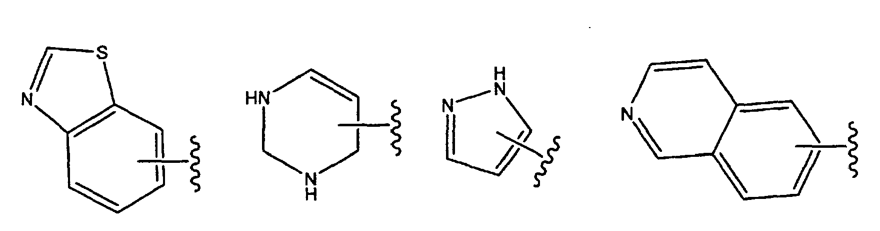 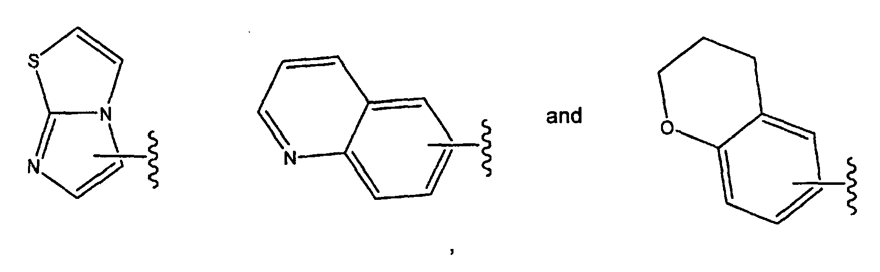
which is unsubstituted or substituted with 1, 2, 3, 4 or 5 substituents independently selected from the group consisting of H, F, Cl, methyl, ethyl, -NH₂, -N(CH₃)₂, oxo (=O), -O-CH₃, -O-CH₂-CH₃, -NH-(C=O)-CH₃, - C(=O)-O-CH₃, -C(=O)-CF₃; whereby the substitution can take place on any suitable position in the aforementioned radicals, including the heteroatom(s);
or a substituted phenyl radical selected from the group consisting of: 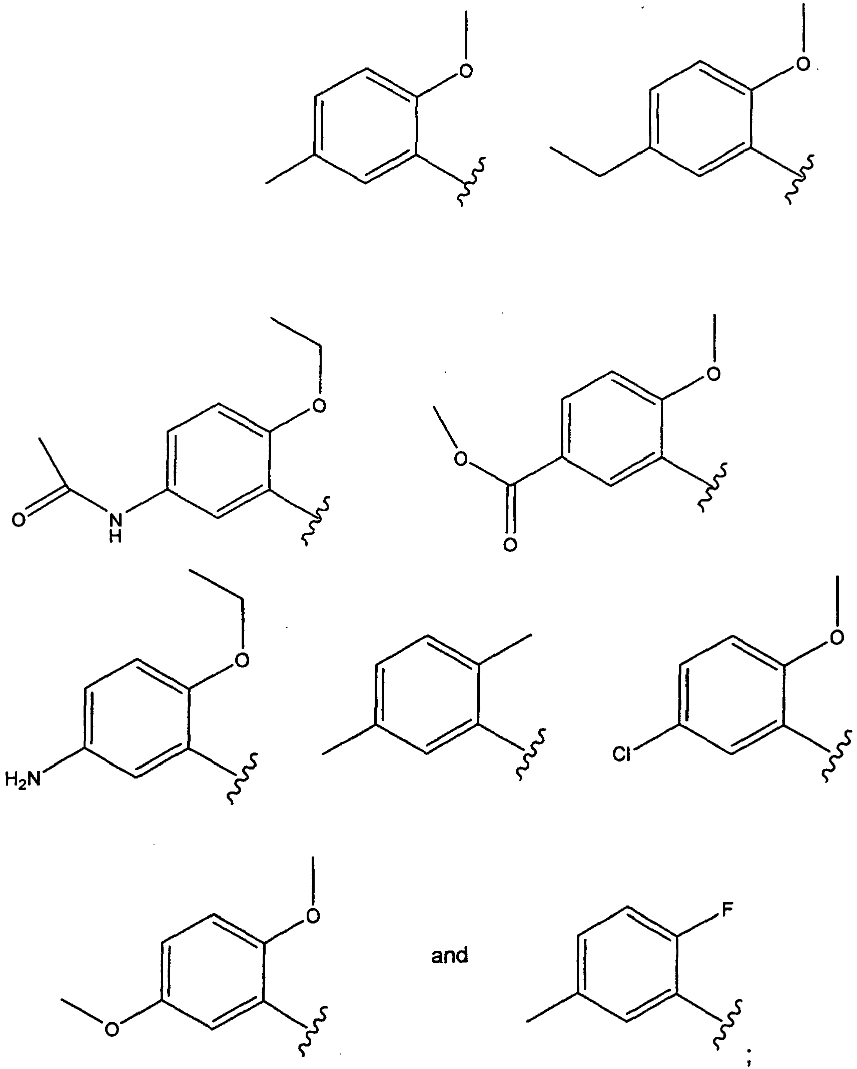 optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

A further aspect of the present invention relates to a medicament comprising at least one substituted tetrahydroisoquinoline compound of general formula Ih, Ig or Ih optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one physiologically acceptable auxiliary agent.

Said medicament is particularly suitable for 5-HT₆-receptor regulation and therefore for the prophylaxis and/or treatment of a disorder or a disease that is at least partially mediated via 5-HT₆-receptors.

Preferably said medicament is suitable for the prophylaxis and/or treatment of the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

More preferably said medicament is suitable for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

More preferably said medicament is suitable for improvement of cognition (cognitive enhancement) or cognitive memory (cognitive memory enhancement).

Most preferably, said medicament is suitable for the prophylaxis and/or treatment of obesity and/or disorders or diseases related thereto.

In another aspect the present invention relates to the use of at least one substituted tetrahydroisoquinoline compound of general formula Ih given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

In another aspect the present invention relates to the use of at least one substituted tetrahydroisoquinoline compound of general formula Ih given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

The present invention also relates to the use of at least one substituted tetrahydroisoquinoline compound of general formula Ih given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder).

The present invention also relates to the use of at least one substituted tetrahydroisoquinoline compound of general formula Ih given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

The present invention also relates to the use of at least one substituted tetrahydroisoquinoline compound of general formula Ih given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

More preferred is the use of at least one substituted tetrahydroisoquinoline compound of general formula Ih as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

More preferred is also the use of at least one substituted tetrahydroisoquinoline compound of general formula Ih as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the improvement of cognition (cognitive enhancement) and/or for the improvement of cognitive memory (cognitive memory enhancement).

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective tetrahydroisoquinoline compound is liberated in the intestinal tract. Preferably the enteric coating is soluble at a pH value of 5 to 7.5. Suitable materials and methods for the preparation are are known from the prior art.

Typically, the medicaments according to the present invention may contain 1-60 % by weight of one or more substituted tetrahydroisoquinoline compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from 1 to 2000 mg, preferably 1 to 1000 mg, more preferably 1 to 500 mg, even more preferably 1 to 200 mg, of active substance to be administered during one or several intakes per day.

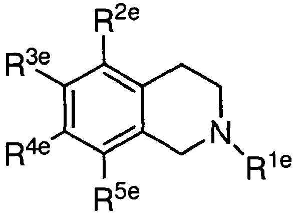

In yet another aspect the present invention relates to a substituted tetrahydroisoquinoline compound of general formula Ig, 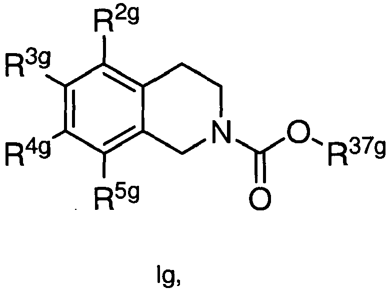 wherein
R^{1g} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and - CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a - (CH₂)_{1,2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, - C(=O)-CH₃, -C(=O)-C₂H₅, -C(=O)-CH₂-CH₂-CH₃, -C(=O)-CH(CH₃)₂, -C(=O)-C(CH₃)₃, - C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
R^{2g}, R^{3g}, R^{4g} and R^{5g}, independently of one another, each represent a hydrogen atom; F, Cl, Br, I, -NO₂; -NH₂; -SH; -OH; -CN; -C(=O)-OH; -C(=O)-H; -S(=O)₂-OH; - C(=O)-NH₂; -S(=O)₂-NH₂; -OR^{8g}; -SR^{9g}; -N(R^{11g})-S(=O)₂-R^{12g}; a radical selected from the group consisting of methyl, ethyl, n-propyl, isopropyl, n-butyl, sec-butyl, isobutyl, tert-butyl, vinyl, allyl, ethinyl, -CF₃, -CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2g}, R^{3g}, R^{4g} and R^{5g} represents a - N(R^{11g})-S(=O)₂-R^{12g} moiety;
R^{8g} and R^{9g}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a - (CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, - CF₃, -OCF₃, -SCF₃, -OH, -SH, -NH₂, -NO₂, -CHO, -CF₂H and -CFH₂;
R^{11g} represents a hydrogen atom, -S(=O)₂-R^{12g} or an alkyl radical selected from the group consisting of methyl, ethyl, n-propyl, isopropyl, n-butyl, sec-butyl, isobutyl and tert-butyl;
R^{12g} represents a phenyl radical of general formula (Ag), 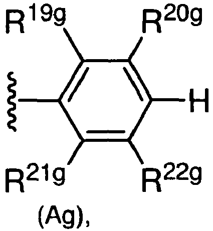 wherein
R^{19g}, R^{20g}, R^{21g} and R^{22g}, independently of one another, each represent a hydrogen atom; F, Cl, Br, I, -NO₂; -NH₂; -SH; -OH; -CN; -C(=O)-OH; -C(=O)-H; -S(=O)₂-OH; - C(=O)-NH₂; -S(=O)₂-NH₂; -C(=O)-R^{23g}; -S(=O)-R^{24g}; -S(=O)₂-R^{24g}; -OR^{25g}; -SR^{26g}; - C(=O)-OR^{27g}; methyl, ethyl, n-propyl, isopropyl, n-butyl, isobutyl, sec-butyl, tert-butyl, - CF₃, -CF₂H, -CFH₂, -CH₂-CF₃, -CF₂-CF₃, cyclopropyl, cyclobutyl, cyclopentyl and cyclohexyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, -NO₂, - CHO, -CF₂H and -CFH₂;
R^{23g} and R^{27g}, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R^{24g} and R^{26g}, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, -NH₂, -NO₂, -CHO, -CF₂H and -CFH₂;
R^{25g} represents a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and -CF₂-CF₃
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl.

Preferred is a substituted tetrahydroisoquinoline compound of general formula Ig, wherein
R^{1g} represents a hydrogen atom; or a radical selected from the group consisting of methyl, ethyl, n-propyl, n-butyl, n-pentyl and n-hexyl;
R^{2g}, R^{3g}, R^{4g} and R^{5g}, independently of one another, each represent a hydrogen atom or -N(R^{11g})-S(=O)₂-R^{12g};
with the proviso that at least one of the substituents R^{2g}, R^{3g}, R^{4g} and R^{5g} represents a - N(R^{11g})-S(=O)₂-R^{12g} moiety;
R^{11g} represents a hydrogen atom, -S(=O)₂-R^{12g} or an alkyl radical selected from the group consisting of methyl, ethyl and n-propyl;
R^{12g} represents a phenyl radical of general formula (Ag), 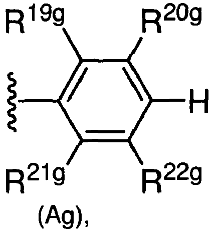 wherein
R^{19g}, R^{20g}, R^{21g} and R^{22g}, independently of one another, each represent a hydrogen atom; F, Cl, Br, I, -O-CH₃; -O-C₂H₅; -O-CF₃; -O-CFH₂; -O-CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, F, Cl and Br;
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl.

More preferred is a substituted tetrahydroisoquinoline compound of general formula Ig, wherein
[13] 6-(naphthalene-1-sulfonylamino)-3,4-dihydro-1H-isoquinoline-2-carboxylic acid tert-butyl ester,
[15] 6-(4-methyl-naphthalene-1-sulfonylamino)-3,4 -dihydro-1 H-isoquinoline-2-carboxylic acid tert-butyl ester,
[16] 6-(naphthalene-2-sulfonylamino)-3,4-dihydro-1H-isoquinoline-2-carboxylic acid tert-butyl ester and
[17] 6-(2-methoxy-5-methyl-benzenesulfonylamino)-3,4-dihydro-1H-isoquinoline-2-carboxylic acid tert-butyl ester.

If any of the substituents in any of the above defined formulae represents or comprises a 3- to 9-membered (hetero)cycloaliphatic radical, C₃₋₉ cycloalkyl radical or C₄₋₉ cycloalkenyl radical, said (hetero)cycloaliphatic radical, C₃₋₉ cycloalkyl radical or C₄₋₉ cycloalkenyl radical may - if not defined otherwise - be unsubstituted or substituted by one or more substituents, preferably unsubstituted or optionally substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and -NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br,-CN, -CF₃, -OCF₃, -SCF₃, -OH, -SH, -NH₂ and -NO₂.

If any of the substituents in any of the above defined formulae represents or comprises a cycloaliphatic radical, C₃₋₉ cycloalkyl radical or C₄₋₉ cycloalkenyl radical which contains one or more, preferably 1, 2 or 3 heteroatom(s) as ring member(s), unless defined otherwise, each of these heteroatom(s) may preferably be selected independently from the group consisting of N, O and S.

Suitable saturated or unsaturated, optionally at least one heteroatom as ring member containing cycloaliphatic radicals, C₃₋₉ cycloalkyl radicals or C₄₋₉ cycloalkenyl radicals may preferably be selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated, optionally at least one heteroatom as ring member containing cycloaliphatic radicals, C₃₋₉ cycloalkyl radicals or C₄₋₉ cycloalkenyl radicals which are condensed with an unsubstituted or at least mono-substituted mono- or bicyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydrocarbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, fluorenyl and (1,2,3,4)-tetrahydroquinoxazlinyl.

If any of the substituents in any of the above defined formulae represents an alkylene group, preferably an C₁₋₆ alkylene group, an alkenylene group, preferably an C₂₋₆ alkenylene group or an alkinylene group, preferably an C₂₋₆ alkinylene group, which may be substituted, said alkylene group, C₂₋₆alkylene group, alkenylene group, C₂₋₆ alkenylene group, alkinylene group or C₂₋₆alkinylene group may be unsubstituted or substituted by one or more substituents, preferably unsubstituted or optionally substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, - CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-,-(CH₂)₄-,-(CH₂)₅ and -(CH₂)₆-, suitable alkenylene groups include -CH=CH-, -CH₂-CH=CH- and -CH=CH-CH₂- and suitable alkinylene groups include -C≡C-, -CH₂-C≡C- and -C≡C-CH₂-.

If any of the substituents in any of the above defined formulae represents or comprises an aryl radical, including a 6-membered aryl radical such as phenyl or a 10-membered aryl radical such as naphthyl or a 14-membered aryl radical such as anthracenyl, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents in any of the above defined formulae represents or comprises a heteroaryl radical, including a monocyclic 5- or 6-membered heteroaryl radical or a bi- or tricyclic 8-, 9-, 10-, 11-, 12-, 13- or 14 membered heteroaryl radical, said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2, 3 or 4 heteroatom(s).

Suitable bi- or tricyclic heteroaryl radicals, which may optionally be at least mono-substituted, may preferably be selected from the group consisting of indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl.

Suitable mono-, bi- or tricyclic heteroaryl radicals, which are condensed with an unsubstituted or at least mono-substituted saturated or unsaturated mono- or bicyclic ring system, may preferably be selected from the group consisting of [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

Suitable monocyclic heteroaryl radicals, which may optionally be at least mono-substituted, may preferably be selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, O and S. The rings of the mono-or bicyclic ring system are preferably 5-, 6- or 7-membered.

Preferably a mono-or bicyclic ring system according to the present invention is a phenyl or naphthyl ring system.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂- phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and -NO₂.

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂, -S(=O)₂-CH₃, - S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituents in any of the above defined formulae represents a saturated or unsaturated aliphatic radical, i.e. an alkyl radical, preferably an C₁₋₁₀ alkyl radical; an alkenyl radical, preferably an C₂₋₁₀ alkenyl radical or an alkinyl radical, preferably an C₂₋₁₀ alkinyl radical; said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃, - SCF₃, -OH, -SH, -NH₂, -NH(C₁₋₅-alkyl) and -N(C₁₋₅-alkyl)₂, whereby in each occurrence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

In yet another aspect the present invention relates to a substituted tetrahydroisoquinoline compound of general formula Ih 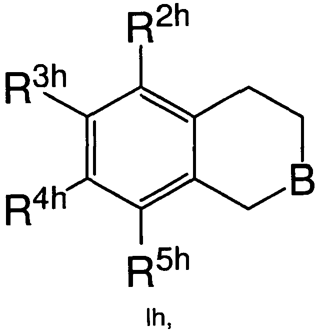 wherein
B represents a radical selected from the group consisting of 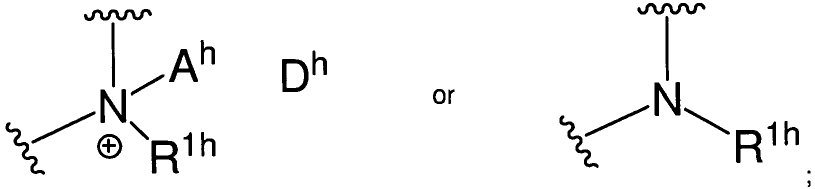
- A^{h}: represents a hydrogen atom or a radical selected from the group consisting of methyl, ethyl, n-propyl, isopropyl, n-butyl, isobutyl, 2-butyl and tert-butyl;
- D^{h}: represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate and benzenesulfonate;
R^{1h} represents a radical selected from the group consisting of H, methyl, ethyl, -C(=O)-cyclopropyl, -C(=O)-O-tert-butyl and -CH₂-cyclopropyl;
R^{2h}, R^{3h}, R^{4h} and R^{5h}, independently of one another, each represents a hydrogen atom or a -N(R^{11h})-S(=O)₂-R^{12h} radical;
with the proviso that at least one of the substituents R^{2h}, R^{3h}, R^{4h} and R^{5h} represents a - N(R^{11h}-S(=O)₂-R^{12h} moiety;
R^{11h} represents a radical selected from the group consisting of H, methyl and ethyl;
R^{12h} represents a radical selected from the group consisting of 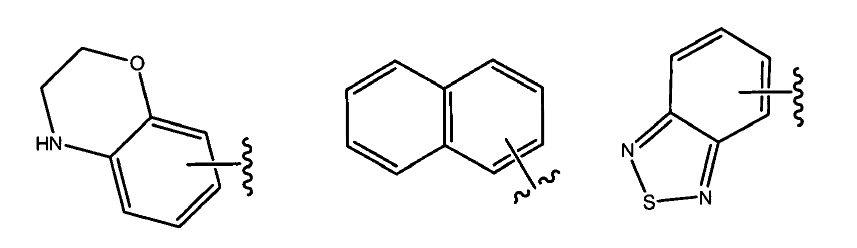 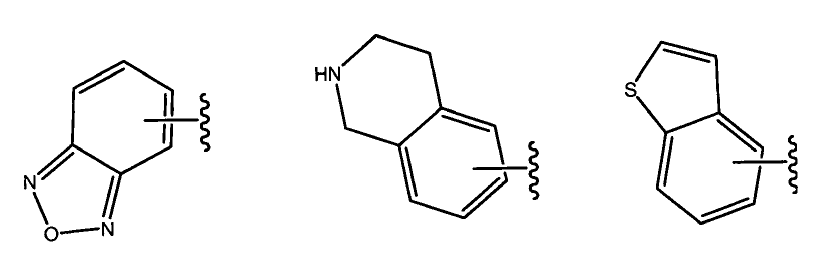 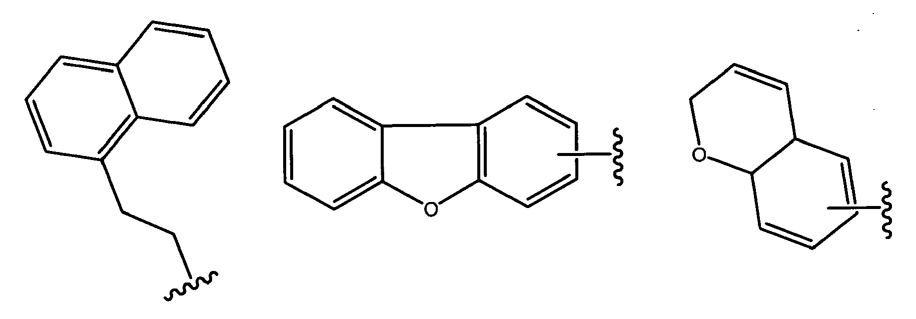 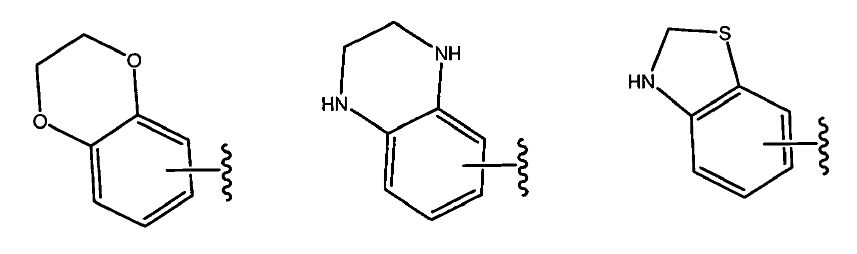 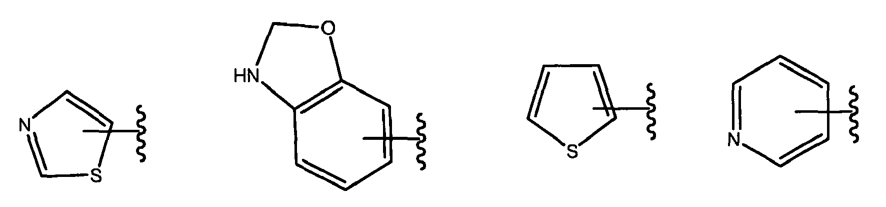 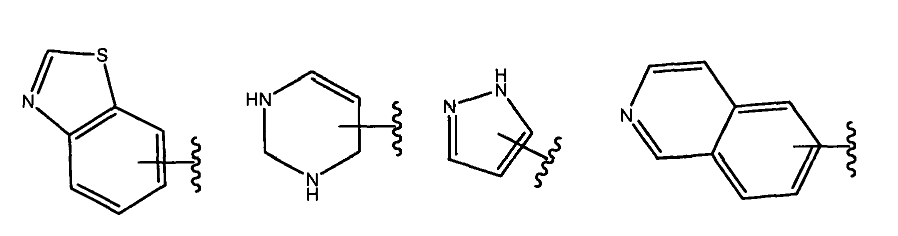 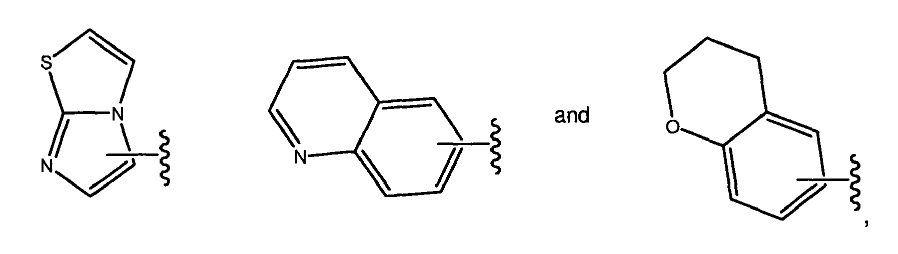 which is unsubstituted or substituted with 1, 2, 3, 4 or 5 substituents independently selected from the group consisting of H, F, Cl, methyl, ethyl, -NH₂, -N(CH₃)₂, oxo (=O), -O-CH₃, -O-CH₂-CH₃, -NH-(C=O)-CH₃, -C(=O)-O-CH₃, -C(=O)-CF₃, whereby the substitution can take place on any suitable position in the aforementioned radicals, including the heteroatom(s);
or a substituted phenyl radical selected from the group consisting of: 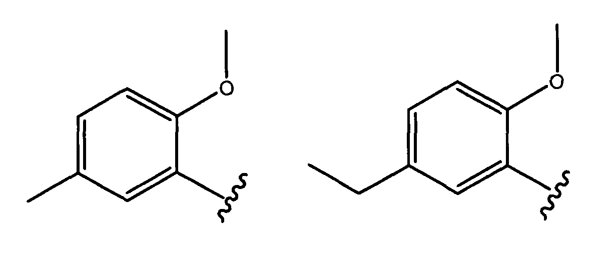 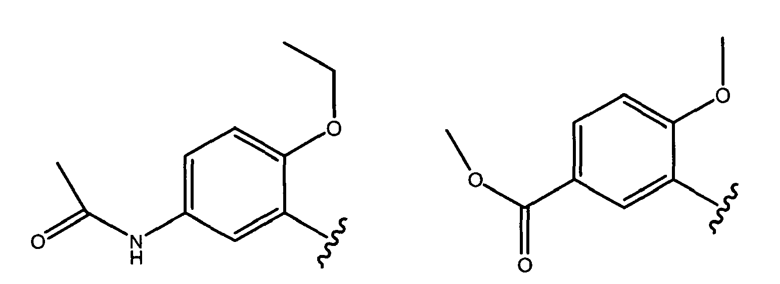 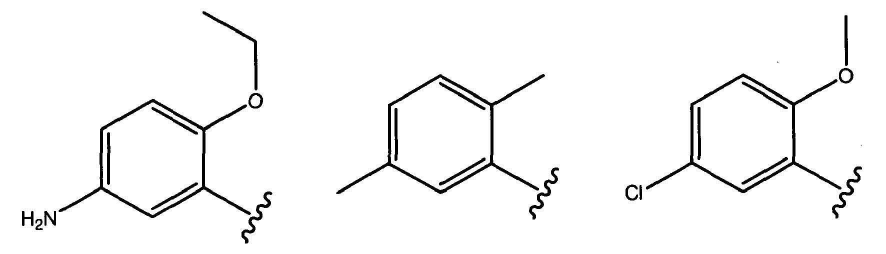 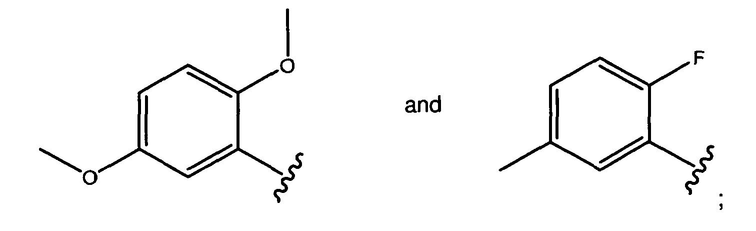 optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

Also preferred is a substituted tetrahydroisoquinoline compound of general formula Ih given above,
wherein
R^{1h}, R^{2h}, R^{3h}, R^{4h}, R^{5h} and R^{11h} have the meaning as defined above;
with the proviso that at least one of the substituents R^{2h}, R^{3h}, R^{4h} and R^{5h} represents a - N(R^{11h})-S(=O)₂-R^{12h} moiety;
and
R^{12h} represents a radical selected from the group consisting of 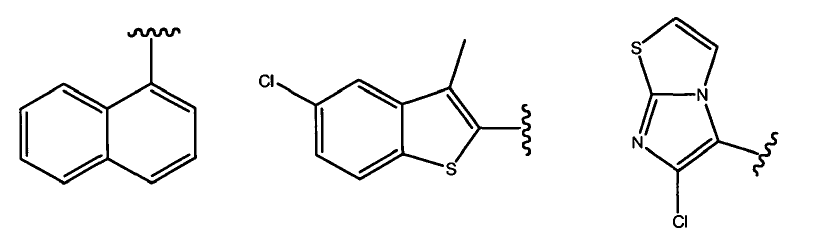 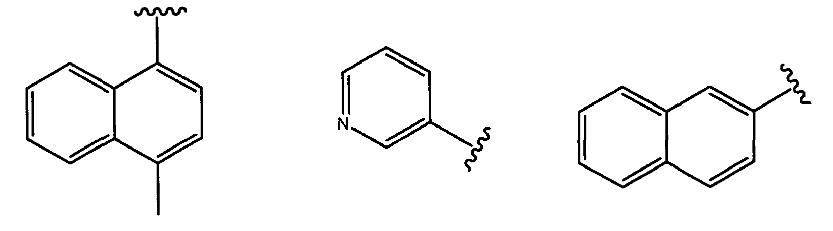 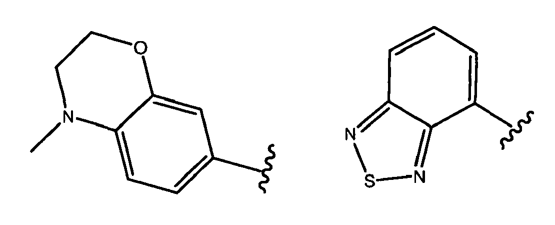 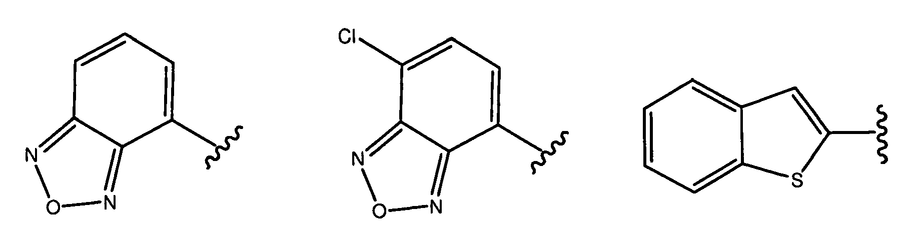 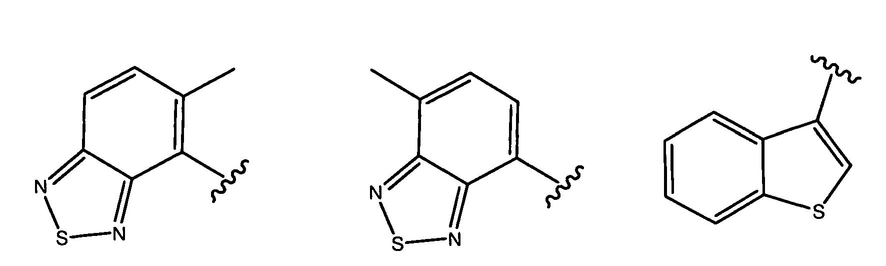 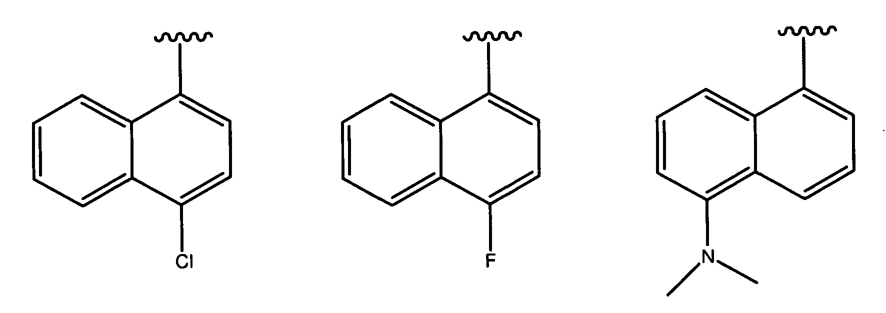 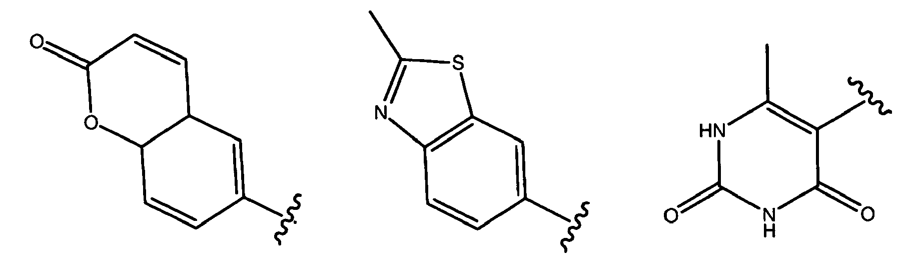 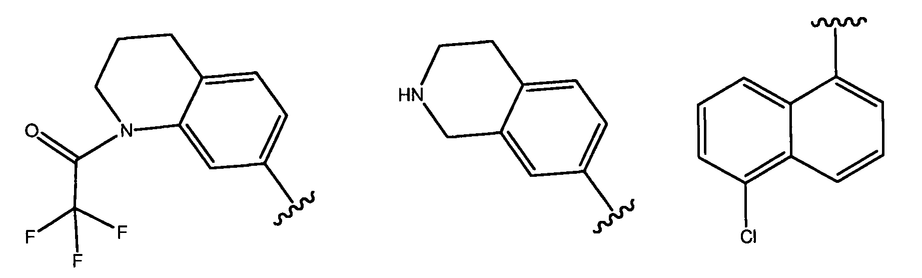 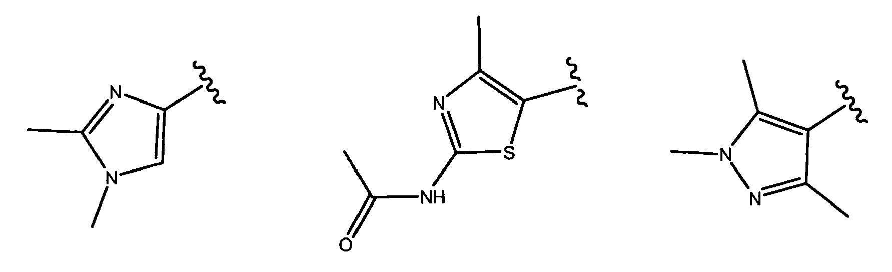 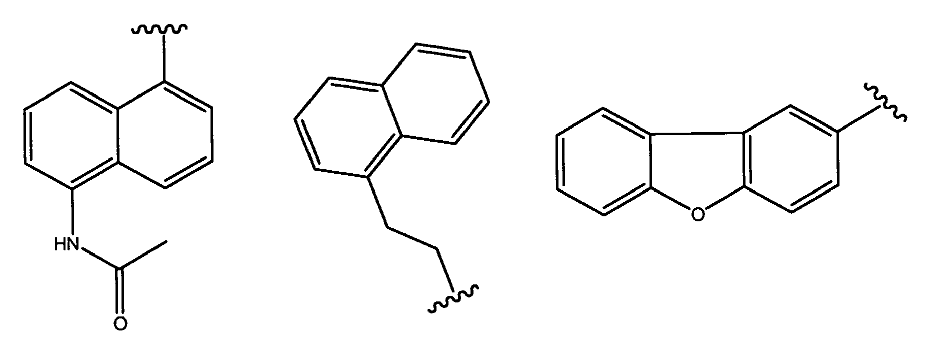 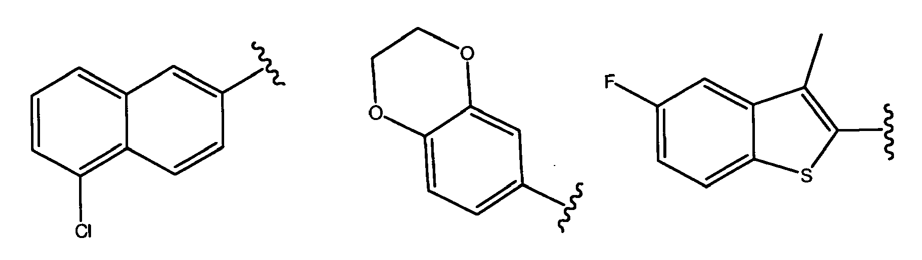 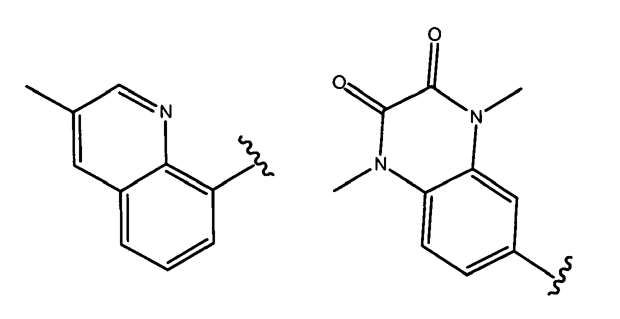 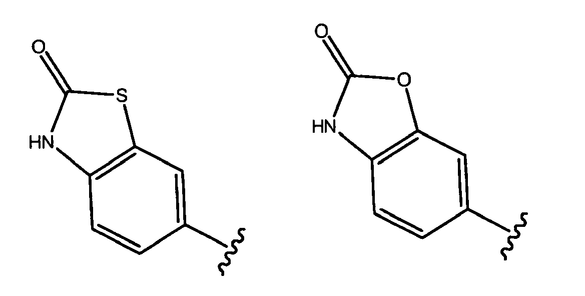 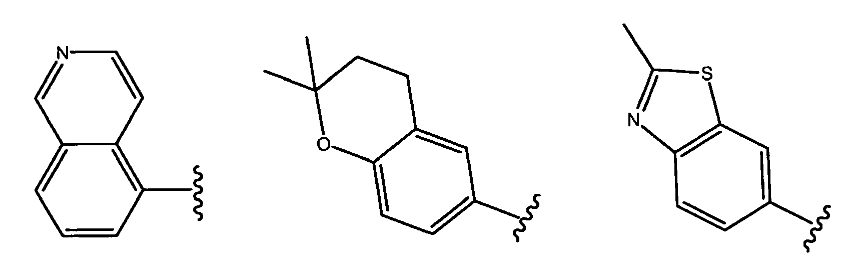 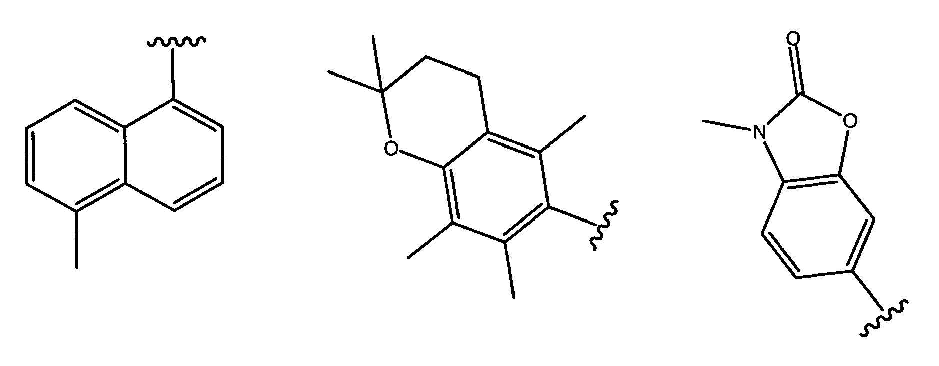 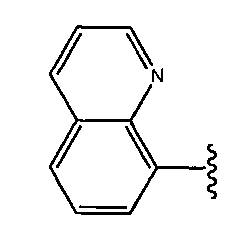 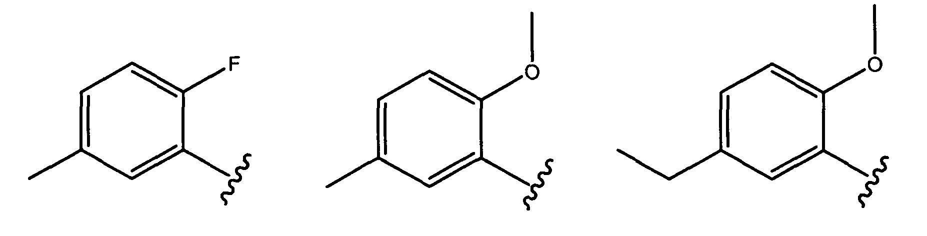 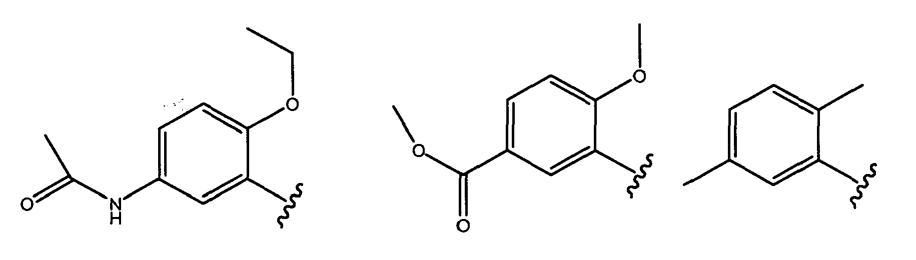 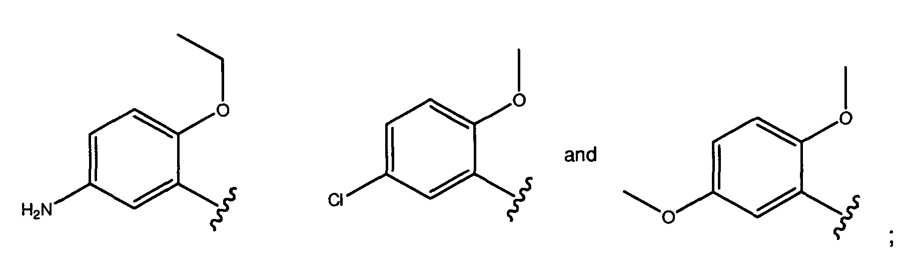 optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

Also preferred is a substituted tetrahydroisoquinoline compound of general formula Ih given above,
wherein R^{2h} represents a -N(R^{11h})-S(=O)₂-R^{12h} moiety;
and
B, A^{h}, D^{h}, R^{1h}, R^{3h}, R^{4h}, R^{5h}, R^{11h} and R^{12h} have the meaning as defined above;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

Also preferred is a substituted tetrahydroisoquinoline compound of general formula Ih given above,
wherein
R^{3h} represents a -N(R^{11h})-S(=O)₂-R^{12h} moiety;
and
B, A^{h}, D^{h}, R^{1h}, R^{2h}, R^{4h}, R^{5h}, R^{11h} and R^{12h} have the meaning as defined above;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

Also preferred is a substituted tetrahydroisoquinoline compound of general formula Ih given above,
wherein
R^{4h} represents a -N(R^{11h})-S(=O)₂-R^{12h} moiety;
and
B, A^{h}, D^{h}, R^{1h}, R^{2h}, R^{3h}, R^{5h}, R^{11h} and R^{12h} have the meaning as defined above;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

Also preferred is a substituted tetrahydroisoquinoline compound of general formula Ih given above,
wherein
R^{5h} represents a -N(R^{11h})-S(=O)₂-R^{12h} moiety;
and
B, A^{h}, D^{h}, R^{1h}, R^{2h}, R^{3h}, R^{4h}, R^{11h} and R^{12h} have the meaning as defined above;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

Also preferred is a substituted tetrahydroisoquinoline compound selected from the group consisting of
[1] N-(1,2,3,4-tetrahydroisoquinolin-6-yl)naphthalene-1-sulfonamide hydrochloride,
[2] 2,2-dimethyl-6-(N-methylnaphthalene-1-sulfonamido)-1,2,3,4-tetrahydroisoquinolinium iodide,
[3] N-(2-methyl-1,2,3,4-tetrahydroisoquinolin-6-yl)naphthalene-1-sulfonamide hydrochloride,
[4] 5-chloro-3-methyl-N-(1,2,3,4-tetrahydroisoquinolin-6-yl)benzo[b]thiophene-2-sulfonamide hydrochloride,
[5] 5-chloro-3-methyl-N-(2-methyl-1,2,3,4-tetrahydroisoquinolin-6-yl)benzo[b]thiophene-2-sulfonamide hydrochloride,
[6] 4-methyl-N-(1,2,3,4-tetrahydroisoquinolin-6-yl)naphthalene-1-sulfonamide hydrochloride,
[7] 4-methyl-N-(2-methyl-1,2,3,4-tetrahydroisoquinolin-6-yl)naphthalene-1-sulfonamide hydrochloride,
[8] N-(1,2,3,4-tetrahydroisoquinolin-6-yl)naphthalene-2-sulfonamide hydrochloride,
[9] N-(2-methyl-1,2,3,4-tetrahydroisoquinolin-6-yl)naphthalene-2-sulfonamide hydrochloride,
[10] 6-chloro-N-(1,2,3,4-tetrahydroisoquinolin-6-yl)imidazo[2,1-b]thiazole-5-sulfonamide hydrochloride,
[11] 2-methoxy-5-methyl-N-(1,2,3,4-tetrahydroisoquinolin-6-yl)benzenesulfonamide hydrochloride,
[12] N-(1,2,3,4-tetrahydroisoquinolin-6-yl)pyridine-3-sulfonamide dihydrochloride,
[13] 6-(naphthalene-1-sulfonylamino)-3,4-dihydro-1H-isoquinoline-2-carboxylic acid tert-butyl ester,
[14] 6-(5-chloro-3-methyl-benzo[b]thiophene-2- sulfonylamino)-3,4-dihydro-1H-isoquinoline-2-carboxylic acid tert-butyl ester,
[15] 6-(4-methyl-naphthalene-1-sulfonylamino)-3,4-dihydro-1H-isoquinoline-2-carboxylic acid tert-butyl ester,
[16] 6-(naphthalene-2-sulfonylamino)-3,4-dihydro-1H-isoquinoline-2-carboxylic acid tert-butyl ester,
[17] 6-(2-methoxy-5-methyl-benzenesulfonylamino)-3,4-dihydro-1H-isoquinoline-2-carboxylic acid tert-butyl ester
[18] 6-(pyridine-3-sulfonylamino)-3,4-dihydro-1H-isoquinoline-2carboxylic acid tert-butyl ester;
[19] 6-Chloro-imidazo[2,1-b]thiazole-5-sulfonic acid (2-methyl-1,2,3,4tetrahydro-isoquinolin-6-yl)-amide hydrochloride
[20] 6-(6-Chloro-imidazo[2,1-b]thiazole-5-sulfonylamino)-3,4-dihydro-1Hisoquinoline-2-carboxylic acid tert-butyl ester
[21] 4-Methyl-3,4-dihydro-2H-benzo[1,4]oxazine-7-sulfonic acid (1,2,3,4-tetrahydro-isoquinolin-6-yl)-amide hydrochloride
[23] Benzo[1,2,5]thiadiazole-4-sulfonic acid (1,2,3,4-tetrahydro-isoquinolin-6-yl)-amide hydrochloride
[24] Benzo[b]thiophene-2-sulfonic acid (1,2,3,4-tetrahydro-isoquinolin-6-yl)-amide hydrochloride
[25] 7-Chloro-benzo[1,2,5] oxadiazole-4-sulfonic acid (1,2,3,4-tetrahydro-isoquinolin - 6-yl)-amide hydrochloride
[26] Benzo[1,2,5]oxadiazole-4-sulfonic acid (1,2,3,4-tetrahydro-isoquinolin-6-yl)-amide hydrochloride
[27] 5-Methyl-benzo[1,2,5] thiadiazole-4-sulfonic acid (1,2,3,4-tetrahydro-isoqui- nolin-6-yl)-amide hydrochloride
[28] 7-Methyl-benzo[1,2,5]thiadiazole-4-sulfonic acid (1,2,3,4-tetrahydro-isoqui- nolin-6-yl)-amide hydrochloride
[29] Benzo[b]thiophene-3-sulfonic acid (1,2,3,4- tetrahydro-isoquinolin-6-yl) -amide hydrochloride
[30] 4-Fluoro-naphthalene-1-sulfonic acid (1,2,3,4-tetrahydro-isoquinolin-6-yl)-amide hydrochloride
[31] 4-Chloro-naphthalene-1-sulfonic acid (1,2,3,4-tetrahydro-isoquinolin-6-yl)-amide hydrochloride
[32] 5-Dimethylaminonaphtha- lene-1-sulfonic acid (1,2,3,4 -tetrahydro-isoquinolin -6-yl)-amide hydrochloride
[33] 2-Oxo-4a,8a-dihydro-2H-chromene-6-sulfonic acid (1,2,3,4-tetrahydro-isoquinolin-6-yl)-amide hydrochloride
[34] 2-Methyl-benzothiazole-6-sulfonic acid (1,2,3,4- tetrahydro-isoquinolin-6-yl)-amide hydrochloride
[35] 5-Ethyl-2-methoxy-N-(1,2,3,4-tetrahydro- isoquinolin-6-yl)-benzene sulfonamide hydrochloride
[36] 6-Methyl-2,4-dioxo-1,2,3,4-tetrahydro-pyrimidine-5-sulfonic acid (1,2,3,4-tetrahydro-isoquinolin-6-yl)-amide hydrochloride
[37] N-[4-Ethoxy-3-(1,2,3,4-tetrahydro-isoquinolin-6-ylsulfamoyl)-phenyl]-acetamide hydrochloride
[38] 4-Methoxy-3-(1,2,3,4-tetrahydro-isoquinolin-6-yl sulfamoyl)-benzoic acid methyl ester hydrochloride
[39] 2-(2,2,2-Trifluoro-acetyl)-1,2,3,4-tetrahydro-isoquinoline-7-sulfonic acid (1,2,3,4-tetrahydro-isoquinolin-6-yl)-amide hydrochloride
[40] 1,2,3,4-Tetrahydro-isoquino line-7-sulfonic acid (1,2,3,4-tetrahydro-isoquinolin-6-yl)-amide dihydrochloride
[41] 5-Amino-2-ethoxy-N-(1,2,3,4-tetrahydro-isoquinolin-6-yl)-benzenesulfonamide dihydrochloride
[42] 5-Chloro-naphthalene-1-sulfonic acid (1,2,3,4- tetrahydro-isoquinolin-6-yl)-amide hydrochloride
[43] 1,2-Dimethyl-1H-imidazole-4-sulfonic acid (1 ,2,3,4-tetrahydro-isoquinolin- 6-yl)-amide hydrochloride
[44] N-[4-Methyl-5-(1,2,3,4-tetrahydro-isoquinolin-6- ylsulfamoyl)-thiazol-2-yl] - acetamide hydrochloride
[45] 1,3,5-Trimethyl-1H-pyrazole -4-sulfonic acid (1,2,3,4-tetrahydro-isoquinolin-6-yl) - amide hydrochloride
[46] N-[5-(1,2,3,4-Tetrahydro-isoquinolin-6-ylsulfamoyl) -naphthalen-1-yl]-acetamide hydrochloride
[47] 2-Naphthalen-1-yl-ethanesulfonic acid (1,2,3,4 -tetrahydro-isoquinolin-6-yl) - amide hydrochloride
[48] Dibenzofuran-2-sulfonic acid (1,2,3,4-tetrahydro- isoquinolin-6-yl)-amide hydrochloride
[49] 2,5-Dimethoxy-N-(1,2,3,4-tetrahydro-isoquinolin -6-yl)-benzenesulfonamide hydrochloride
[50] 5-Chloro-2-methoxy-N-(1,2,3,4-tetrahydro-iso quinolin-6-yl)-benzene sulfonamide hydrochloride
[51] 2,5-Dimethyl-N-(1,2,3,4-tetrahydro-isoquinolin-6-yl)-benzenesulfonamide hydrochloride
[52] 2-Fluoro-5-methyl-N-(1,2,3,4-tetrahydro-isoquinolin-6-yl)-benzene sulfonamide hydrochloride
[53] 6-Chloro-imidazo[2,1-b]thiazole-5-sulfonic acid (2-methyl-1,2,3,4-tetrahydro - isoquinolin-7-yl)-amide hydrochloride
[54] 6-Chloro-imidazo[2,1-b]thiazole-5-sulfonic acid (1,2,3,4-tetrahydro-iso- quinolin-7-yl)-amide hydrochloride
[55] 4-Methyl-naphthalene-1-sulfonic acid (1,2,3,4-tetrahydro-isoquinolin-7-yl)-amide hydrochloride
[56] 4-Methyl-naphthalene-1-sulfonic acid (2-methyl-1,2,3,4-tetrahydro-isoquinolin-7-yl)-amide hydrochloride
[57] 5-Chloro-naphthalene-2-sulfonic acid (2-methyl-1,2,3,4-tetrahydro-isoquinolin-7-yl)-amide hydrochloride
[58] 5-Chloro-naphthalene-2-sulfonic acid (1,2,3,4-tetrahydro-isoquinolin-7-yl) -amide hydrochloride
[59] 4-Methyl-3,4-dihydro-2H-benzo[1,4]oxazine-7-sulfonic acid (1,2,3,4-tetrahydro-isoquinolin-7-yl)-amide hydrochloride
[60] 5-Chloro-3-methyl-benzo[b]thiophene-2-sulfonic acid (1,2,3,4-tetrahydro-isoquinolin -7-yl)-amide hydrochloride
[61] Naphthalene-1-sulfonic acid (1,2,3,4-tetrahydro- isoquinolin-7-yl)-amide hydrochloride
[63] 2,3-Dihydro-benzo[1,4] dioxine-6-sulfonic acid (1,2,3,4-tetrahydro-iso- quinolin-7-yl)-amide hydrochloride
[64] 5-Fluoro-3-methyl-benzo[b] thiophene-2-sulfonic acid (1,2,3,4-tetrahydro-isoquinolin -7-yl)-amide hydrochloride
[65] 3-Methyl-quinoline-8-sulfonic acid (1,2,3,4-tetra hydro-isoquinolin-7-yl)-amide hydrochloride
[66] Naphthalene-2-sulfonic acid (1,2,3,4-tetrahydro -isoquinolin-7-yl)-amide hydrochloride
[67] Benzo[1,2,5]thiadiazole-4-sulfonic acid (1,2,3,4-tetrahydro-isoquinolin-7-yl) - amide hydrochloride
[68] 1,4-Dimethyl-2,3-dioxo-1,2,3,4-tetrahydro-quino xaline-6-sulfonic acid (1,2,3,4-tetrahydro-isoquinolin-7-yl)-amide hydrochloride
[69] Benzo[b]thiophene-2-sulfonic acid (1,2,3,4-tetrahydro -isoquinolin-7-yl)-amide hydrochloride
[71] 7-Chloro-benzo[1,2,5] oxadiazole-4-sulfonic acid (1,2,3,4-tetrahydro-isoquinolin-7-yl)-amide hydrochloride
[72] Benzo[1,2,5]oxadiazole-4-sulfonic acid (1,2,3,4-tetrahydro-isoquinolin-7-yl)-amide hydrochloride
[73] 2-Oxo-2,3-dihydro-benzo thiazole-6-sulfonic acid (1,2,3,4-tetrahydro-isoqui- nolin-7-yl)-amide hydro- chloride
[74] 2-Oxo-2,3-dihydro-benzo oxazole-6-sulfonic acid (1,2,3,4-tetrahydro-isoquinolin-7-yl)-amide hydrochloride
[76] 5-Methylbenzo[1,2,5] thiadiazole -4-sulfonic acid (1,2,3,4-tetrahydro-isoquinolin-7-yl)-amide hydrochloride
[77] 7-Methyl-benzo[1,2,5] thiadiazole-4-sulfonic acid (1,2,3,4-tetrahydro-isoquinolin-7-yl)-amide hydrochloride
[78] Benzo[b]thiophene-3-sulfonic acid (1,2,3,4- tetrahydro-isoquinolin-7-yl) -amide hydrochloride
[79] Isoquinoline-5-sulfonic acid (1,2,3,4-tetrahydro-isoquinolin-7-yl)-amide hydrochloride
[80] 4-Fluoro-naphthalene-1-sulfonic acid (1,2,3,4 -tetrahydro-isoquinolin-7-yl) -amide hydrochloride
[81] 4-Chloro-naphthalene-1-sulfonic acid (1,2,3,4 -tetrahydro-isoquinolin-7-yl) -amide hydrochloride
[82] 2,2-Dimethyl-chroman-6-sulfonic acid (1,2,3,4- tetrahydro-isoquinolin-7-yl) -amide hydrochloride
[83] 5-Dimethylamino-naphtha lene-1-sulfonic acid (1,2,3,4-tetrahydro-isoquinolin-7-yl) -amide hydrochloride
[84] 2-Oxo-2H-chromene-6-sulfonic acid (1,2,3,4-tetrahydro-isoquinolin-7-yl)- amide hydrochloride
[85] 2-Methyl-benzothiazole-6-sulfonic acid (1,2,3,4-tetrahydro-isoquinolin-7-yl) - amide hydrochloride
[86] 5-Ethyl-2-methoxy-N-(1,2,3,4-tetrahydro-isoquinolin-7-yl)-benzenesulfonamide hydrochloride
[87] 6-Methyl-2,4-dioxo-1,2,3,4-tetrahydro-pyrimidine- 5-sulfonic acid (1,2,3,4-tetrahydro-isoquinolin-7-yl)-amide hydrochloride
[88] 6-Chloro-imidazo[2,1-b] thiazole-5-sulfonic acid ethyl-(2-ethyl-1,2,3,4-tetrahydro-isoquinolin-5-yl) -amide hydrochloride
[89] 6-Chloro-imidazo[2,1-b] thiazole-5-sulfonic acid (2-ethyl-1,2,3,4-tetrahydro-isoquinolin-5-yl)-amide hydrochloride
[90] 6-Chloro-imidazo[2,1-b] thiazole-5-sulfonic acid (1,2,3,4-tetrahydro-isoquinolin-5-yl)-amide hydrochloride
[91] 5-Methyl-naphthalene-1-sulfonic acid (1,2,3,4- tetrahydro-isoquinolin-5-yl) -amide hydrochloride
[92] Naphthalene-1-sulfonic acid (1,2,3,4-tetrahydro- isoquinolin-5-yl)-amide hydrochloride
[93] 5-Chloro-naphthalene-2-sulfonic acid (1,2,3,4- tetrahydro-isoquinolin-5-yl) -amide hydrochloride
[94] 4-Methyl-3,4-dihydro-2H-benzo[1,4]oxazine-7- sulfonic acid (1,2,3,4-tetrahydro-isoquinolin-5-yl)-amide hydrochloride
[95] 5-Chloro-3-methyl-benzo[b] thiophene-2-sulfonic acid ethyl-(2-ethyl-1 ,2,3,4-tetrahydro-isoquinolin-5-yl)-amide hydrochloride
[96] 5-Chloro-3-methyl-benzo[b] thiophene-2-sulfonic acid (1,2,3,4-tetrahydro-isoquinolin-5-yl)-amide hydrochloride
[98] 2,3-Dihydro-benzo[1,4] dioxine-6-sulfonic acid (1,2,3,4-tetrahydro-isoquinolin-5-yl)-amide hydrochloride
[99] 5-Fluoro-3-methyl-benzo[b] thiophene-2-sulfonic acid (1,2,3,4-tetrahydro-isoquinolin-5-yl)-amide hydrochloride
[100] 3-Methyl-quinoline-8-sulfonic acid ethyl-(2-ethyl -1,2,3,4-tetrahydro-isoquinolin-5-yl)-amide hydrochloride
[101] 3-Methyl-quinoline-8-sulfonic acid (1,2,3,4- tetrahydro-isoquinolin-5-yl) -amide hydrochloride
[102] Naphthalene-2-sulfonic acid (1,2,3,4-tetrahydro-isoquino lin-5-yl)-amide hydrochloride
[103] Benzo[1,2,5]thiadiazole-4-sulfonic acid (1,2,3,4- tetrahydro-isoquinolin-5-yl) - amide hydrochloride
[104] Benzo[b]thiophene-2-sulfonic acid (1,2,3,4- tetrahydro-isoquinolin-5-yl) -amide hydrochloride
[105] Benzo[1,2,5]oxadiazole-4-sulfonic acid (1,2,3,4-tetra hydro-isoquinolin-5-yl)-amide hydrochloride
[106] 7-Chlorobenzo[1,2,5]oxa diazole -4-sulfonic acid (1,2,3,4-tetrahydro-isoquinolin-5-yl)-amide hydrochloride
[107] 2-Oxo-2,3-dihydro-benzooxazole-6-sulfonic acid (1,2,3,4-tetrahydro-isoquinolin-5-yl) -amide hydrochloride
[108] 5-Methyl-benzo[1,2,5] thiadiazole-4-sulfonic acid (1,2,3,4-tetrahydro-isoquinolin-5-yl)-amide hydrochloride
[109] 7-Methyl-benzo[1,2,5] thiadiazole-4-sulfonic acid (1,2,3,4-tetrahydro-isoquinolin-5-yl)-amide hydrochloride
[110] Benzo[b]thiophene-3-sulfonic acid (1,2,3,4- tetrahydro-isoquinolin-5-yl) -amide hydrochloride
[111] Isoquinoline-5-sulfonic acid (1,2,3,4-tetrahydro-isoquinolin-5-yl)-amide dihydrochloride
[112] 4-Fluoro-naphthalene-1-sulfonic acid (1,2,3,4- tetrahydro-isoquinolin-5-yl) -amide hydrochloride
[113] 4-Chloro-naphthalene-1-sulfonic acid (1,2,3,4- tetrahydro-isoquinolin-5-yl) -amide hydrochloride
[114] 2,2-Dimethyl-chroman-6-sulfonic acid (1,2,3,4- tetrahydro-isoquinolin-5-yl) -amide hydrochloride
[115] 5-Dimethylamino-naphthalene-1-sulfonic acid (1,2,3,4-tetrahydro-isoquinolin-5-yl)-amide hydrochloride
[116] 2,2,5,7,8-Pentamethyl-chroman-6-sulfonic acid (1,2,3,4-tetrahydro-isoquino lin-5-yl)-amide hydrochloride
[117] 2-Oxo-2H-chromene-6-sulfonic acid (1,2,3,4-tetrahy dro-isoquinolin-5-yl) -amide hydrochloride
[118] 5-Ethyl-2-methoxy-N(1,2, 3,4-tetrahydro-isoquinolin-5-yl)-benzenesulfonamide hydrochloride
[119] N-[4-Ethoxy-3-(1,2,3,4-tetrahydro-isoquinolin-5-yl sulfamoyl)-phenyl]-acetamide hydrochloride
[120] 2-Methoxy-5-methyl-N-(1,2,3,4-tetrahydro-isoquinolin-5-yl)-benze nesulfonamide hydrocloride
[121] 5-Chloro-naphthalene-1-sulfonic acid (1,2,3,4-tetrahydro-isoquinolin-5-yl) -amide hydrochloride
[122] 2-Methoxy-5-methyl-N-(1,2,3,4-tetrahydro-isoquinolin-5-yl)-benzenesulfonamide hydrocloride
[123] Quinoline-8-sulfonic acid (1,2,3,4-tetrahydro-isoquinolin -5-yl)-amide hydrochloride
[124] Dibenzofuran-2-sulfonic acid (1,2,3,4-tetrahydro- isoquinolin-5-yl)-amide hydrochloride
[125] 6-Chloro-imidazo[2,1-b] thiazole-5-sulfonic acid (1,2,3,4-tetrahydro-isoquinolin-8-yl)-amide hydrochloride
[126] 6-chloro-N-(2-methyl-1,2,3,4-tetrahydroisoquinolin-8-yl)imidazo[2,1-b]thiazole-5-sulfonamide hydrochloride
[127] Benzo[b]thiophene-2-sulfonic acid (1,2,3,4-tetra hydro-isoquinolin-8-yl) -amide hydrochloride
[128] Benzo[b]thiophene-2-sulfonic acid (2-methyl- 1,2,3,4-tetrahydro-isoquinolin-8-yl)-amide hydrochloride
[129] Benzo[b]thiophene-3-sulfonic acid (2-methyl- 1,2,3,4-tetrahydro-isoquinolin-8-yl)-amide hydrochloride
[130] Benzo[b]thiophene-3-sulfonic acid (1,2,3,4-tetra hydro-isoquinolin-8-yl) -amide hydrochloride
[131] 5-Chloro-3-methyl-benzo[b]thiophene-2-sulfonic acid (2-methyl-1,2,3,4-tetrahydro-isoquinolin-8-yl)-amide hydrochloride
[132] 5-Chloro-3-methyl-benzo[b]thiophene-2-sulfonic acid (1,2,3,4-tetrahydro-isoquinolin -8-yl) -amide hydrochloride
[133] Naphthalene-2-sulfonic acid (1,2,3,4-tetrahydro-isoquinolin-8-yl)-amide hydrochloride
[134] Naphthalene-2-sulfonic acid (2-methyl-1 ,2,3,4-tetrahydro-isoquinolin-8-yl) -amide hydrochloride
[137] Naphthalene-1-sulfonic acid (1,2,3,4-tetrahydro-isoquinolin-8-yl)-amide hydrochloride
[138] Naphthalene-1-sulfonic acid (2-methyl-1,2,3,4-tetrahydro-isoquinolin-8-yl)-amide Hydrochloride
[139] 4-Methyl-naphthalene-1-sulfonic acid (2-methyl- 1,2,3,4-tetrahydro-isoquinolin-8-yl)-amide Hydrochloride
[140] 4-Methyl-naphthalene-1-sulfonic acid (1,2,3,4-tetrahydro-isoquinolin-8-yl)-amide Hydrochloride
[141] 4-Chloro-naphthalene-1-sulfonic acid (1,2,3,4-tetrahydro-isoquinolin-8-yl)- amide Hydrochloride
[142] 4-Chloro-naphthalene-1-sulfonic acid (2-methyl -1,2,3,4-tetrahydro-isoquinolin-8-yl)-amide Hydrochloride
[143] 5-Chloro-naphthalene-1-sulfonic acid (2-methyl -1,2,3,4-tetrahydro-isoquinolin-8-yl)-amide Hydrochloride
[144] 5-Chloro-naphthalene-1-sulfonic acid (1,2,3,4-tetrahydro-isoquinolin-8-yl)- amide Hydrochloride
[145] 5-Chloro-naphthalene-2-sulfonic acid (1 ,2,3,4-tetrahydro-isoquinolin-8-yl)- amide Hydrochloride
[146] 5-Chloro-naphthalene-2-sulfonic acid (2-methyl -1,2,3,4-tetrahydro-isoquinolin-8-yl)-amide Hydrochloride
[147] 4-Methyl-3,4-dihydro-2H-benzo[1,4]oxazine-7- sulfonic acid (2-methyl-1,2,3,4-tetrahydro-isoquinolin-8-yl)-amide Hydrochloride
[148] 4-Methyl-3,4-dihydro-2H-benzo[1,4]oxazine-7- sulfonic acid (1,2,3,4-tetrahydro-isoquinolin-8-yl)-amide Hydrochloride
[149] 5-Ethyl-2-methoxy-N-(1,2,3,4-tetrahydro- isoquinolin-8-yl)-benzenesulfonamide Hydrochloride
[150] 5-Ethyl-2-methoxy-N-(2-methyl-1,2,3,4-tetrahydro-isoquinolin-8-yl)-benzene sulfonamide Hydrochloride
[151] 5-Methyl-benzo[1,2,5] thiadiazole-4-sulfonic acid (2-methyl-1,2,3,4-tetrahydro - isoquinolin-8-yl)-amide Hydrochloride
[152] 5-Methyl-benzo[1,2,5] thiadiazole-4-sulfonic acid (1,2,3,4-tetrahydro-isoquinolin-8-yl)-amide Hydrochloride
[153] Naphthalene-2-sulfonic acid (1,2,3,4-tetrahydro-isoquinolin-6-yl)-amide and
[154] 5-Chloro-naphthalene-1-sulfonic acid (1,2,3,4- tetrahydro-isoquinolin-7-yl)-amide Hydrochloride
[155] Naphthalene-2-sulfonic acid (2-cyclopropanecarbonyl- 1,2,3,4-tetrahydro-isoquinolin-6-yl)-amide
[156] Naphthalene-2-sulfonic acid (2-cyclopropylmethyl- 1,2,3,4-tetrahydro-isoquinolin-6-yl)-amide Hydrochloride
[157] 6-Chloro-imidazo[2,1-b] thiazole-5-sulfonic acid (2-cyclopropanecarbonyl- 1,2,3,4-tetrahydro-isoquino lin-6-yl)-amide and
[158] 6-Chloro-imidazo[2,1-b] thiazole-5-sulfonic acid (2-cyclopropylmethyl-1,2,3,4-tetrahydro-isoquinolin-6-yl)-amide hydrochloride;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

In still another aspect the present invention relates to a process for the preparation of a substituted tetrahydroisoquinoline compound of general formula Ih, wherein at least one compound of general formula IVh, 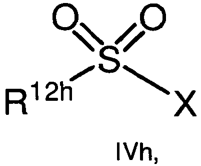 wherein R^{12h} has the meaning given above and X represents a leaving group, preferably a halogen atom, particularly preferably a chlorine atom, is reacted with at least one compound of general formula VhA, 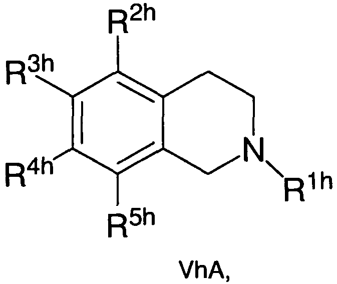 wherein R^{1h} to R^{5h} have the meaning given above, with the proviso that at least one substituent of the group consisting of R^{2h}, R^{3h}, R^{4h} and R^{5h} represents a -N(H)(R^{11h}) moiety, wherein R^{11e} has the meaning given above, or a protected derivative thereof, in a reaction medium, preferably in a reaction medium selected from the group consisting of pyridine, chloroform, dichloromethane, tetrahydrofurane and mixtures thereof, preferably in the presence of at least one base, more preferably in the presence of at least one base selected from the group consisting of triethylamine, diisopropylethylamine and diethylisopropylamine, preferably at a temperature between 0 °C and 30 °C.

If the substituted tetrahydroisoquinoline compounds of general formula Ih are obtained in form of a mixture of stereoisomers, particularly enantiomers or diastereomers, said mixtures may be separated by standard procedures known to those skilled in the art, e.g. chromatographic methods or crystallization with chiral reagents.

Compounds of general formula IV and/or IVhA are in most cases commercially available or may be prepared by processes known to those skilled in the art.

Compounds of general formula V and/or VhA are in most cases commercially available or may be prepared by processes known to those skilled in the art.

In particular, 1,2,3,4-tetrahydroisoquinoline compounds with an amino group in position 5 can be prepared starting from 5-nitro-1,2,3,4-tetrahydroisoquinoline compounds. A process for the preparation of the latter compounds is described in K. V. Rao et al., Journal of Heterocyclic Chemistry, 1973, 10, 213 to 215.

In particular, 1,2,3,4-tetrahydroisoquinoline compounds with an amino group in position 6 are commercially available or can be prepared starting from 6-nitro-1,2,3,4-tetrahydroisoquinoline compounds. A process for the preparation of the latter compounds is described in G. J. Quallich, Journal of Organic Chemistry, 1998, 63, 4116 to 4119.

1,2,3,4-tetrahydroisoquinoline compounds with a nitro group in position 6 or 8 may be prepared by established procedures described in M. Tercel, Journal of Medicinal Chemistry, 1996, 39, 1084 to 1094.

In particular, 1,2,3,4-tetrahydroisoquinoline compounds with an amino group in position 7 are commercially available or can be prepared starting from 7-nitro-1,2,3,4-tetrahydroisoquinoline compounds. A process for the preparation of the latter compounds is described in J. F. Ajao et al., Journal of Heterocyclic Chemistry, 1985, 22, 329 to 331.

The N-methyl-8-amino-substituted 1,2,3,4-tetrahydroisoquinoline compounds were prepared by bromination and nitration of the corresponding 1,2,3,4-tetrahydroisoquinolines followed by two-step standard reduction conditions as described in M. Rey, Helvetica Chimica Acta, 1985, 66, 1828 to 1834.

In all the following schemes 1 to 7 protecting groups for the nitrogen atom may be used. Some examples include cyclic imide derivatives, such as maleimides or succinimides, a variety of carbamates, such as tert-butoxy-carbonyl (BOC) and fluorenylmethyloxycarbonyl (Fmoc)m a variety of amides, such as acetamides, and alkyl and aryl amine derivatives, such as *N*-benzyl or *N*-allyl amines. Additional examples of nitrogen protecting groups can be found in reference books such as Protective groups in Organic Chemistry, ed. J. F. W. McOmie, Plenum Press, 1973; and T.W. Greene & P.G.M. Wuts, Protective Groups in Organic Chemistry, John Wiley & sons, 1999.

### Preparation of substituted tetrahydroisoquinoline compounds of general formula Ia (scheme 1 and 2)

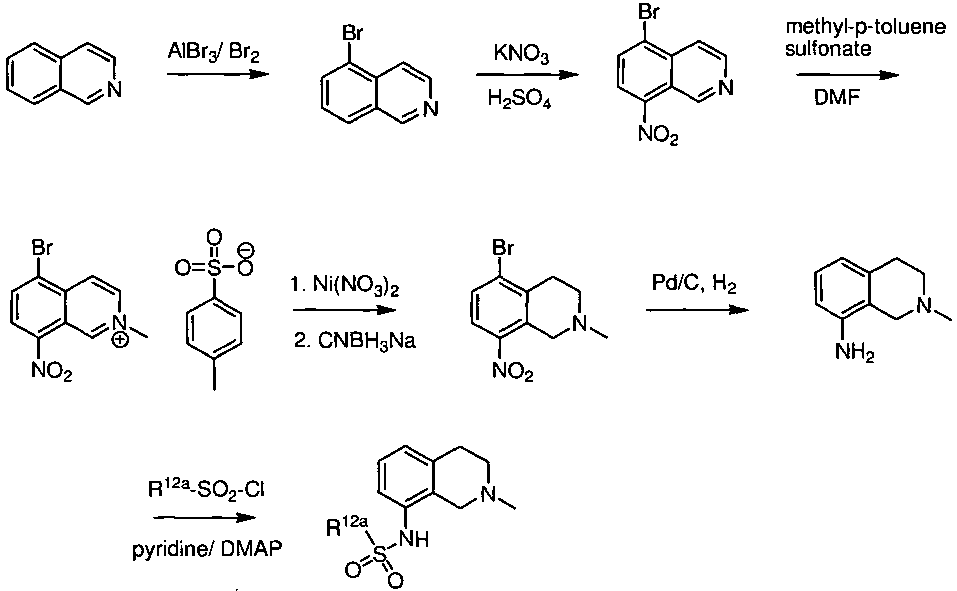

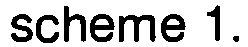

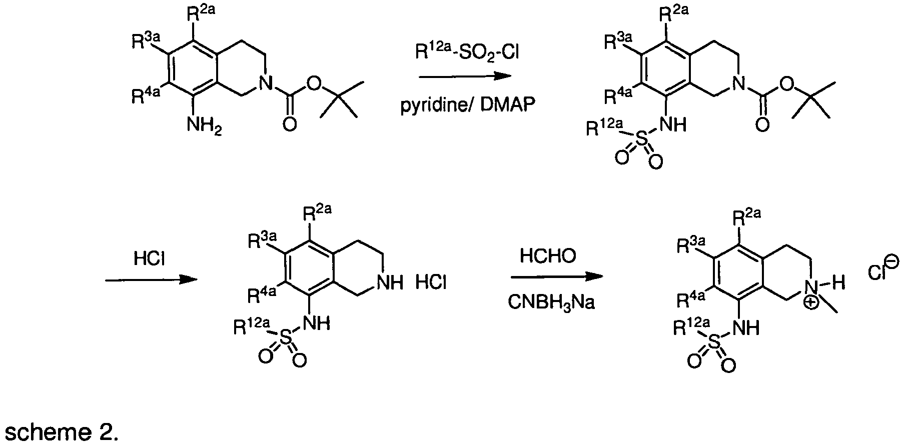

### Preparation of substituted tetrahydroisoquinoline compounds of general formula Ib (scheme 3)

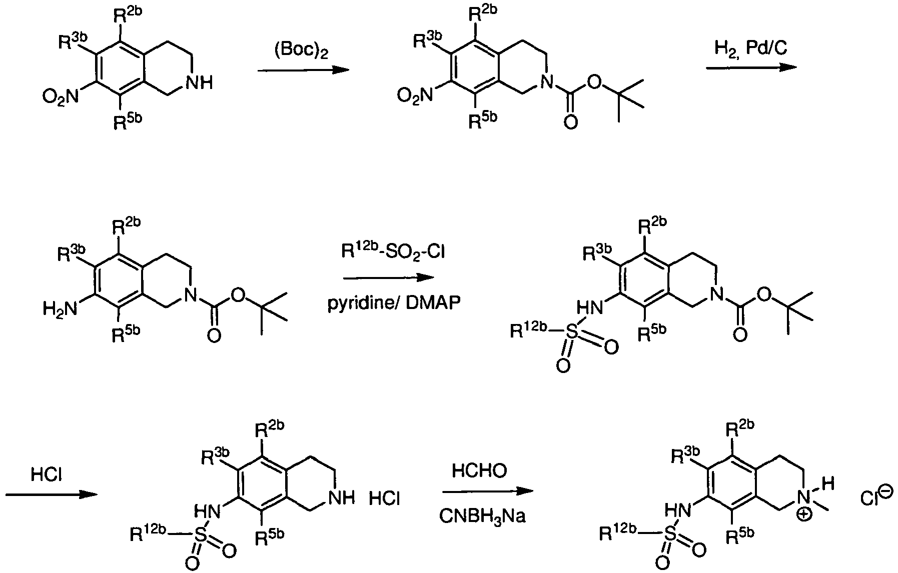

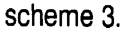

### Preparation of substituted tetrahydroisoquinoline compounds of general formula Ic (scheme 4, 5 and 6)

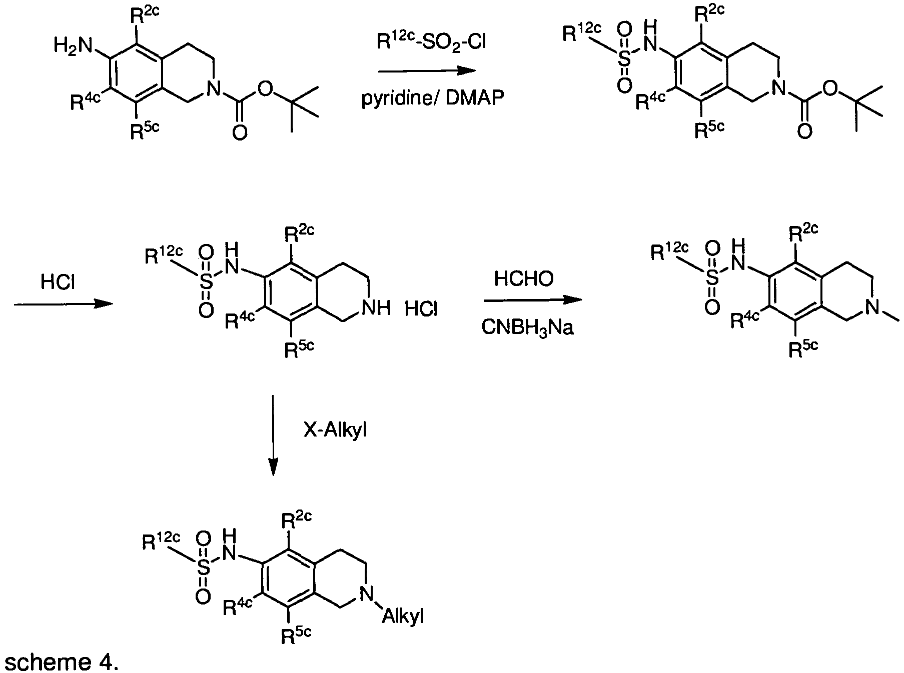

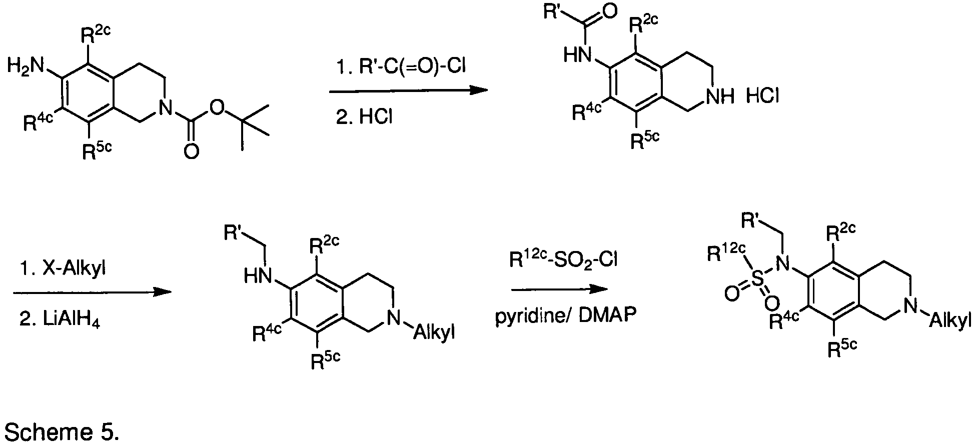

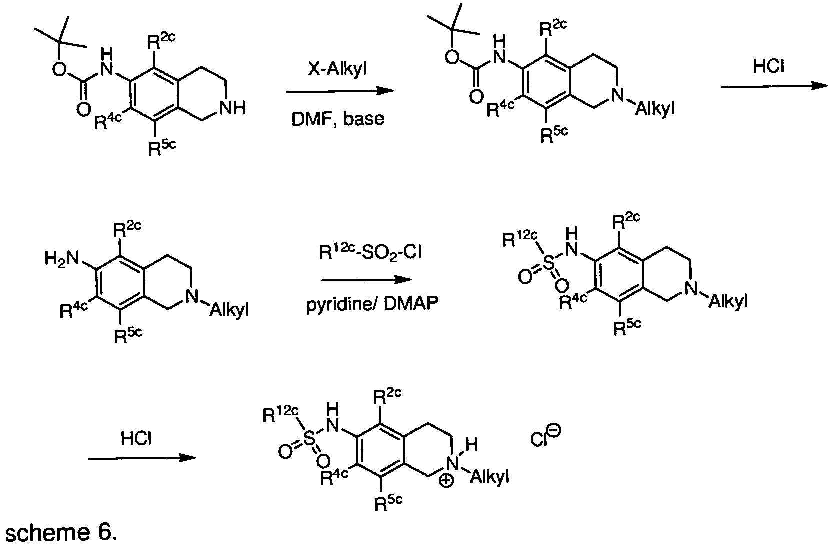

### Preparation of substituted tetrahydroisoquinoline compounds of general formula Id (scheme 7)

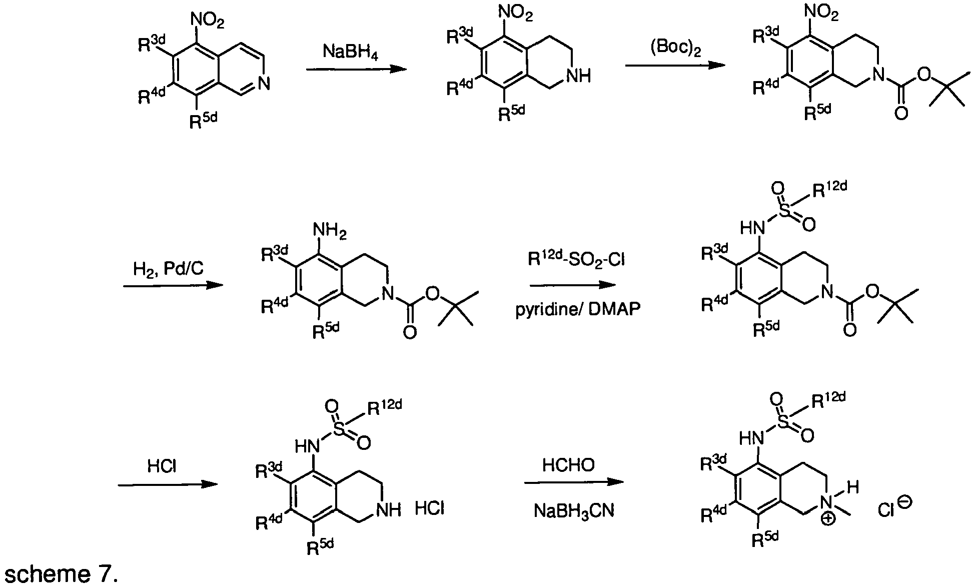

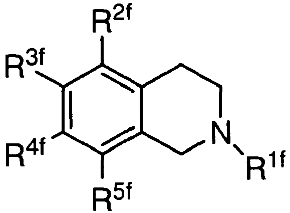

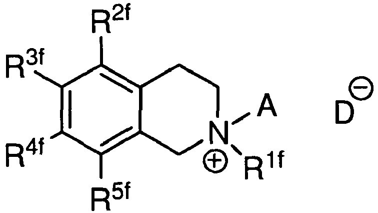

In still another aspect the present invention relates to a process for the preparation of a salt of a substituted tetrahydroisoquinoline compound of general formula Ih, wherein at least one compound of general formula VIhA, 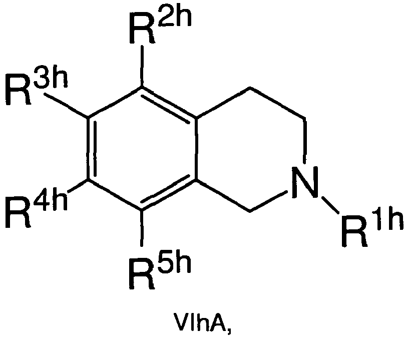 wherein R^{1h} to R^{5h} have the meaning given above, preferably with the proviso that at least one substituent of the group consisting of R^{2h}, R^{3h}, R^{4h} and R^{5h} represents a - NR^{11h}-S(=O)₂-R^{12h} moiety, wherein R^{11h} and R^{12h} have the meaning given above, is reacted with at least one compound of general formula A^{h}-D^{h}, wherein A^{h} and D^{h} have the meaning given above, in a reaction medium, preferably in a reaction medium selected from the group consisting of acetone, tetrahydrofurane, water, ethyl acetate, chloroform, acetonitrile, dichloromethane and mixtures thereof, to yield at least one salt of general formula Ih, 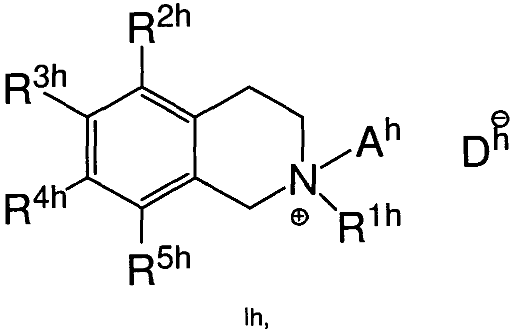 wherein A^{h}, D^{h} and R^{1h} to R^{5h} have the meaning given above with the proviso that at least one substituent of the group consisting of R^{2h}, R^{3h}, R^{4h} and R^{5h} represents a - NR^{11h}-S(=O)₂-R^{12h} moiety, wherein R^{11h} and R^{12h} have the meaning given above.

The term "salt" is to be understood as meaning any form of the substituted tetrahydroisoquinoline compounds of general formula I in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the substituted tetrahydroisoquinoline compounds of general formula I or in each case of corresponding stereoisomers may also be obtained by standard procedures known to those skilled in the art.

The term "solvate" according to this invention is to be understood as meaning any form of the substituted tetrahydroisoquinoline compounds of general formula I in which they have attached to it via non-covalent binding another molecule (most likely a polar solvent) especially including hydrates and alcoholates, e.g. methanolate.

In the following methods for determining the pharmacological activity of the substituted tetrahydroisoquinoline compounds are described.

### Pharmacological Methods:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40. dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 2.9 µg membrane protein), and is prolonged for 60 minutes at a temperature of 37 ºC. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted tetrahydroisoquinoline compounds according to the present invention in fasted rats is then determined as follows:
The rats were fasted for 23 hours in their single homecages. After this period, the rats are orally or intraperitoneally dosed with a composition comprising a substituted tetrahydroisoquinoline compound or a corresponding composition (vehicle) without said substituted tetrahydroisoquinoline compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Preparation of example compound 15: 6-(4-Methyl-naphthalene-1-sulfonylamino)-3,4-dihydro-1H-isoquinoline-2 -carboxylic acid tert-butyl ester

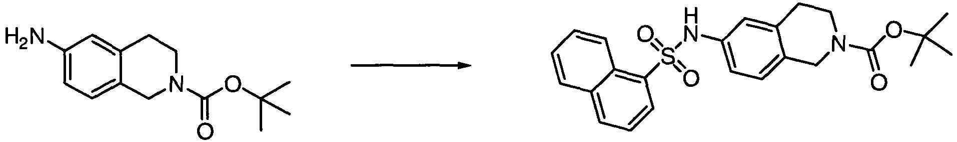

4-Methyl-naphthalene-1-sulfonyl chloride (310 mg, 1.288 mmol) was added to a solution of tert-butyl 6-amino-3,4-dihydroisoquinoline-2(1H)-carboxylate (318 mg, 1.28 mmol), pyridine (102 mg, 1.28 mmol) and dimethylaminopyridine (15 mg) in dichloromethane (50 mL). After stirring overnight at room temperature the mixture was washed with water, dried over sodium sulfate and filtered. The organic extracts were diluted with ethanol and partial evaporation afforded the title compound (435 mg, 74 %).

The example compounds 13, 14, 16, 17, 18 were prepared analogously to the process described for the preparation of example compound 15.

### Preparation of example compound 6: 4-Methyl-naphthalene-1-sulfonic acid (1,2,3,4-tetrahydroisoquinolin-6-yl)amide hydrochloride

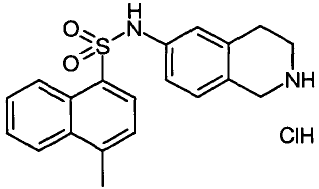

A solution of hydrogen chloride [2.0 M in diethylether] was added to a suspension of 6-(4-methyl-naphthalene-1-sulfonylamino)-3,4-dihydro-1H-isoquinoline-2-carboxylic acid tert-butyl ester (390 mg, 0.86mmol) in ethyl acetate (15 mL). Stirring was continued overnight at room temperature to precipitate the hydrochloride of 4-methyl-naphthalene-1-sulfonic acid (1,2,3,4-tetrahydro-isoquinolin-6-yl)amide (320 mg, 95 %).

The example compounds 1, 4, 8, 10, 11 and 12 were prepared analogously to the process described for the preparation of example compound 6.

### Preparation of example compound 7:

### 4-Methyl-naphthalene-1-sulfonic acid (2-methyl-1,2,3,4-tetrahydroiso quinolin-6-yl)amide hydrochloride

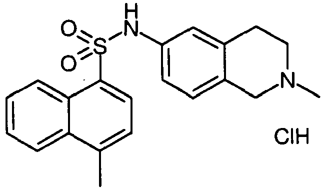

A solution of 4-methyl-naphthalene-1-sulfonic acid(1,2,3,4-tetrahydro-isoquinolin-6-yl)amide (254 mg, 0.72 mmol) in acetonitrile (15 mL) was treated with formaldehyde (37% aqueous solution, 1 mL) and stirred for 2 hours at room temperature. Sodium cyanoborohydride (210mg, 3.3 mmol) was added and the mixture was allowed to stir for 2 hours following addition of acetic acid (pH 7). After stirring overnight the mixture was evaporated and the residue partitioned between chloroform and water. The organic layer was separated and evaporated to give the crude free base which was purified by chromatography eluting with 2% methanol in chloroform. Treatment with a 2.0 M ethereal hydrogen chloride solution gave the title compound as the hydrochloride salt (104 mg, 41 %).

The example compounds 3, 4 and 9 were prepared analogously to the process described for the preparation of example compound 7.

### Preparation of example compound 2:

### 2,2-Dimethyl-6-[methyl-(naphthalene-1-sulfonyl)-amino]-1,2,3,4-tetrahydro isoquinolinium iodide

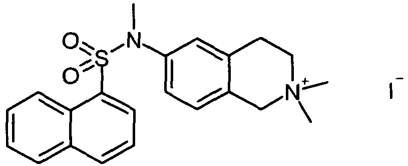

Naphthalene-1-sulfonic acid (1,2,3,4-tetrahydrisoquinolin-6-yl)-amide hydrochloride (100 mg, 0.26 mmol) and potassium carbonate( 111 mg, 0.80 mmol) were dissolved in acetone (20 ml) and methyl iodide ( 150mg,1.04mmol) was added. The mixture was heated under reflux for 6 hours. The solution was evaporated and the crude quaternary ammonium salt was crystallised by addition of ethanol ( 5 ml ) to afforded the title compound (120 mg, 91 %).

### Preparation of example compound 55:

### 4-Methyl-naphthalene-1-sulfonic acid (1,2,3,4-tetrahydro-isoquinolin-7 -yl)amide hydrochloride

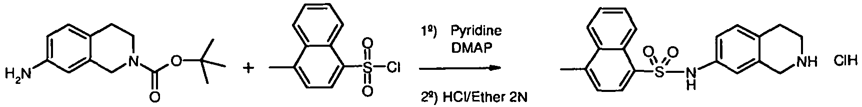

To a solution of 7-Amino-2-N-BOC-1,2,3,4-tetrahydroisoquinoline (318 mg, 1.28 mmol), pyridine (102 mg, 1.28 mmol) and dimethylaminopyridine (15 mg) in dichloromethane (50 mL) was added 4-methyl-naphthalene-1-sulfonyl chloride (310 mg, 1.288 mmol). After stirring overnight at room temperature the mixture was washed with water, dried over sodium sulfate and filtered. The organic extracts were diluted in ethyl acetate (15 mL) and a solution of hydrogen chloride 2,0 M in diethylether was added. Stirring was continued overnight at room temperature to precipitate the hydrochloride of 4-Methylnaphthalene-1-sulfonic acid (1,2,3,4-tetrahydro-isoquinolin-7-yl)amide (292mg, 83 %) [MH]⁺= 353.
¹H NMR (400 MHz, DMSO-*d*₆) δ ppm 2.67 (s, 3 H) 2.77 (t, *J*=6.06 Hz, 2 H) 3.21 (m, 2 H) 4.07 (br. s., 2 H) 6.89 (d, *J*=1.95 Hz, 1 H) 6.86 (s, 1 H) 6.94 - 7.00 (m, 1 H) 7.46 (d, *J*=7.42 Hz, 1 H) 7.65 - 7.76 (m, 2 H) 8.12 (t, *J*=7.23 Hz, 2 H) 8.72 (d, *J*=7.82 Hz, 1 H) 9.14 (br. s., 2 H) 10.72 (s, 1 H)

### Preparation of example compound 91:

### 4-Methyl-naphthalene-1-sulfonic acid (1,2,3,4-tetrahydro-isoquinolin-5 -yl)amide hydrochloride

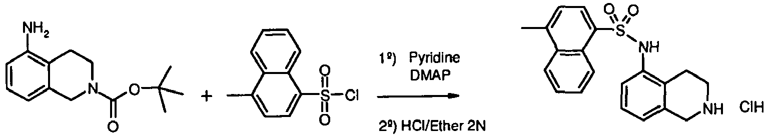

To a solution of 5-Amino-2-N-BOC-1,2,3,4-tetrahydroisoquinoline (318 mg, 1.28 mmol), pyridine (102 mg, 1.28 mmol) and dimethylaminopyridine (15 mg) in dichloromethane (50 mL) was added 4-methyl-naphthalene-1-sulfonyl chloride (310 mg, 1.288 mmol). After stirring overnight at room temperature the mixture was washed with water, dried over sodium sulfate and filtered. The organic extracts were diluted in ethyl acetate (15 mL) and a solution of hydrogen chloride 2,0 M in diethylether was added. Stirring was continued overnight at room temperature to precipitate the hydrochloride of 4-Methylnaphthalene-1-sulfonic acid (1,2,3,4-tetrahydro-isoquinolin-5-yl)amide (274mg, 78 %) [MH]⁺= 353.
¹H NMR (300 MHz, DMSO-*d*₆) δ ppm 2.72 (s, 3 H) 2.85 (br. s., 2 H) 3.16 (br. s., 2 H) 4.15 (br. s., 2 H) 6.65 (d, *J*=7.03 Hz, 1 H) 6.93 - 7.13 (m, 2 H) 7.45 (d, *J*=7.62 Hz, 1 H) 7.73 (d, *J*=2.93 Hz, 1 H) 7.71 (br. s., 1 H) 7.93 (d, *J*=7.32 Hz, 1 H) 8.19 (br. s., 1 H) 8.72 (d, *J*=6.44 Hz, 1 H) 9.15 (br. s., 2 H) 10.00 (s, 1 H)

### Preparation of example compound 140

### 4-Methyl-naphthalene-1-sulfonic acid (1,2,3,4-tetrahydro-isoquinolin-8 -yl)amide hydrochloride

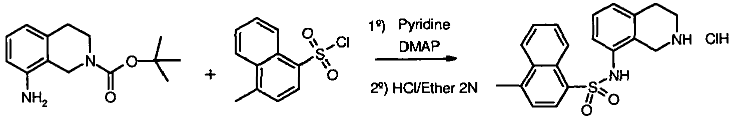

To a solution of 8-Amino-2-N-BOC-1,2,3,4-tetrahydroisoquinoline (318 mg, 1.28 mmol), pyridine (102 mg, 1.28 mmol) and dimethylaminopyridine (15 mg) in dichloromethane (50 mL) was added 4-methyl-naphthalene-1-sulfonyl chloride (310 mg, 1.288 mmol). After stirring overnight at room temperature the mixture was washed with water, dried over sodium sulfate and filtered. The organic extracts were diluted in ethyl acetate (15 mL) and a solution of hydrogen chloride 2,0 M in diethylether was added. Stirring was continued overnight at room temperature to precipitate the hydrochloride of 4-Methylnaphthalene-1-sulfonic acid (1,2,3,4-tetrahydro-isoquinolin-8-yl)amide (182 mg, 51 %). [MH]⁺= 353.

### Preparation of example compound 155

### Naphthalene-2-sulfonic acid (2-cyclopropanecarbonyl-1,2,3,4-tetrahydro-isoquinolin-6-yl)amide

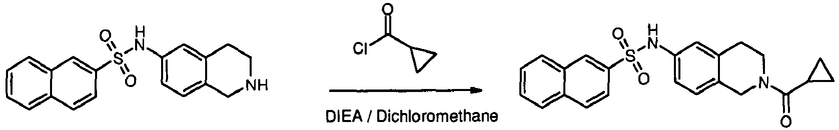

To a solution of naphthalene-2-sulfonic acid (1,2,3,4-tetrahydroisoquinoli-6-yl)amide (338 mg, 1 mmol) in dichloromethane (50 mL), N-ethyldiisopropylamine (194 mg, 1,50 mmol) and cyclopropanecarbonyl chloride (105 mg, 1 mmol) were added. After stirring overnight at room temperature the mixture was washed with water, dried over sodium sulfate and filtered, and recristallized in etanol to gave Naphthalene-2-sulfonic acid (2-cyclopropanecarbonyl-1,2,3,4-tetrahydro-isoquinolin-6-yl)amide (310 mg, 76%) [MH]⁺= 407
¹H NMR (300 MHz, DMSO-*d₆*) δ ppm 0.68 (m, 4 H) 1.95 (br. s., 1 H) 2.60 (br. s., 1 H) 2.72 (br.s., 1 H) 3.54 (br. s., 1 H) 3.75 (br. s., 1 H) 4.41 (br. s., 1 H) 4.69 (br. s., 1 H) 6.86 - 7.02 (m, 3 H) 7.65 (td, *J*=7.76, 1.32 Hz, 2 H) 7.76 (dd, *J*=8.72, 1.39 Hz, 1 H) 7.98 (d, *J*=7.76 Hz, 1 H) 8.01 - 8.15 (m, 2 H) 8.43 (s, 1 H) 10.25 (br. s., 1 H)

### Preparation of example compound 156

### Naphthalene-2-sulfonic acid (2-cyclopropylmethyl-1,2,3,4-tetrahydroisoquinolin 6-yl) amide hydrochloride

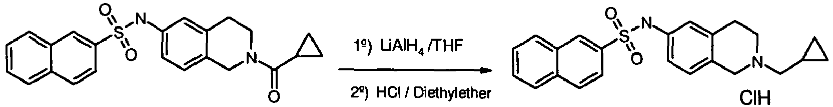

To a solution of lithium aluminium hydride 1.0 M in tetrahydrofuran (5 mL,5 mmol) under nitrogen, naphthalene-2-sulfonic acid (2-cyclopropanecarbonyl -1,2,3,4-tetrahydro-isoquinolin-6-yl)amide (200 mg, 0.49 mmol) was added. The reaction mixture was heated to reflux for 3 hours, and then at room temperatura overnight.
After cooling to 0 ºC, water was added and the mixture was filtered.The filtrate was extracted with dichloromethane and the organic layer was washed with saturated NaCl, dried and concentrated in vacuo. The resulting residue was chromatografed on silica gel with CHCl₃-MeOH (98:2). To a solution of Naphthalene-2-sulfonic acid (2-cyclopropylmethyl-1,2,3,4-tetrahydroisoquinolin 6-yl) amide in ethyl acetate ( 3 mL) a solution of hydrogen chloride 2,0 M in diethylether (2mL) was added. Stirring was continued overnight at room temperature to precipitate the hydrochloride of naphthalene-2-sulfonic acid (2-cyclopropylmethyl-1,2,3,4-tetrahydroisoquinolin-6-yl) amide (126 mg, 60 %) [MH]⁺= 394
¹H NMR (300 MHz, DMSO-*d*₆) δ ppm 0.38 (m, 2 H) 0.62 (m, 2 H) 1.06 - 1.19 (m, 1 H) 2.86 - 3.12 (m, 4 H) 3.19 (m, 1 H) 3.61 (m, 1 H) 4.15 (dd, *J*=15.09, 7.47 Hz, 1 H) 4.34 - 4.45 (m, 1 H) 6.99 - 7.08 (m, 3 H) 7.68 (td, *J*=7.47, 1.39 Hz, 2 H) 7.81 (dd, *J*=8.72, 1.83 Hz, 1 H) 8.01 (d, *J*=7.76 Hz, 1 H) 8.15 (d, *J*=7.47 Hz, 1 H) 8.10 (d, J=8.72 Hz, 1 H) 8.49 (d, *J*=1.32 Hz, 1 H) 10.49 (br.s., 1 H) 10.58 (s, 1 H)

| **N°** | **STRUCTURE** | **Autonom** | **¹H-NMR** | **MS (APCI (M+H)⁺)** |
|---|---|---|---|---|
| 1 | 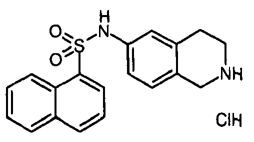 | Naphthalene-1-sulfonic acid (1,2,3,4-tetrahydroisoquinolin-6-yl)-amide hydrochloride | ¹H NMR (300 MHz, DMSO-*d₆*) δ ppm 2.73 - 2.84 (m, 2 H) 3.19 (s, 2 H) 4.02 (s, 2 H) 6.83 - 6.92 (m, 2 H) 6.92 - 6.99 (m, 1 H) 7.56 - 7.69 (m, 2 H) 7.69 - 7.77 (m, 1 H) 8.05 (d, *J*=8.06 Hz, 1 H) 8.16 - 8.24 (m, 2 H) 8.71 (d, *J*=8.49 Hz, 1 H) 9.23 (s, 2 H) 10.79 (s, 1 H) | 339 |
| 2 | 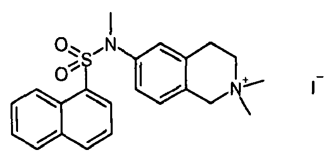 | 2,2-Dimethyl-6-[methyl-(naphthalene-1-sulfonyl)-amino]-1,2,3,4-tetrahydro isoquinolinium iodide | ¹H NMR (300 MHz, DMSO-*d₆*) δ ppm 2.95 - 3.03 (m, 2 H) 3.11 (s, 6 H) 3.15 (s, 3 H) 3.56 - 3.68 (m, 2 H) 4.56 (s, 2 H) 7.01 - 7.13 (m, 3 H) 7.42 - 7.51 (m, 1 H) 7.56 - 7.64 (m, 1 H) 7.64 - 7.71 (m, 1 H) 8.03 - 8.15 (m, 3 H) 8.29 (d, *J*=8.20 Hz, 1 H) | 381 |
| 3 | 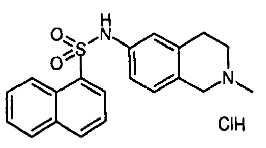 | Naphthalene-1-sulfonic acid (2-methyl-1,2,3,4-tetrahydro-isoquinolin-6-yl)-amide hydrochloride | ¹H NMR (300 MHz, DMSO-*d*₆) δ ppm 2.52 (s, 3 H) 2.73 - 2.86 (m, 2 H) 3.02 - 3.12 (m, 2 H) 3.79 - 3.95 (m, 2 H) 6.82 - 6.95 (m, 3 H) 7.57 - 7.76 (m, 3 H) 8.06 (d, *J*=7.76 Hz, 1 H) 8.17 - 8.25 (m, 2 H) 8.69 (d, *J*=8.50 Hz, 1 H) 10.72 (s, 1 H) | 353 |
| 4 | 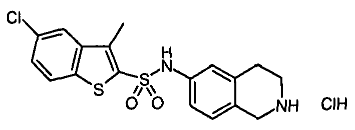 | 5-Chloro-3-methyl-benzo[b]thiophene-2-sulfonic acid (1,2,3,4-tetrahydro-isoquinolin-6-yl)-amide hydrochloride | ¹H NMR (300 MHz, DMSO-d₆) δ ppm 2.53 (s, 3 H) 2.83 - 2.91 (m, 2 H) 3.20 - 3.29 (m, 2 H) 4.10 (s, 2 H) 6.95 - 7.05 (m, 2 H) 7.05 - 7.11 (m, 1 H) 7.52 - 7.59 (m, 1 H) 8.00 (d, *J*=1.90 Hz, 1 H) 8.06 (d, *J*=8.64 Hz, 1 H) 9.27 (s, 2 H) 10.89 (s, 1 H) | 393 |
| 5 | 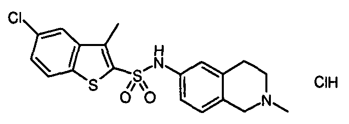 | 5-Chloro-3-methyl-benzo[b]thiophene-2-sulfonic acid (2-methyl-1,2,3,4-tetrahydro-isoquinolin-6-yl)-amide hydrochloride | ¹H NMR (300 MHz, DMSO-*d*₆) δ ppm 2.52 (s, 3 H) 2.80 (s, 3 H) 2.96 (s, 2 H) 3.48 (m, 2 H) 4.21 (s, 2 H) 6.93 - 7.12 (m, 3 H) 7.48 - 7.63 (m, 1 H) 7.99 (d, *J*=1.90 Hz, 1 H) 8.05 (d, *J*=8.64 Hz, 1 H) 10.67 (s, 1 H) 10.90 (s, 1 H) | 407 |
| 6 | 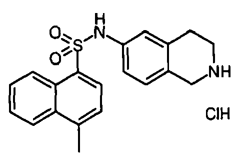 | 4-Methyl-naphthalene-1-sulfonic acid (1,2,3,4-tetrahydro-isoquinolin-6-yl)amide hydrochloride | ¹H NMR (300 MHz, DMSO-*d*₆) δ ppm 2.67 (s, 3 H) 2.72 - 2.84 (m, 2 H) 3.19 (s, 2 H) 4.02 (s, 2 H) 6.78 - 6.91 (m, 2 H) 6.91 - 7.00 (m, 1 H) 7.46 (d, *J*=7.91 Hz, 1 H) 7.60 - 7.77 (m, 2 H) 8.07 - 8.17 (m, 2 H) 8.67 - 8.78 (m, 1 H) 9.23 (s, 2 H) 10.74 (s, 1 H) | 353 |
| 7 | 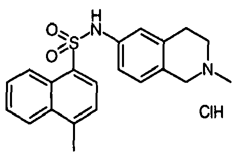 | 4-Methyl-naphthalene-1-sulfonic acid (2-methyl-1,2,3,4 -tetrahydro-isoqui-nolin-6-yl)amide hydrochloride | ¹H NMR (300 MHz, DMSO-*d₆*) δ ppm 2.67 (s, 3 H) 2.77 (s, 3 H) 2.97 (m, 2 H) 3.46 (m, 2 H) 4.13 (m, 2 H) 6.82 - 6.97 (m, 3 H) 7.47 (d, *J*=7.62 Hz, 1 H) 7.61 - 7.78 (m, 2 H) 8.12 (d, *J*=7.47 Hz, 2 H) 8.72 (d, *J*=7.91 Hz, 1 H) 10.61 (s, 1 H) 10.78 (s, 1 H) | 367 |
| 8 | 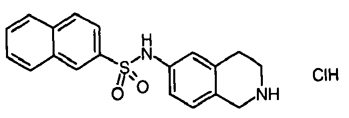 | Naphthalene-2-sulfonic acid (1,2,3,4-tetrahydro-isoquinolin-6-yl)-amide hydrochloride | ¹H NMR (300 MHz, DMSO-d₆) δ ppm 2.80 - 2.89 (m, 2 H) 3.22 (t, *J*=6.15 Hz, 2 H) 4.05 (s, 2 H) 6.89 - 7.07 (m, 3 H) 7.59 - 7.72 (m, 2 H) 7.73 - 7.82 (m, 1 H) 7.99 (d, *J*=7.62 Hz, 1 H) 8.03 - 8.16 (m, 2 H) 8.47 (d, *J*=0.59 Hz, 1 H) 9.22 (s, 2 H) 10.52 (s, 1 H) | 339 |
| 9 | 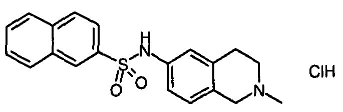 | Naphthalene-2-sulfonic acid (2-methyl-1,2,3,4-tetrahydro-isoquinolin-6-yl)-amide hydrochloride | ¹H NMR (400 MHz, DMSO-*d₆*) δ ppm 2.79 (s, 3 H) 2.95 (m, 2 H) 3.23 (m, 2 H) 4.09 - 4.25 (m, 2 H) 6.97 - 7.03 (m, 3 H) 7.61 - 7.70 (m, 2 H) 7.78 (dd, *J*=8.60, 1.95 Hz, 1 H) 7.99 (d, *J*=7.82 Hz, 1 H) 8.08 (d, *J*=8.99 Hz, 1 H) 8.13 (d, *J*=8.21 Hz, 1 H) 8.47 (s, 1 H) 10.37 (s, 1 H) 10.52 (s, 1 H) | 353 |
| 10 | 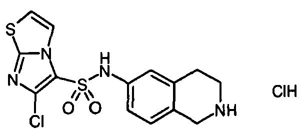 | 6-Chloro-imidazo[2,1-b]thiazole-5-sulfonic acid (1,2,3,4-tetrahydro-isoquinolin-6-yl)-amide hydrochloride | ¹H NMR (300 MHz, DMSO-*d₆*) δ ppm 2.80 - 2.91 (m, 2 H) 3.25 (m, 2 H) 4.10 (s, 2 H) 6.93 - 6.99 (m, 2 H) 7.04 - 7.11 (m, 1 H) 7.65 (d, *J*=4.39 Hz, 1 H) 8.07 (d, *J=*4.39 Hz, 1 H) 9.31 (s, 2 H) 11.18 (s, 1 H) | 369 |
| 11 | 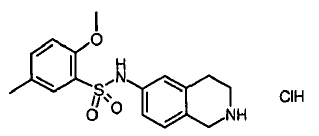 | 2-Methoxy-5-methyl-N-(1,2,3,4-tetrahydro-isoquinolin-6-yl)-benzene sulfonamide hydrochloride | ¹H NMR (300 MHz, DMSO-*d₆*) δ ppm 2.22 (s, 3 H) 2.80 - 2.88 (m, 2 H) 3.24 (t, *J*=6.15 Hz, 2 H) 3.81 (s, 3 H) 4.07 (s, 2 H) 6.87 - 6.97 (m, 2 H) 6.98 - 7.05 (m, 2 H) 7.30 - 7.37 (m, 1 H) 7.55 (d, *J*=1.90 Hz, 1 H) 9.28 (s, 2 H) 9.99 (s, 1 H) | 333 |
| 12 | 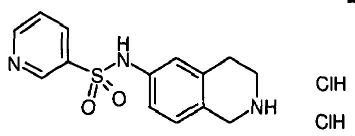 | Pyridine-3-sulfonic acid (1,2,3,4-tetrahydro-isoquinolin-6-yl)-amide dihydrochloride | ¹H NMR (300 MHz, DMSO-*d₆*) δ ppm 2.90 (t, *J*=6.01 Hz, 2 H) 3.27 (s, 2 H) 4.13 (s, 2 H) 6.93 - 7.05 (m, 2 H) 7.05 - 7.14 (m, 1 H) 7.61 (dd, *J*=8.06, 4.83 Hz, 1 H) 8.10 - 8.22 (m, 1 H) 8.78 (dd, *J*=4.83, 1.46 Hz, 1 H) 8.89 (d, *J*=2.05 Hz, 1 H) 9.31 (s, 2 H) 10.66 (s, 1 H) | 290 |
| 13 | 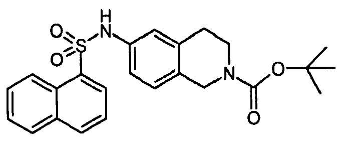 | 6-(Naphthalene-1-sulfonylamino)-3,4-dihydro-1H-isoquinoline-2-carboxylic acid tert-butyl ester | ¹H NMR (300 MHz, DMSO-*d*₆) δ ppm 1.35 (s, 9 H) 2.55 (t, *J*=5.71 Hz, 2 H) 3.38 (t, *J*=5.86 Hz, 2 H) 4.27 (s, 2 H) 6.72 - 6.85 (m, 2 H) 6.85 - 6.96 (m, 1 H) 7.52 - 7.78 (m, 3 H) 8.05 (d, *J*=7.91 Hz, 1 H) 8.11 - 8.23 (m, 2 H) 8.69 (d, *J=*8.35 Hz, 1 H) 10.59 (s, 1 H) | 439 |
| 14 | 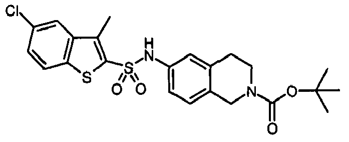 | 6-(5-Chloro-3-methyl-benzo[b]thiophene-2-sulfonylamino)-3,4-dihydro-1H-isoquinoline-2-carboxylic acid tert-butyl ester | ¹H NMR (300 MHz, DMSO-*d*₆) δ ppm 1.37 (s, 9 H) 2.50 (s, 3 H) 2.63 (t, *J*=5.71 Hz, 2 H) 3.44 (t, *J*=5.86 Hz, 2 H) 4.34 (s, 2 H) 6.91 - 6.96 (m, 2 H) 6.99 - 7.05 (m, 1 H) 7.51 - 7.57 (m, 1 H) 7.98 (d, *J*=2.05 Hz, 1 H) 8.04 (d, *J*=8.79 Hz, 1 H) 10.69 (s, 1 H) | 493 |
| 15 | 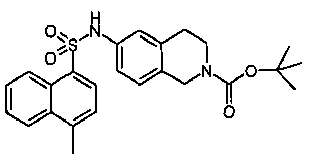 | 6-(4-Methyl-naphthalene-1-sulfonylamino)-3,4-dihydro-1H-isoquinoline-2-carboxylic acid tert-butyl ester | ¹H NMR (300 MHz, DMSO-*d*₆) δ ppm 1.35 (s, 9 H) 2.55 (t, *J*=5.71 Hz, 2 H) 2.67 (s, 3 H) 3.39 - 3.48 (m, 2 H) 4.27 (s, 2 H) 6.75 - 6.86 (m, 2 H) 6.86 - 6.94 (m, 1 H) 7.45 (d, *J*=7.76 Hz, 1 H) 7.61 - 7.76 (m, 2 H) 8.04 - 8.17 (m, 2 H) 8.65 - 8.79 (m, 1 H) 10.57 (s, 1 H) | 453 |
| 16 | 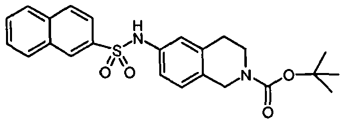 | 6-(Naphthalene-2-sulfonylamino)-3,4-dihydro-1H-isoquinoline-2-carboxylic acid tert-butyl ester | ¹H NMR (300 MHz, DMSO-*d*₆) δ ppm 1.35 (s, 9 H) 2.60 (t, *J*=5.71 Hz, 2 H) 3.40 (t, *J*=5.86 Hz, 2 H) 4.30 (s, 2 H) 6.86 - 7.00 (m, 3 H) 7.59 - 7.71 (m, 2 H) 7.75 (dd, *J*=8.64, 1.61 Hz, 1 H) 7.98 (d, *J*=7.76 Hz, 1 H) 8.07 (d, *J*=8.64 Hz, 1 H) 8.11 (d, *J*=7.76 Hz, 1 H) 8.42 (s, 1 H) | 439 |
| 17 | 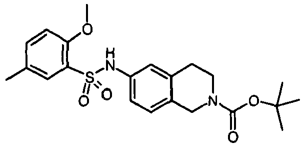 | 6-(2-Methoxy-5-methyl-benzenesulfonylamino)-3,4-dihydro-1H-isoquinoline-2-carboxylic acid tert-butyl ester | ¹H NMR (300 MHz, DMSO-*d*₆) δ ppm 1.37 (s, 9 H) 2.21 (s, 3 H) 2.55 - 2.65 (m, 2 H) 3.43 (t, *J*=5.86 Hz, 2 H) 3.82 (s, 3 H) 4.32 (s, 2 H) 6.82 - 6.91 (m, 2 H) 6.91 - 6.98 (m, 1 H) 7.03 (d, *J*=8.50 Hz, 1 H) 7.33 (dd, *J*=8.50, 2.05 Hz, 1 H) 7.52 (d, *J*=1.90 Hz, 1 H) 9.84 (s, 1 H) | 433 |
| 18 | 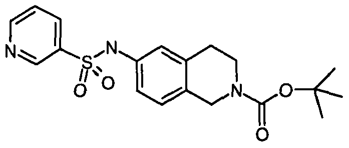 | 6-(Pyridine-3-sulfonylamino)-3,4-dihydro-1H-isoquinoline-2-carboxylic acid tert-butyl ester | ¹H NMR (300 MHz, DMSO-*d*₆) δ ppm 1.38 (s, 9 H) 2.62 (t, *J*=5.71 Hz, 2 H) 3.39 - 3.49 (m, 2 H) 4.35 (s, 2 H) 6.77 - 6.93 (m, 2 H) 7.03 (d, *J*=8.94 Hz, 1 H) 7.62 (d, *J*=4.39 Hz, 1 H) 7.95 (d, *J*=4.54 Hz, 1 H) 10.86 (s, 1 H) | 469 |
| 19 | 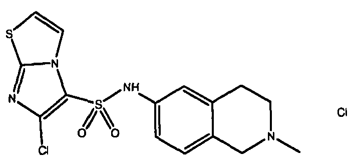 | 6-Chloro-imidazo[2,1-b]thiazole-5-sulfonic acid (2-methyl-1,2,3,4tetrahydro-isoquinolin-6-yl)-amide hydrochloride | | 383 |
| 20 | 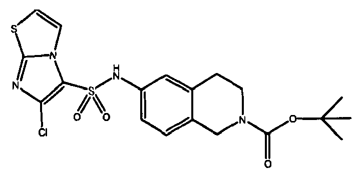 | 6-(6-Chloro-imidazo[2,1-b]thiazole-5-sulfonylamino)-3,4-dihydro-1Hisoquinoline-2-carboxylic acid tert-butyl ester | | 469 |
| 21 | 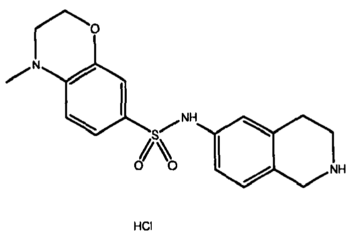 | 4-Methyl-3,4-dihydro-2H-benzo[1,4]oxazine-7-sulfonic acid (1,2,3,4-tetrahydro-isoquinolin-6-yl)-amide hydrochloride | | 360 |
| 23 | 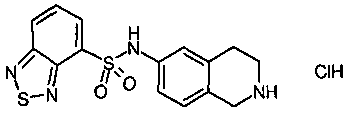 | Benzo[1,2,5]thiadiazol e-4-sulfonic acid (1,2,3,4-tetrahydro-isoquinolin-6-yl)-amide hydrochloride | | 347 |
| 24 | 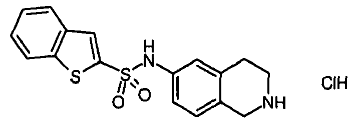 | Benzo[b]thiophene-2-sulfonic acid (1,2,3,4-tetrahydro-isoquinolin-6-yl)-amide hydrochloride | | 345 |
| 25 | 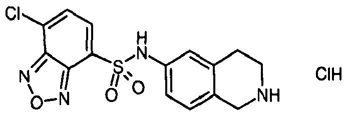 | 7-Chloro-benzo[1,2,5]oxadiazole-4-sulfonic acid (1,2,3,4-tetrahydro-isoquinolin-6-yl)-amide hydrochloride | | 365 |
| 26 | 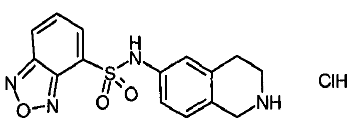 | Benzo[1,2,5]oxadiazole-4-sulfonic acid (1,2,3,4-tetrahydro-isoquinolin-6-yl)-amide hydrochloride | | 331 |
| 27 | 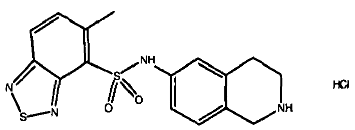 | 5-Methyl-benzo[1,2,5]thiadiazole-4-sulfonic acid (1,2,3,4-tetrahydro-isoquinolin-6-yl)-amide hydrochloride | | 361 |
| 28 | 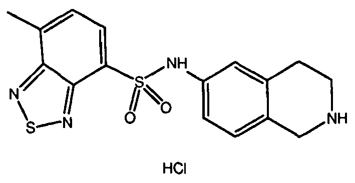 | 7-Methyl-benzo[1,2,5]thiadiazole-4-sulfonic acid (1,2,3,4-tetrahydro-isoquinolin-6-yl)-amide hydrochloride | | 361 |
| 29 | 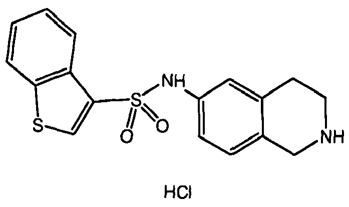 | Benzo[b]thiophene-3-sulfonic acid (1,2,3,4-tetrahydro-isoquinolin-6-yl)-amide hydrochloride | | 345 |
| 30 | 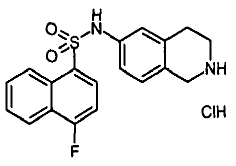 | 4-Fluoro-naphthalene-1-sulfonic acid (1,2,3,4-tetrahydro-isoquinolin-6-yl)-amide hydrochloride | | 357 |
| 31 | 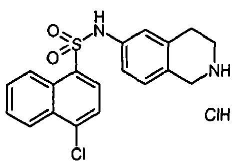 | 4-Chloro-naphthalene-1-sulfonic acid (1,2,3,4-tetrahydro-isoquinolin-6-yl)-amide hydrochloride | | 373 |
| 32 | 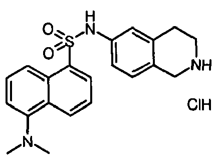 | 5-Dimethylaminonaphthalene-1-sulfonic acid (1,2,3,4-tetrahydro-isoquinolin-6-yl)-amide hydrochloride | | 382 |
| 33 | 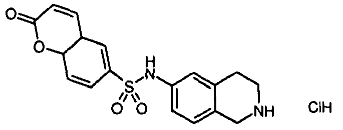 | 2-Oxo-4a,8a-dihydro-2H-chromene-6-sulfonic acid (1,2,3,4-tetrahydro-isoquinolin-6-yl)-amide hydrochloride | | 359 |
| 34 | 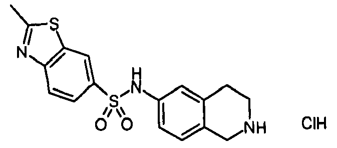 | 2-Methyl-benzothiazole-6-sulfonic acid (1,2,3,4-tetrahydro-isoquinolin-6-yl)-amide hydrochloride | | 360 |
| 35 | 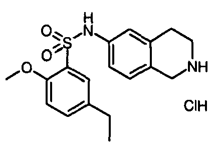 | 5-Ethyl-2-methoxy-N-(1,2,3,4-tetrahydro-isoquinolin-6-yl)-benzene sulfonamide hydrochloride | | 347 |
| 36 | 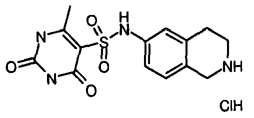 | 6-Methyl-2,4-dioxo-1,2,3,4-tetrahydro-pyrimidine-5-sulfonic acid (1,2,3,4-tetrahydro-isoquinolin-6-yl)-am ide hydrochloride | | 337 |
| 37 | 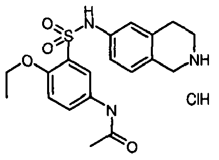 | N-[4-Ethoxy-3-(1,2,3,4-tetrahydro-isoquinolin-6-ylsulfamoyl)-phenyl]-acetamide hydrochloride | | 390 |
| 38 | 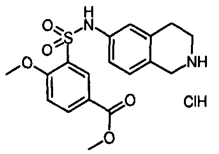 | 4-Methoxy-3-(1,2,3,4-tetrahydro-isoquinolin-6-yl sulfamoyl)-benzoic acid methyl ester hydrochloride | | 377 |
| 39 | 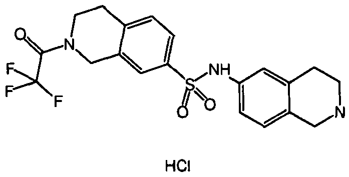 | 2-(2,2,2-Trifluoro-acetyl)-1,2,3,4-tetrahydro-isoquinoline-7-sulfonic acid (1,2,3,4-tetrahydro-isoquinolin-6-yl)-amide hydrochloride | | 440 |
| 40 | 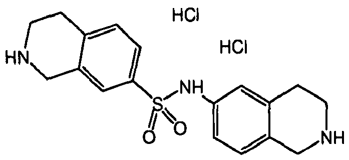 | 1,2,3,4-Tetrahydro-isoquinoline-7-sulfonic acid (1,2,3,4-tetrahydro-isoquinolin-6-yl)-amide dihydrochloride | | 344 |
| 41 | 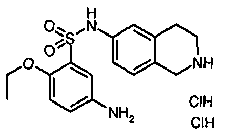 | 5-Amino-2-ethoxy-N-(1,2,3,4-tetrahydro-isoquinolin-6-yl)-benzenesulfon amide dihydrochloride | | 348 |
| 42 | 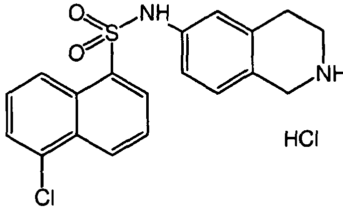 | 5-Chloro-naphthalene-1-sulfonic acid (1,2,3,4-tetrahydro-isoquinolin-6-yl)-amide hydrochloride | | 373 |
| 43 | 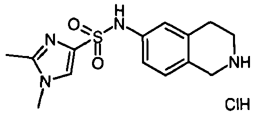 | 1,2-Dimethyl-1H-imidazole-4-sulfonic acid (1,2,3,4-tetrahydro-isoquinolin-6-yl)-amide hydrochloride | | 307 |
| 44 | 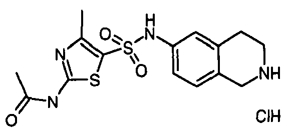 | N-[4-Methyl-5-(1,2,3,4-tetrahydro-isoquinolin-6-ylsulfamoyl)-thiazol-2-yl]-acetamide hydrochloride | | 367 |
| 45 | 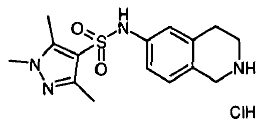 | 1,3,5-Trimethyl-1H-pyrazole -4-sulfonic acid (1,2,3,4-tetrahydro-isoquinolin-6-yl)-amide hydrochloride | | 321 |
| 46 | 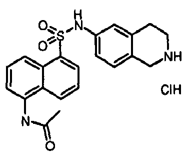 | N-[5-(1,2,3,4-Tetrahydro-isoquinolin-6-ylsulfamoyl)-naphthalen-1-yl]-acetamide hydrochloride | | 396 |
| 47 | 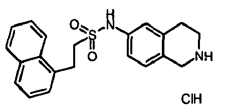 | 2-Naphthalen-1-yl-ethanesulfonic acid (1,2,3,4-tetrahydro-isoquinolin-6-yl)-amide hydrochloride | | 367 |
| 48 | 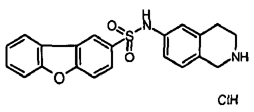 | Dibenzofuran-2-sulfonic acid (1,2,3,4-tetrahydro-isoquinolin-6-yl)-ami de hydrochloride | | 379 |
| 49 | 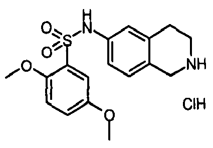 | 2,5-Dimethoxy-N-(1,2,3,4-tetrahydro-isoquinolin-6-yl)-benzenesulfonamide hydrochloride | | 349 |
| 50 | 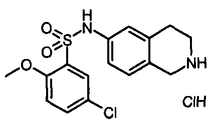 | 5-Chloro-2-methoxy-N-(1,2,3,4-tetrahydro-isoquinolin-6-yl)-benzene sulfonamide hydrochloride | | 353 |
| 51 | 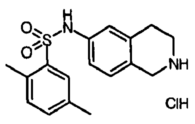 | 2,5-Dimethyl-N-(1,2,3,4-tetrahydro-isoquinolin-6-yl)-benzenesulfonamide hydrochloride | | 317 |
| 52 | 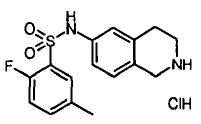 | 2-Fluoro-5-methyl-N-(1,2,3,4-tetrahydro-isoquinolin-6-yl)-benzene sulfonamide hydrochloride | | 321 |
| 53 | 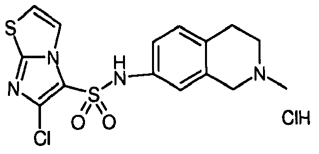 | 6-Chloro-imidazo[2,1-b]thiazole-5-sulfonic acid (2-methyl-1,2,3,4-tetrahydro-isoquinolin-7-yl)-amide hydrochloride | | 383 |
| 54 | 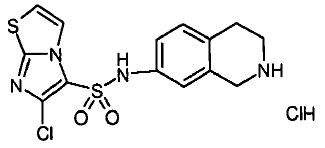 | 6-Chloro-imidazo[2,1-b]thiazole-5-sulfonic acid (1,2,3,4-tetrahydro-isoquinolin-7-yl)-amide hydrochloride | | 369 |
| 55 | 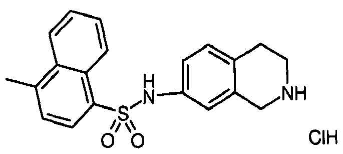 | 4-Methyl-naphthalene-1-sulfonic acid (1,2,3,4-tetrahydro-isoquinolin-7-yl)-amide hydrochloride | ¹H NMR (400 MHz, DMSO-*d₆*) δ ppm 2.67 (s, 3 H) 2.77 (t, *J*=6.06 Hz, 2 H) 3.21 (m, 2 H) 4.07 (br. s., 2 H) 6.89 (d, *J*=1.95 Hz, 1 H) 6.86 (s, 1 H) 6.94 - 7.00 (m, 1 H) 7.46 (d, *J*=7.42 Hz, 1 H) 7.65 - 7.76 (m, 2 H) 8.12 (t, *J*=7.23 Hz, 2 H) 8.72 (d, *J*=7.82 Hz, 1 H) 9.14 (br. s., 2 H) 10.72 (s, 1 H) | 353 |
| 56 | 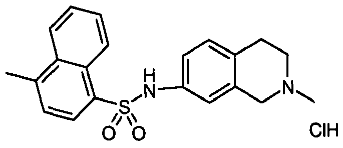 | 4-Methyl-naphthalene-1-sulfonic acid (2-methyl-1,2,3,4-tetrahydro-isoqui-nolin-7-yl)-amide hydrochloride | | 367 |
| 57 | 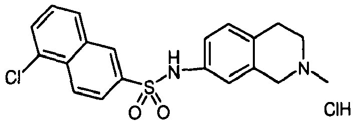 | 5-Chloro-naphthalene-2-sulfonic acid (2-methyl-1,2,3,4-tetrahydro-isoquinolin-7-yl)-amide hydrochloride | | 387 |
| 58 | 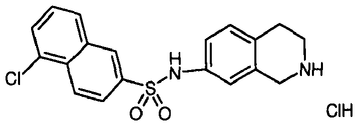 | 5-Chloro-naphthalene-2-sulfonic acid (1,2,3,4-tetrahydro-isoquinolin-7-yl)-amide hydrochloride | | 373 |
| 59 | 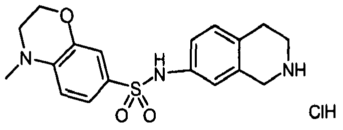 | 4-Methyl-3,4-dihydro-2H-benzo[1,4]oxazine-7-sulfonic acid (1,2,3,4-tetrahydro-isoquinolin-7-yl)-amide hydrochloride | | 360 |
| 60 | 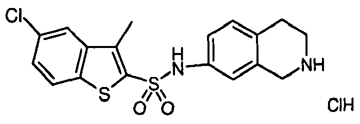 | 5-Chloro-3-methyl-benzo[b]thiophene -2-sulfonic acid (1,2,3,4-tetrahydro-isoquinolin-7-yl)-amide hydrochloride | | 393 |
| 61 | 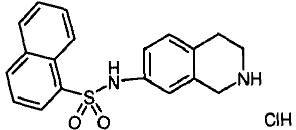 | Naphthalene-1-sulfonic acid (1,2,3,4-tetrahydro-isoquinolin-7-yl)-amide hydrochloride | | 339 |
| 63 | 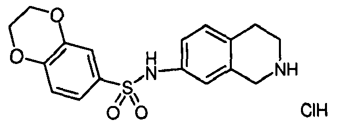 | 2,3-Dihydro-benzo[1,4]dioxine-6-sulfonic acid (1,2,3,4-tetrahydro-iso-quinolin-7-yl)-amide hydrochloride | | 347 |
| 64 | 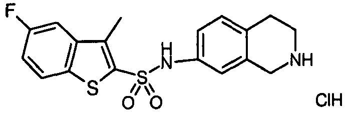 | 5-Fluoro-3-methyl-benzo[b] thiophene-2-sulfonic acid (1,2,3,4-tetrahydro-isoquinolin-7-yl)-amide hydrochloride | | 377 |
| 65 | 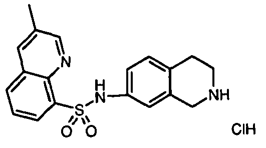 | 3-Methyl-quinoline-8-sulfonic acid (1,2,3,4-tetrahydro-isoquinolin-7-y l)-amide hydrochloride | | 354 |
| 66 | 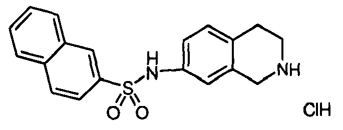 | Naphthalene-2-sulfonic acid (1,2,3,4-tetrahydro-isoquinolin-7-yl)-amide hydrochloride | | 339 |
| 67 | 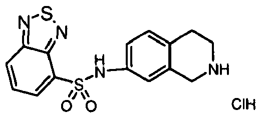 | Benzo[1,2,5]thiadiazol e-4-sulfonic acid (1,2,3,4-tetrahydro-isoquinolin-7-yl) -amide hydrochloride | | 347 |
| 68 | 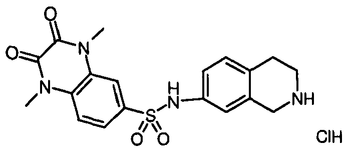 | 1,4-Dimethyl-2,3-dioxo-1,2,3,4-tetrahydro-quino xaline-6-sulfonic acid (1,2,3,4-tetrahydro-isoquinolin-7-yl)-amide hydrochloride | | 401 |
| 69 | 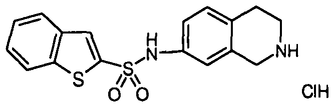 | Benzo[b]thiophene-2-sulfonic acid (1,2,3,4-tetrahydro -isoquinolin-7-yl)-amide hydrochloride | | 345 |
| 71 | 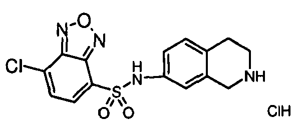 | 7-Chloro-benzo[1,2,5]oxadiazole-4-sulfonic acid (1,2,3,4-tetrahydro-isoquinolin-7-yl)-amide hydrochloride | | 365 |
| 72 | 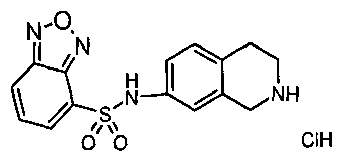 | Benzo[1,2,5]oxadiazol e-4-sulfonic acid (1,2,3,4-tetrahydro-isoquinolin-7-yl)-amide hydrochloride | | 331 |
| 73 | 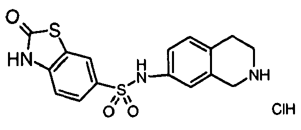 | 2-Oxo-2,3-dihydro-benzo thiazole-6-sulfonic acid (1,2,3,4-tetrahydro-isoquinolin-7-yl)-amide hydro- chloride | | 362 |
| 74 | 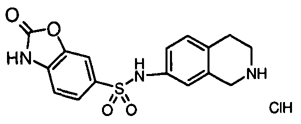 | 2-Oxo-2,3-dihydro-benzo oxazole-6-sulfonic acid (1,2,3,4-tetrahydro-iso quinolin-7-yl)-amide hydrochloride | | 346 |
| 76 | 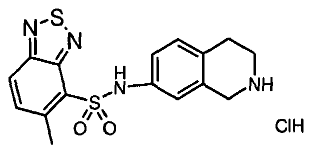 | 5-Methylbenzo[1,2,5] thiadiazole -4-sulfonic acid (1,2,3,4-tetrahydro-isoquinolin-7-yl)-amide hydrochloride | | 361 |
| 77 | 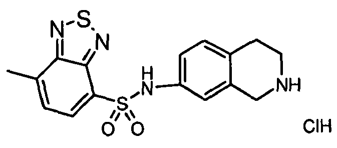 | 7-Methyl-benzo[1,2,5]thiadiazole-4-sulfonic acid (1,2,3,4-tetrahydro-isoquinolin-7-yl)-amide hydrochloride | | 361 |
| 78 | 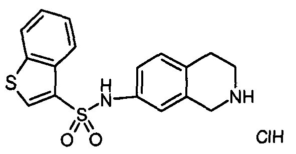 | Benzo[b]thiophene-3-sulfonic acid (1,2,3,4-tetrahydro-isoquinolin-7-yl)-amide hydrochloride | | 345 |
| 79 | 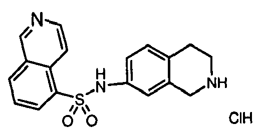 | Isoquinoline-5-sulfonic acid (1,2,3,4-tetrahydro -isoquinolin-7-yl)-ami de hydrochloride | | 340 |
| 80 | 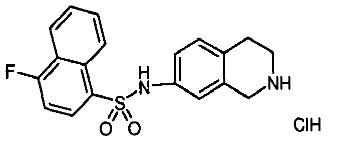 | 4-Fluoro-naphthalene-1-sulfonic acid (1,2,3,4-tetrahydro-isoquinolin-7-yl)-amide hydrochloride | | 357 |
| 81 | 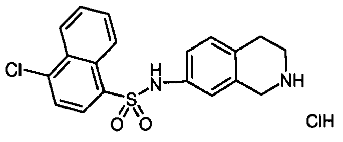 | 4-Chloro-naphthalene-1-sulfonic acid (1,2,3,4-tetrahydro-isoquinolin-7-yl)-amide hydrochloride | | 373 |
| 82 | 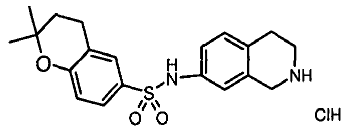 | 2,2-Dimethyl-chroman-6-sulfonic acid (1,2,3,4-tetrahydro-isoquinolin-7-yl)-amide hydrochloride | | 373 |
| 83 | 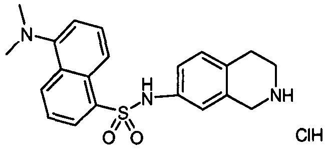 | 5-Dimethylamino-naphtha lene-1-sulfonic acid (1,2,3,4-tetrahydro-isoquinolin-7-yl)-amide hydrochloride | | 382 |
| 84 | 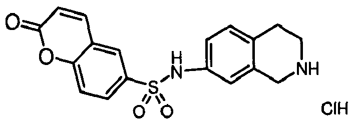 | 2-Oxo-2H-chromene-6-sulfonic acid (1,2,3,4-tetrahydro-isoquinolin-7-yl)-amide hydrochloride | | 357 |
| 85 | 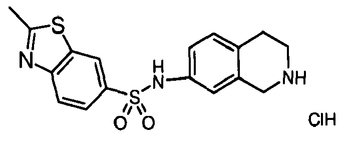 | 2-Methyl-benzothiazole-6-sulfonic acid (1,2,3,4-tetrahydro-isoquinolin-7-yl)-amide hydrochloride | | 360 |
| 86 | 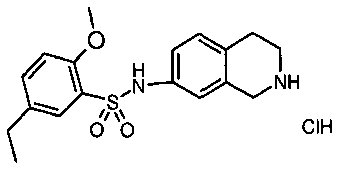 | 5-Ethyl-2-methoxy-N-(1,2,3,4-tetrahydro-isoquinolin-7-yl)-benzenesulfonamide hydrochloride | | 347 |
| 87 | 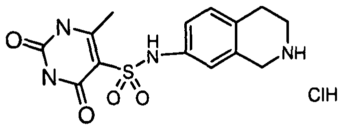 | 6-Methyl-2,4-dioxo-1,2,3,4-tetrahydro-pyrimidine- 5-sulfonic acid (1,2,3,4-tetrahydro-isoquinolin-7-yl)-amide hydrochloride | | 337 |
| 88 | 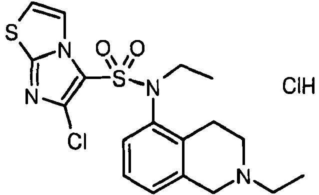 | 6-Chloro-imidazo[2,1-b]thiazole-5-sulfonic acid ethyl-(2-ethyl-1,2,3,4 -tetrahydro-isoquinolin-5-yl) -amide hydrochloride | | 425 |
| 89 | 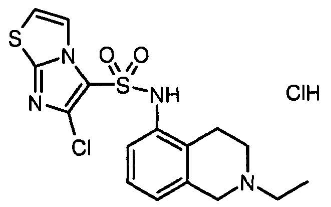 | 6-Chloro-imidazo[2,1-b] thiazole-5-sulfonic acid (2-ethyl-1,2,3,4-tetrahydro-isoquinolin-5-yl)-amide hydrochloride | | 397 |
| 90 | 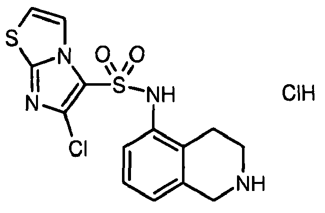 | 6-Chloro-imidazo[2,1-b] thiazole-5-sulfonic acid (1,2,3,4-tetrahydro-isoquinolin-5-yl)-amide hydrochloride | | 369 |
| 91 | 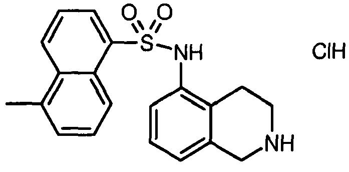 | 5-Methyl-naphthalene-1-sulfonic acid (1,2,3,4-tetrahydro-isoquinolin-5-yl)-amide hydrochloride | ¹H NMR (300 MHz, DMSO-d₆) δ ppm 2.72 (s, 3 H) 2.85 (br.s., 2 H) 3.16 (br. s., 2 H) 4.15 (br. s., 2 H) 6.65 (d, *J*=7.03 Hz, 1 H) 6.93 - 7.13 (m, 2 H) 7.45 (d, *J*=7.62 Hz, 1 H) 7.73 (d, *J*=2.93 Hz, 1 H) 7.71 (br. s., 1 H) 7.93 (d, *J*=7.32 Hz, 1 H) 8.19 (br. s., 1 H) 8.72 (d, *J*=6.44 Hz, 1 H) 9.15 (br. s., 2 H) 10.00 (s, 1 H) | 353 |
| 92 | 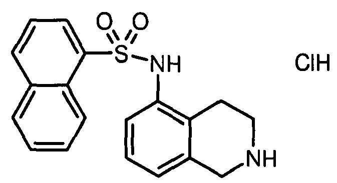 | Naphthalene-1-sulfonic acid (1,2,3,4-tetrahydro-isoquinolin-5-yl)-amide hydrochloride | | 339 |
| 93 | 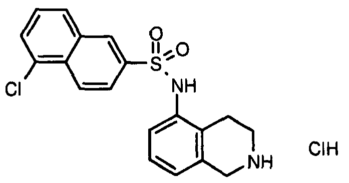 | 5-Chloro-naphthalene-2-sulfonic acid (1,2,3,4-tetrahydro-isoquinolin-5-yl)-amide hydrochloride | | 373 |
| 94 | 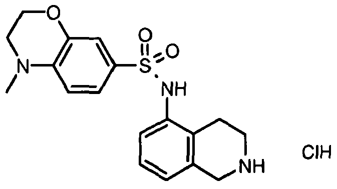 | 4-Methyl-3,4-dihydro-2H-benzo[1,4]oxazine-7- sulfonic acid (1,2,3,4-tetrahydro-isoquinolin-5-yl)-amide hydrochloride | | 360 |
| 95 | 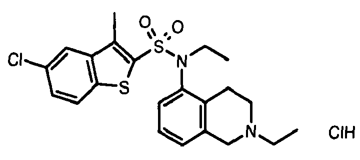 | 5-Chloro-3-methyl-benzo[b]thiophene-2-sulfonic acid ethyl-(2-ethyl-1,2,3,4-tetrahydro-isoquinolin-5-yl)-amide hydrochloride | | 449 |
| 96 | 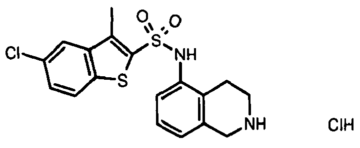 | 5-Chloro-3-methyl-benzo[b]thiophene-2-sulfonic acid (1,2,3,4-tetrahydro-isoquinolin-5-yl)-amide hydrochloride | | 393 |
| 98 | 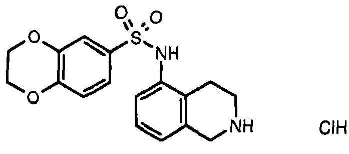 | 2,3-Dihydro-benzo[1,4]dioxine-6-sulfonic acid (1,2,3,4-tetrahydro-isoquinolin-5-yl)-amide hydrochloride | | 347 |
| 99 | 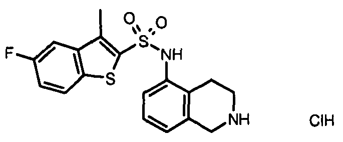 | 5-Fluoro-3-methyl-benzo[b]thiophene-2-sulfonic acid (1,2,3,4-tetrahydro-isoquinolin-5-yl)-amide hydrochloride | | 377 |
| 100 | 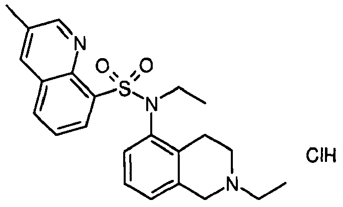 | 3-Methyl-quinoline-8-sulfonic acid ethyl-(2-ethyl -1,2,3,4-tetrahydro-isoquinolin-5-yl)-amide hydrochloride | | 410 |
| 101 | 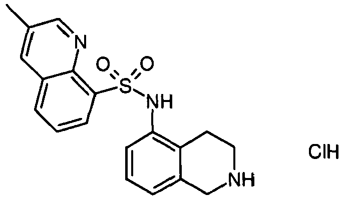 | 3-Methyl-quinoline-8-sulfonic acid (1,2,3,4-tetrahydro-isoquinolin-5-yl)-amide hydrochloride | | 354 |
| 102 | 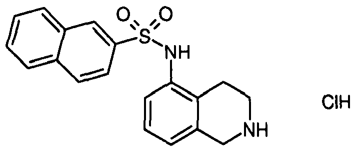 | Naphthalene-2-sulfonic acid (1,2,3,4-tetrahydro-isoquinolin-5-yl)-amide hydrochloride | | 339 |
| 103 | 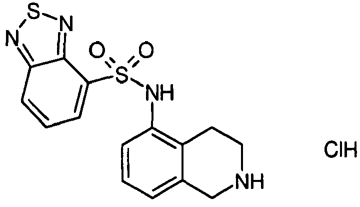 | Benzo[1,2,5]thiadiazol e-4-sulfonic acid (1,2,3,4-tetrahydro-isoquinolin-5-yl)-amide hydrochloride | | 347 |
| 104 | 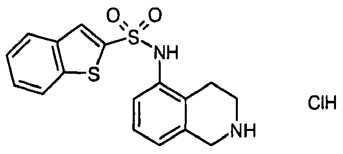 | Benzo[b]thiophene-2-sulfonic acid (1,2,3,4-tetrahydro-isoquinolin-5-yl)-amide hydrochloride | | 345 |
| 105 | 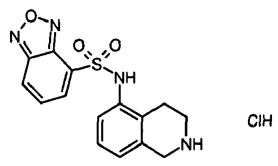 | Benzo[1,2,5]oxadiazol e-4-sulfonic acid (1,2,3,4-tetrahydro-isoquinolin-5-yl)-amide hydrochloride | | 331 |
| 106 | 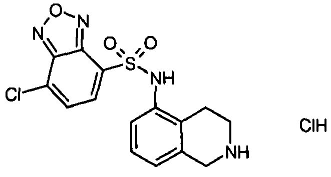 | 7-Chlorobenzo[1,2,5]oxa diazole-4-sulfonic acid (1,2,3,4-tetrahydro-isoquinolin-5-yl)-amide hydrochloride | | 365 |
| 107 | 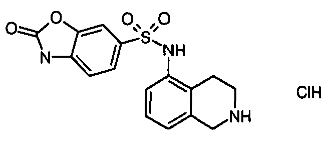 | 2-Oxo-2,3-dihydro-benzooxazole-6-sulfonic acid (1,2,3,4-tetrahydro-isoquinofin-5-yl)-amide hydrochloride | | 346 |
| 108 | 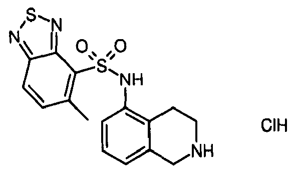 | 5-Methyl-benzo[1,2,5]thiadiazole-4-sulfonic acid (1,2,3,4-tetrahydro-isoquinolin-5-yl)-amide hydrochloride | | 361 |
| 109 | 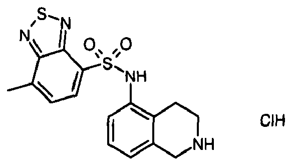 | 7-Methyl-benzo[1,2,5]thiadiazole-4-sulfonic acid (1,2,3,4-tetrahydro-isoquinolin-5-yl)-amide hydrochloride | | 361 |
| 110 | 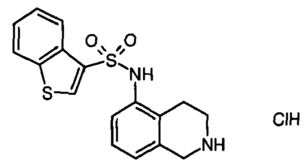 | Benzo[b]thiophene-3-sulfonic acid (1,2,3,4-tetrahydro-isoquinolin-5-yl)-amide hydrochloride | | 345 |
| 111 | 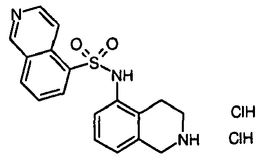 | Isoquinoline-5-sulfonic acid (1,2,3,4-tetrahydro-isoquinolin-5-yl)-amide di hydrochloride | | 340 |
| 112 | 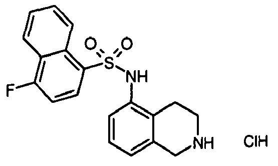 | 4-Fluoro-naphthalene-1-sulfonic acid (1,2,3,4-tetrahydro-isoquinolin-5-yl)-amide hydrochloride | | 357 |
| 113 | 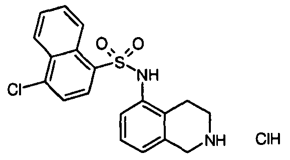 | 4-Chloro-naphthalene-1-sulfonic acid (1,2,3,4-tetrahydro-isoquinolin-5-yl)-amide hydrochloride | | 373 |
| 114 | 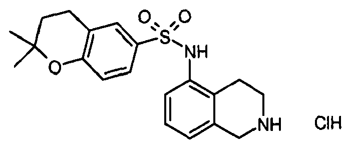 | 2,2-Dimethyl-chroman-6-sulfonic acid (1,2,3,4-tetrahydro-isoquinolin-5-yl)-amide hydrochloride | | 373 |
| 115 | 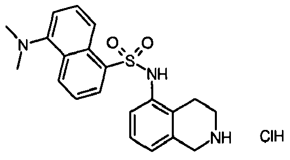 | 5-Dimethylamino-naphthalene-1-sulfonic acid (1,2,3,4-tetrahydro-isoquinolin-5-yl)-amide hydrochloride | | 382 |
| 116 | 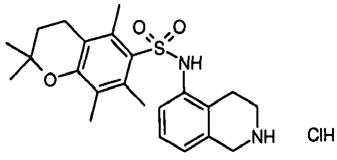 | 2,2,5,7,8-Pentamethyl-chroman-6-sulfonic acid (1,2,3,4-tetrahydro-isoquinolin-5-yl)-amide hydrochloride | | 415 |
| 117 | 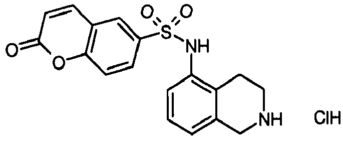 | 2-Oxo-2H-chromene-6-sulfonic acid (1,2,3,4-tetrahydro-isoquinolin-5-yl)-amide hydrochloride | | 357 |
| 118 | 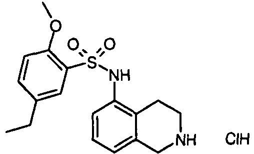 | 5-Ethyl-2-methoxy-N(1,2,3,4-tetrahydro-isoquinolin-5-yl)-benzenesulfonamide hydrochloride | | 347 |
| 119 | 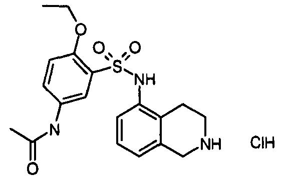 | N-[4-Ethoxy-3-(1,2,3,4-tetrahydro-isoquinolin-5-yl sulfamoyl)-phenyl]-aceta mide hydrochloride | | 390 |
| 120 | 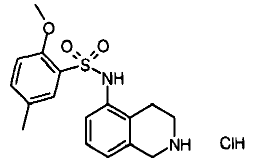 | 2-Methoxy-5-methyl-N-(1,2,3,4-tetrahydro-isoquinolin-5-yl)-benze nesulfonamide hydrocloride | | 333 |
| 121 | 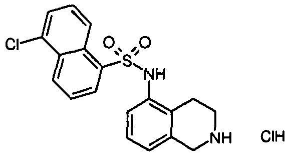 | 5-Chloro-naphthalene-1-sulfonic acid (1,2,3,4-tetrahydro-isoquinolin-5-yl)-amide hydrochloride | | 373 |
| 122 | 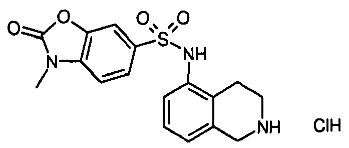 | 3-Methyl-2-oxo-2,3-dihydro-benzooxazole-6-sulfonic acid (1,2,3,4-tetrahydro-isoquinolin-5-yl)-amide hydrochloride | | 360 |
| 123 | 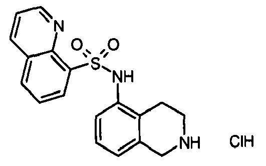 | Quinoline-8-sulfonic acid (1,2,3,4-tetrahydro-isoquinolin-5-yl)-amide hydrochloride | | 340 |
| 124 | 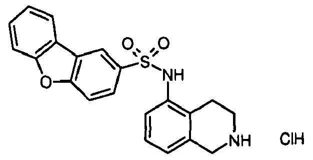 | Dibenzofuran-2-sulfonic acid (1,2,3,4-tetrahydro-isoquinolin-5-yl)-amide hydrochloride | | 379 |
| 125 | 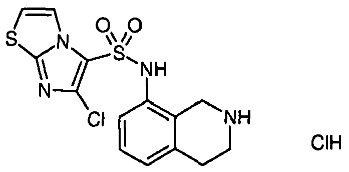 | 6-Chloro-imidazo[2,1-b]thiazole-5-sulfonic acid (1,2,3,4-tetrahydro-is oquinolin-8-yl)-amide hydrochloride | | 369 |
| 126 | 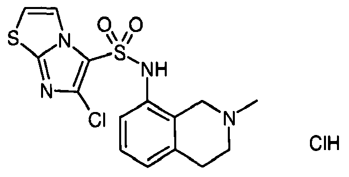 | 6-chloro-N-(2-methyl-1,2,3,4-tetrahydroisoquinolin-8-yl)imidazo[2,1-b]thiazole-5-sulfonamide hydrochloride | | 383 |
| 127 | 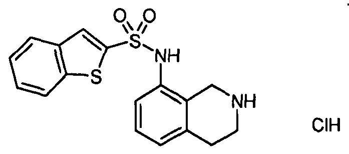 | Benzo[b]thiophene-2-sulfonic acid (1,2,3,4-tetrahydro-isoquinolin-8-yl)-amide hydrochloride | | 345 |
| 128 | 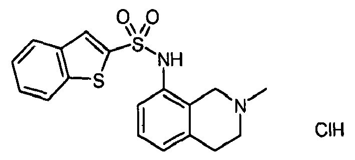 | Benzo[b]thiophene-2-sulfonic acid (2-methyl-1,2,3,4-tetrahydro-isoquinolin-8-yl)-amide hydrochloride | | 359 |
| 129 | 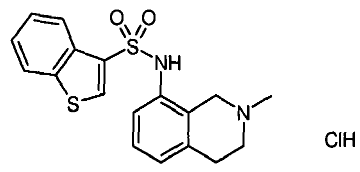 | Benzo[b]thiophene-3-sulfonic acid (2-methyl-1,2,3,4-tetrahydro-isoquinolin-8-yl)-amide hydrochloride | | 359 |
| 130 | 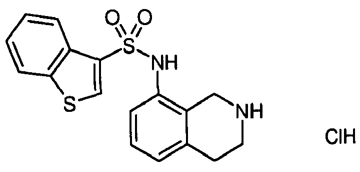 | Benzo[b]thiophene-3-sulfonic acid (1,2,3,4-tetrahydro-isoquinolin-8-yl)-amide hydrochloride | | 345 |
| 131 | 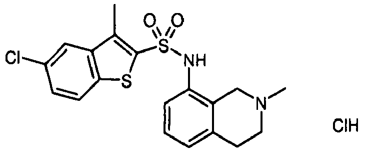 | 5-Chloro-3-methyl-benzo[b]thiophene -2-sulfonic acid (2-methyl-1,2,3,4-tetrahydro-isoquinolin-8-yl)-amide hydrochloride | | 407 |
| 132 | 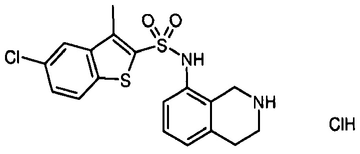 | 5-Chloro-3-methyl-benzo[b]thiophene -2-sulfonic acid (1,2,3,4-tetrahydro-isoquinolin-8-yl)-amide hydrochloride | | 393 |
| 133 | 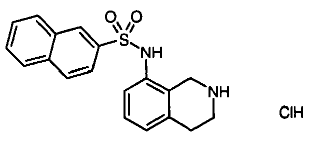 | Naphthalene-2-sulfonic acid (1,2,3,4-tetrahydro-isoquinolin-8-yl)-amide hydrochloride | | 339 |
| 134 | 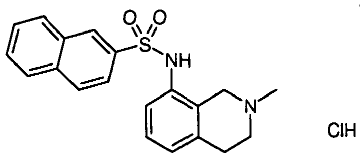 | Naphthalene-2-sulfonic acid (2-methyl-1,2,3,4-tetrahydro-isoquinolin-8-yl)-amide hydrochloride | | 353 |
| 137 | 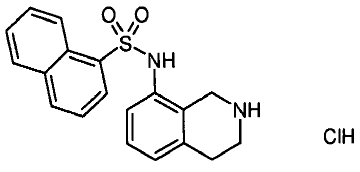 | Naphthalene-1-sulfonic acid (1,2,3,4-tetrahydro-isoquinolin-8-yl)-amide hydrochloride | | 339 |
| 138 | 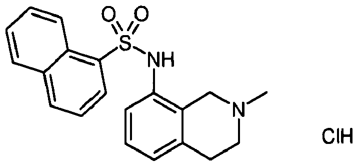 | Naphthalene-1-sulfonic acid (2-methyl-1,2,3,4-tetrahydro-isoquinolin-8-yl)-amide Hydrochloride | | 353 |
| 139 | 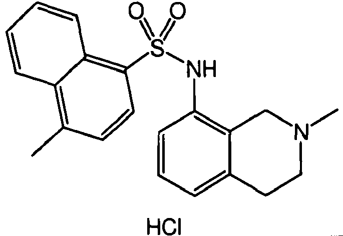 | 4-Methyl-naphthalene-1-sulfonic acid (2-methyl-1,2,3,4-tetrahydro-isoquinolin-8-yl)-amide Hydrochloride | | 367 |
| 140 | 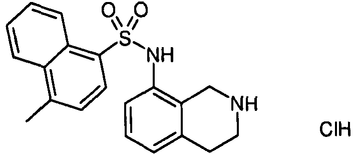 | 4-Methyl-naphthalene-1-sulfonic acid (1,2,3,4-tetrahydro-isoquinolin-8 -yl)-amide Hydrochloride | | 353 |
| 141 | 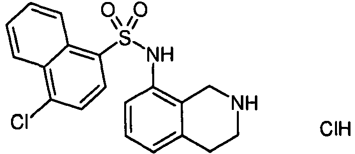 | 4-Chloro-naphthalene-1-sulfonic acid (1,2,3,4-tetra hydro-isoquinolin-8-yl)-amide Hydrochloride | | 373 |
| 142 | 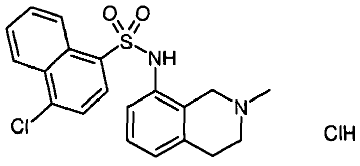 | 4-Chloro-naphthalene-1-sulfonic acid (2-methyl -1,2,3,4-tetrahydro-isoquino lin-8-yl)-amide Hydrochloride | | 387 |
| 143 | 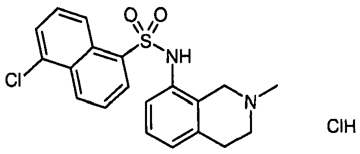 | 5-Chloro-naphthalene-1-sulfonic acid (2-methyl -1,2,3,4-tetrahydro-isoquinolin-8-yl)-amide Hydrochloride | | 387 |
| 144 | 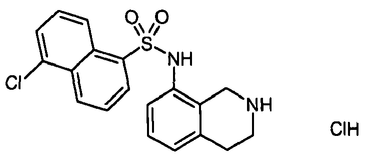 | 5-Chloro-naphthalene-1-sulfonic acid (1,2,3,4-tetrahydro-isoquinolin-8-yl)-amide Hydrochloride | | 373 |
| 145 | 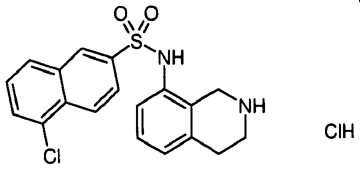 | 5-Chloro-naphthalene-2-sulfonic acid (1,2,3,4-tetrahydro-isoquinolin-8-yl)-amide Hydrochloride | | 373 |
| 146 | 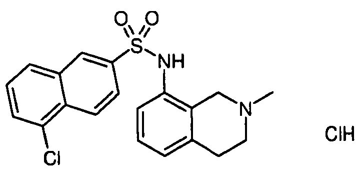 | 5-Chloro-naphthalene-2-sulfonic acid (2-methyl-1,2,3,4-tetrahydro-isoquinolin-8-yl)-amide Hydrochloride | | 387 |
| 147 | 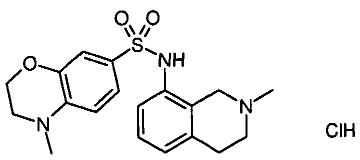 | 4-Methyl-3,4-dihydro-2H-benzo[1,4]oxazine-7-sulfonic acid (2-methyl-1,2,3,4-tetrahydro-isoquinolin-8-yl)-amide Hydrochloride | | 374 |
| 148 | 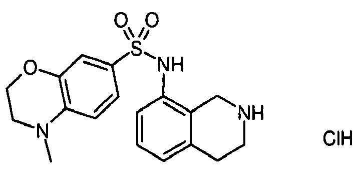 | 4-Methyl-3,4-dihydro-2H-benzo[1,4]oxazine-7-sulfonic acid (1,2,3,4-tetrahydro-isoquinolin-8-yl)-amide Hydrochloride | | 360 |
| 149 | 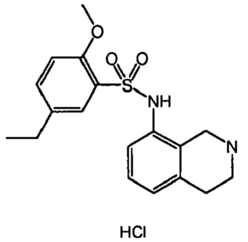 | 5-Ethyl-2-methoxy-N-(1,2,3,4-tetrahydro-isoquinolin-8-yl)-benzenesulfonamide Hydrochloride | | 347 |
| 150 | 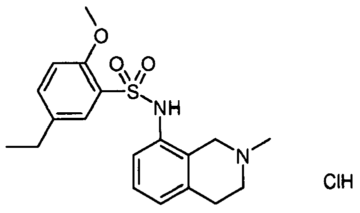 | 5-Ethyl-2-methoxy-N-(2-methyl-1,2,3,4-tetrahydro-isoquinolin-8-yl)-benzene sulfonamide Hydrochloride | | 361 |
| 151 | 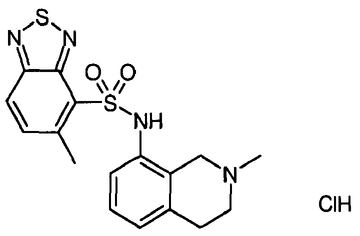 | 5-Methyl-benzo[1,2,5]thiadiazole-4-sulfonic acid (2-methyl-1,2,3,4-tetrahydro-isoquinolin-8-yl)-amide Hydrochloride | | 375 |
| 152 | 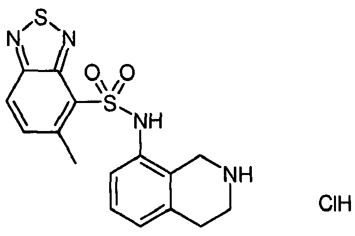 | 5-Methyl-benzo[1,2,5]thiadiazole-4-sulfonic acid (1,2,3,4-tetrahydro-isoquinolin-8-yl)-amide Hydrochloride | | 361 |
| 153 | 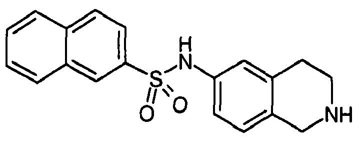 | Naphthalene-2-sulfonic acid (1,2,3,4-tetrahydro-isoquinolin-6-yl)-amide | | 339 |
| 154 | 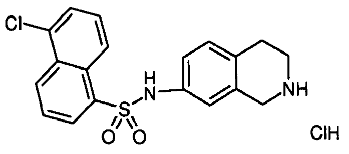 | 5-Chloro-naphthalene-1-sulfonic acid (1,2,3,4-tetrahydro-isoquinolin-7-yl) -amide Hydrochloride | | 373 |
| 155 | 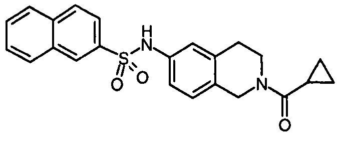 | Naphthalene-2-sulfonic acid (2-cyclopropanecarbonyl-1,2,3,4-tetrahydro-isoquinolin-6-yl)-amide | ¹H NMR (300 MHz, DMSO-*d₆*) δ ppm 0.68 (m, 4 H) 1.95 (br. s., 1 H) 2.60 (br. s., 1 H) 2.72 (br.s., 1 H) 3.54 (br. s., 1 H) 3.75 (br. s., 1 H) 4.41 (br. s., 1 H) 4.69 (br. s., 1 H) 6.86 - 7.02 (m, 3 H) 7.65 (td, *J*=7.76,1.32 Hz, 2 H) 7.76 (dd, *J*=8.72, 1.39 Hz, 1 H) 7.98 (d, *J*=7.76 Hz, 1 H) 8.01 - 8.15 (m, 2 H) 8.43 (s, 1 H) 10.25 (br. s., 1 H) | 407 |
| 156 | 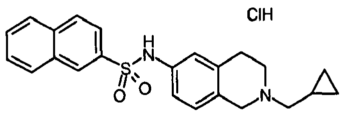 | Naphthalene-2-sulfonic acid (2-cyclopropylmethyl-1,2,3,4-tetrahydro-isoquinolin-6-yl)-amide Hydrochloride | ¹H NMR (300 MHz, DMSO-d₆) δ ppm 0.38 (m, 2 H) 0.62 (m, 2 H) 1.06 - 1.19 (m, 1 H) 2.86 - 3.12 (m, 4 H) 3.19 (m, 1 H) 3.61 (m, 1 H) 4.15 (dd, *J*=15.09, 7.47 Hz, 1 H) 4.34 - 4.45 (m, 1 H) 6.99 - 7.08 (m, 3 H) 7.68 (td, *J*=7.47, 1.39 Hz, 2 H) 7.81 (dd, *J*=8.72, 1.83 Hz, 1 H) 8.01 (d, *J*=7.76 Hz, 1 H) 8.15 (d, *J*=7.47 Hz, 1 H) 8.10 (d, *J*=8.72 Hz, 1 H) 8.49 (d, *J*=1.32 Hz, 1 H) 10.49 (br.s., 1 H) 10.58 (s, 1 H) | 393 |
| 157 | 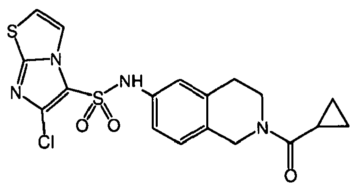 | 6-Chloro-imidazo[2,1-b]thiazole-5-sulfonic acid (2-cyclopropanecarbonyl-1,2,3,4-tetrahydro-isoquinolin-6-yl)-amide | | 437 |
| 158 | 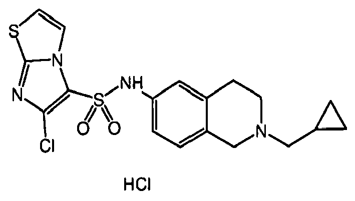 | 6-Chloro-imidazo[2,1-b]thiazole-5-sulfonic acid (2-cyclopropylmethyl-1,2,3,4-tetrahydro-isoquinolin-6-yl)-amide hydrochloride | | 423 |

### Pharmacological data:

The binding of the substituted tetrahydroisoquinoline compounds to the 5-HT₆ receptor was determined as described above.

The binding results for some of these compounds are given in the following table :

| **Compound according to example:** | **Kᵢ (nM)** | **Binding % [100 nM]** | **Binding % [10 nM]** |
|---|---|---|---|
| 1 | 13.8 | 74.8 | 53.3 |
| 2 | | 19.6 | 17.0 |
| 3 | | 18.2 | 3.9 |
| 4 | | 71.1 | 27.3 |
| 5 | 79.8 | 76.3 | 25.9 |
| 6 | 14.5±3.8 | 88.8 | 60.1 |
| 7 | 13.3 | 92.2 | 80.3 |
| 8 | | 74.6 | 37.4 |
| 9 | 9.2±1.2 | 86.5 | 60.1 |
| 10 | 1.3±0.12 | 94.6 | 85.8 |
| 13 | 1.3 | 28.8 | 30.7 |
| 14 | | -3,0 | -7,3 |
| 15 | | 8,2 | -5,0 |
| 16 | | 10,6 | 5,6 |
| 17 | | -2,0 | -1,3 |
| 18 | | 6,8 | -0,8 |
| 19 | 2.3±0.2 | 90.0 | 74.2 |
| 24 | 12.6±1.1 | 86.8 | 53.6 |
| 29 | 5.3±0.4 | 89.6 | 88.0 |
| 30 | 7.8±0.1 | 85.2 | 63.2 |
| 31 | 5.7 | 85.5 | 72.3 |
| 35 | 14.2±1.5 | 81.6 | 48.8 |
| 42 | 9.3±2.2 | 88.9 | 78.2 |
| 53 | 3.8±1.0 | 82.8 | 55.4 |
| 54 | 10.7±4.8 | 91.9 | 81.0 |
| 55 | 3.8±1.2 | 84.9 | 82.3 |
| 56 | 10.3±0.0 | 92.8 | 86.8 |
| 60 | 49.4±8.2 | 65.1 | 44.8 |
| 61 | 3.2±0.4 | 85.5 | 78.6 |
| 64 | 55.8±24.1 | 66.9 | 41.9 |
| 72 | 54,6 | 64.7 | 45.5 |
| 76 | 19.2±2.4 | 83.2 | 73.1 |
| 78 | 2.1±0.0 | 85.8 | 77.5 |
| 80 | 6.5±0.5 | 83.6 | 70.0 |
| 81 | 9.4±3.7 | 84.1 | 70.3 |
| 86 | 6.1±0.7 | 88.4 | 78.9 |
| 153 | 8.7±0.4 | 88.2 | 52.9 |
| 154 | 39.7±7.4 | 85.1 | 65.7 |

## Claims

1. A substituted tetrahydroisoquinoline compound of general formula Ih, 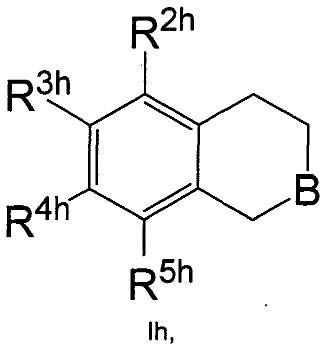 wherein
B represents a radical selected from the group consisting of 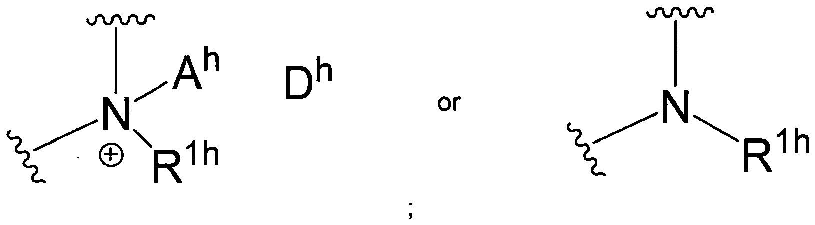
A^{h} represents a hydrogen atom or a radical selected from the group consisting of methyl, ethyl, n-propyl, isopropyl, n-butyl, isobutyl, 2-butyl and tert-butyl;
D^{h} represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate and benzenesulfonate;
R^{1h} represents a radical selected from the group consisting of H, methyl, ethyl, -C(=O)-cyclopropyl, -C(=O)-O-tert-butyl and -CH₂-cyclopropyl;
R^{2h}, R^{3h}, R^{4h} and R^{5h}, independently of one another, each represents a hydrogen atom or a -N(R^{11h})-S(=O)2-R^{12h} radical; with the proviso that at least one of the substituents R^{2h}, R^{3h}, R^{4h} and R^{5h} represents a -N(R^{11h})-S(=O)₂-R^{12h} moiety;
R^{11h} represents a radical selected from the group consisting of H, methyl and ethyl;
R^{12h} represents a radical selected from the group consisting of 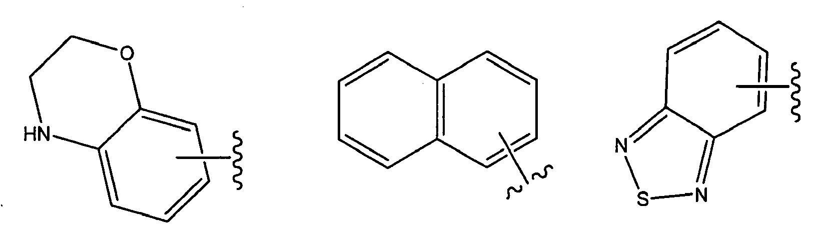 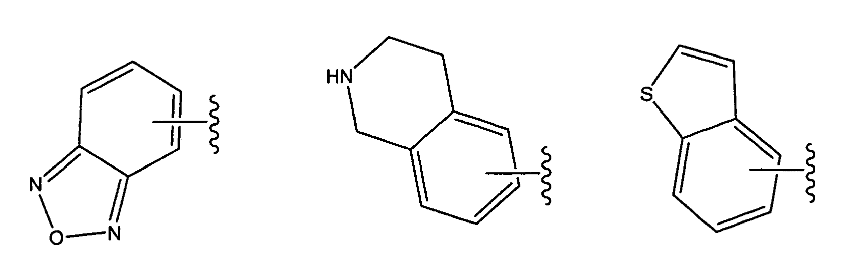 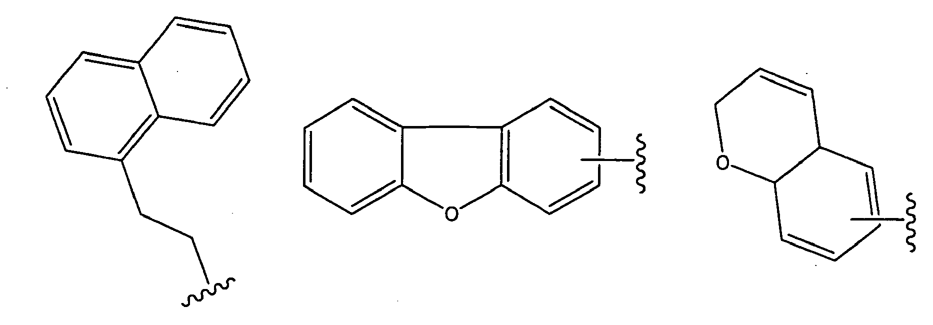 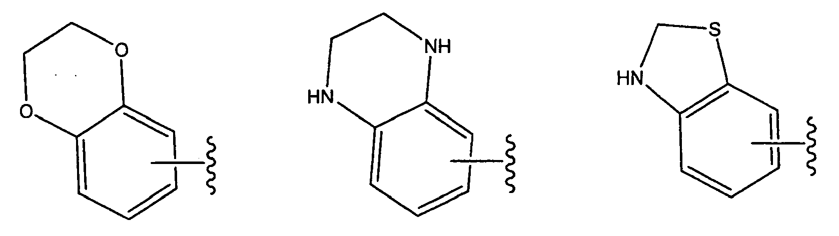 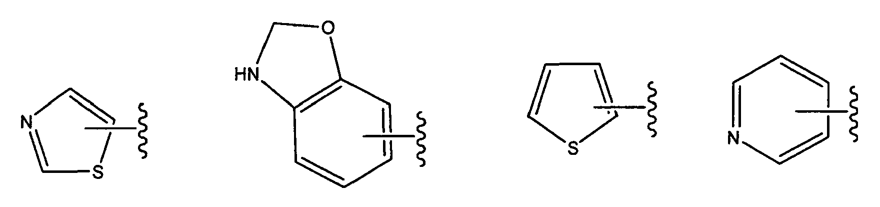 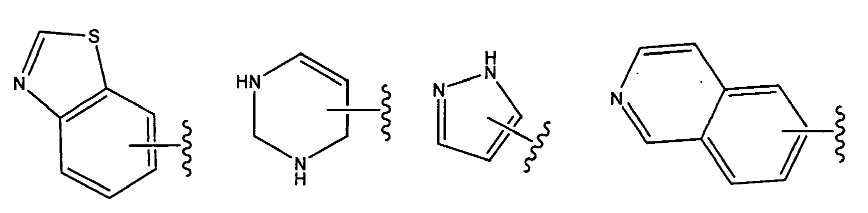 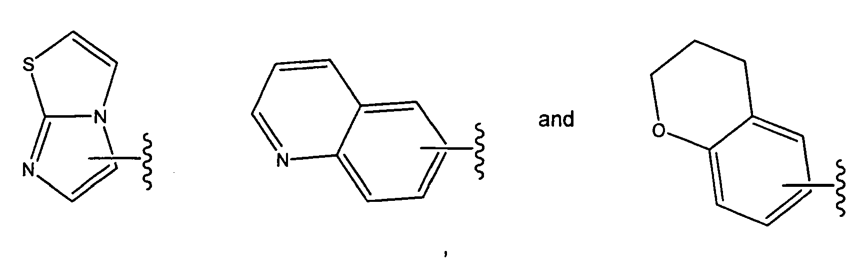
which is unsubstituted or substituted with 1, 2, 3, 4 or 5 substituents independently selected from the group consisting of H, F, Cl, methyl, ethyl, -NH₂, -N(CH₃)₂, oxo (=O), -O-CH₃, -O-CH₂-CH₃, -NH-(C=O)-CH₃, - C(=O)-O-CH₃, -C(=O)-CF₃; whereby the substitution can take place on any suitable position in the aforementioned radicals, including the heteroatom(s);
or a substituted phenyl radical selected from the group consisting of: 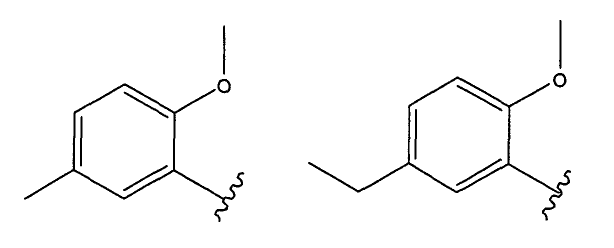 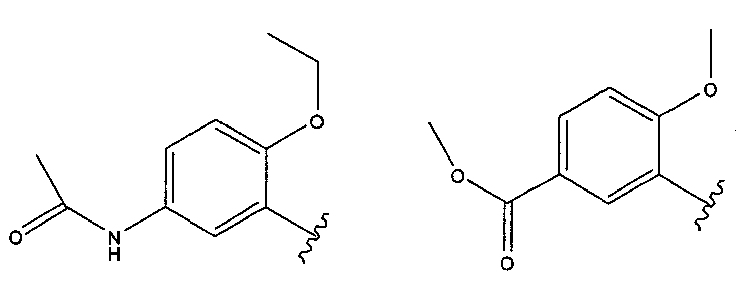 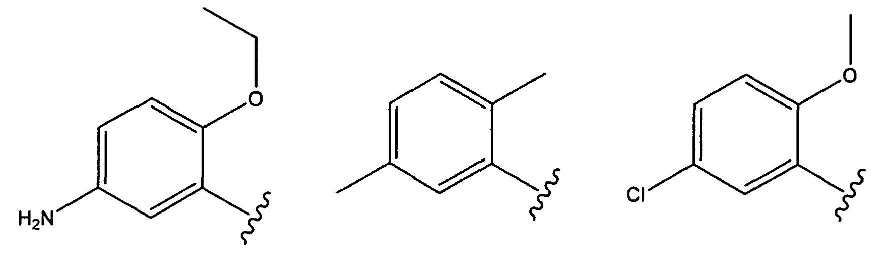 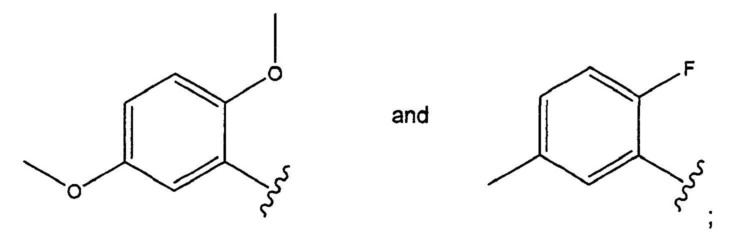 optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

2. A compound according to claim 1, **characterized in that**
B, A^{h}, D^{h}, R^{1h}, R^{2h}, R^{3h}, R^{4h}, R^{5h} and R^{11h} have the meaning as defined in claim 1;
with the proviso that at least one of the substituents R^{2h}, R^{3h}, R^{4h} and R^{5h} represents a -N(R^{11h})-S(=O)₂-R^{12h} moiety;
and
R^{12h} represents a radical selected from the group consisting of 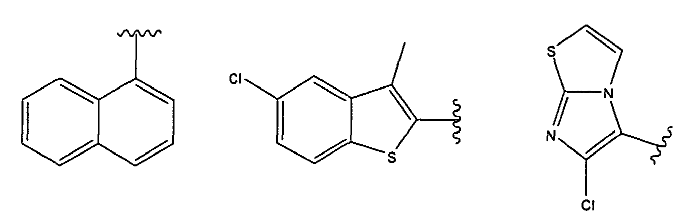 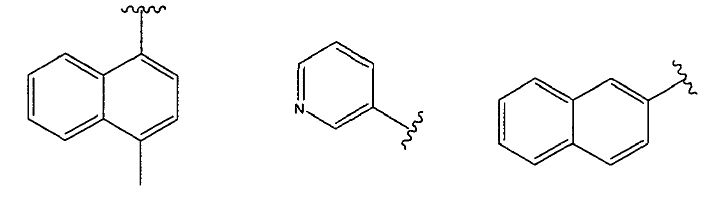 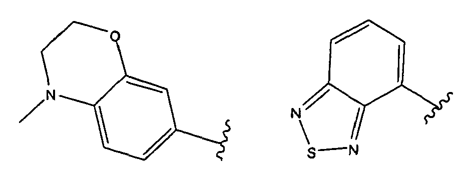 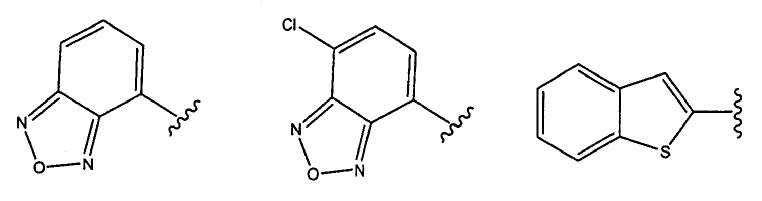 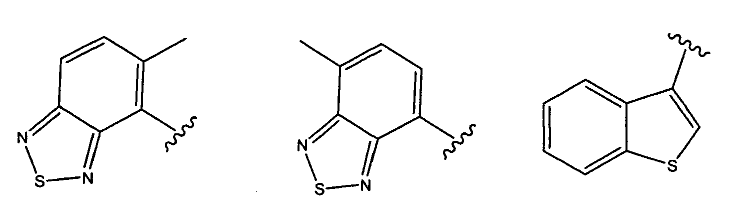 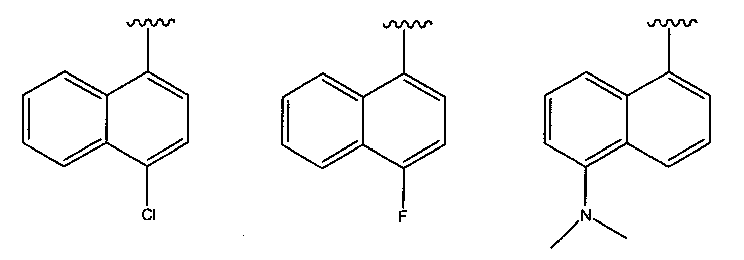 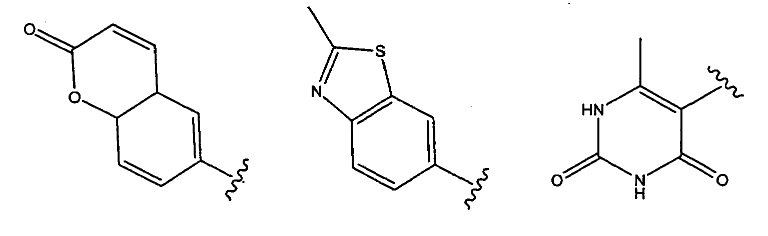 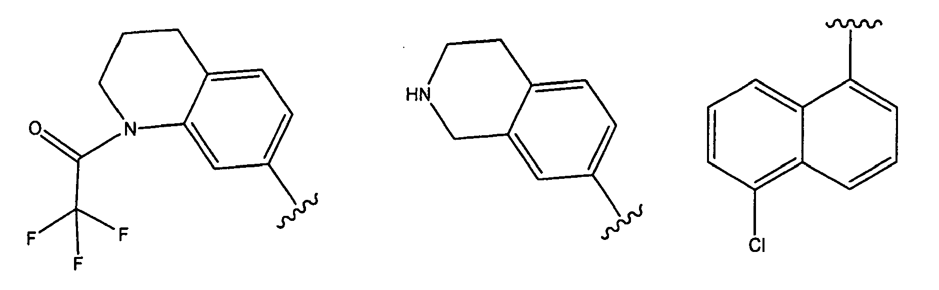 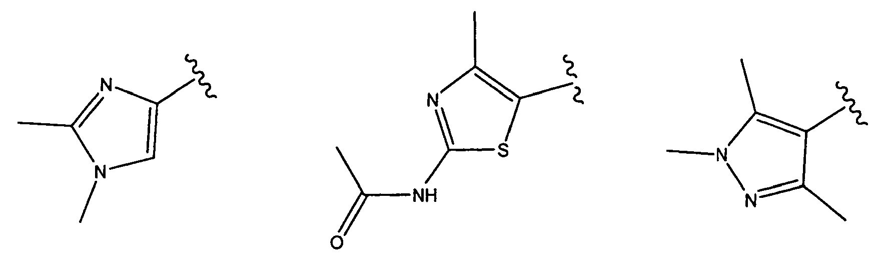 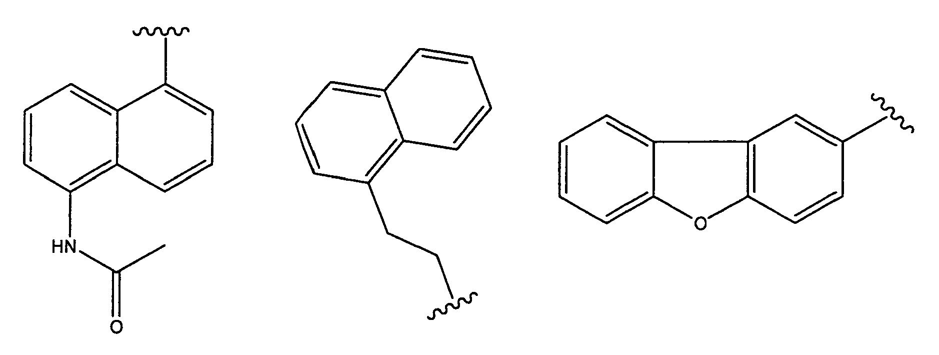 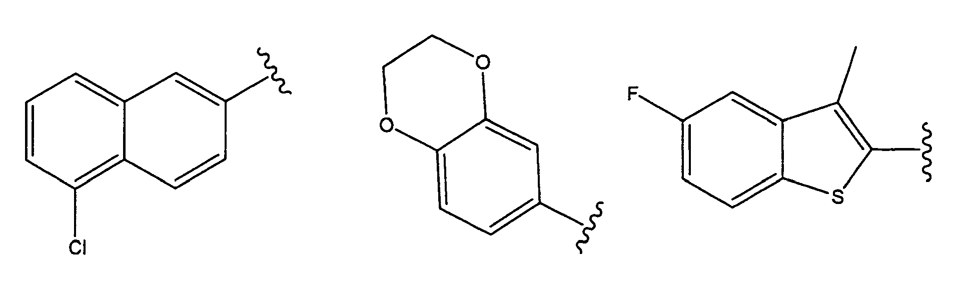 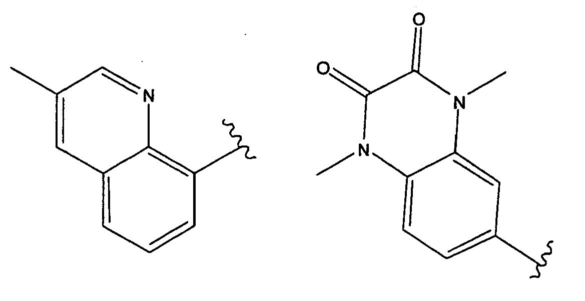 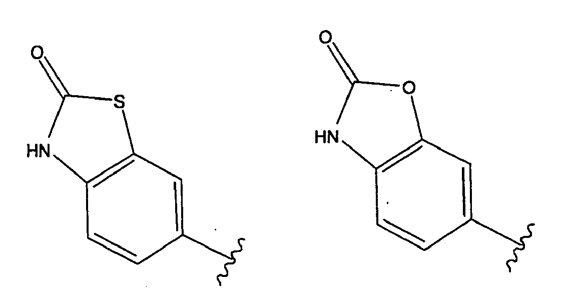 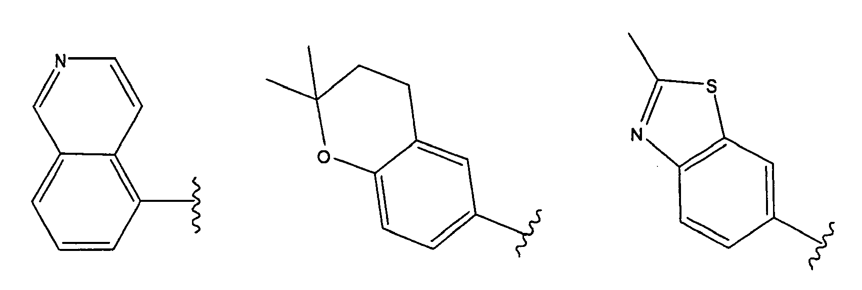 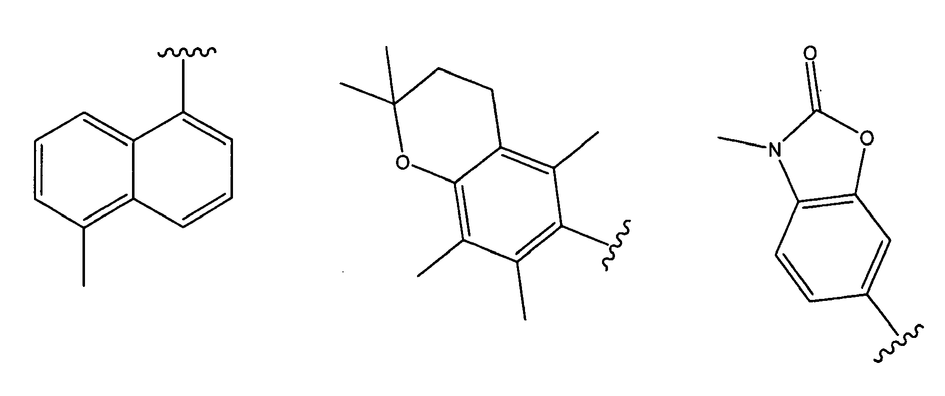 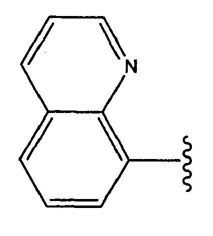 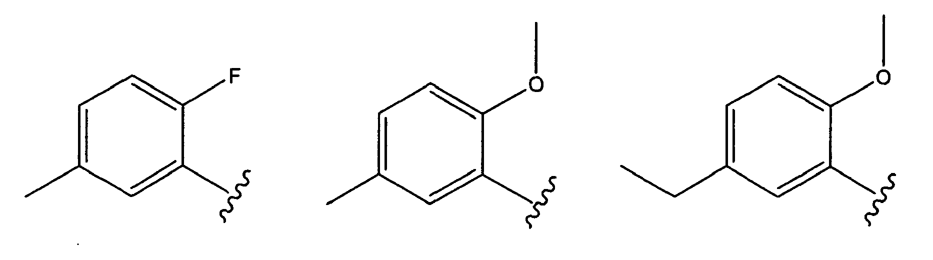 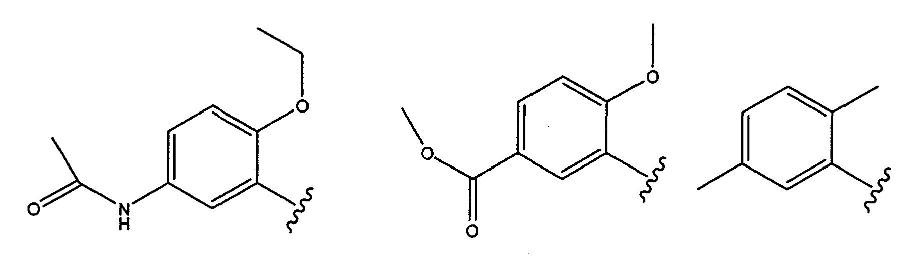 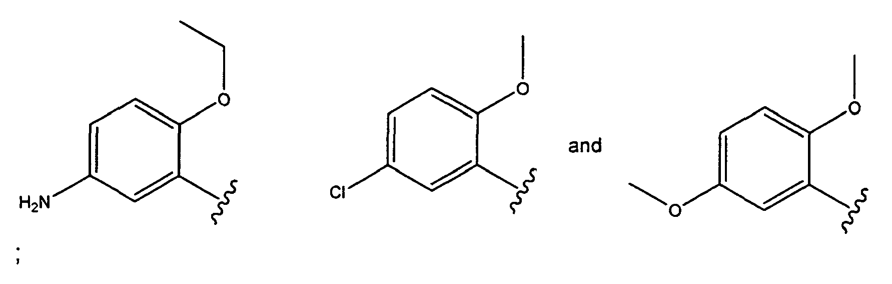 optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

3. A compound according to claim 1 or 2, **characterized in that**
R^{2h} represents a -N(R^{11h})-S(=O)₂-R^{12h} moiety;
and
B, A^{h}, D^{h}, R^{1h}, R^{3h}, R^{4h}, R^{5h}, R^{11h} and R^{12h} have the meaning as defined in claim 1;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

4. A compound according to claim 1 or 2, **characterized in that**
R^{3h} represents a -N(R^{11h})-S(=O)₂-R^{12h} moiety;
and
B, A^{h}, D^{h}, R^{1h}, R^{2h}, R^{4h}, R^{5h}, R^{11h} and R^{12h} have the meaning as defined in claim 1;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

5. A compound according to claim 1 or 2, **characterized in that**
R^{4h} represents a -N(R^{11h})-S(=O)₂-R^{12h} moiety;
and
B, A^{h}, D^{h}, R^{1h}, R^{2h}, R^{3h}, R^{5h}, R^{11h} and R^{12h} have the meaning as defined in claim 1;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

6. A compound according to claim 1 or 2, **characterized in that**
R^{5h} represents a -N(R^{11h})-S(=O)₂-R¹²ⁿ moiety;
and
B, A^{h}, D^{h}, R^{1h}, R^{2h}, R^{3h}, R^{4h}, R^{11h} and R^{12h} have the meaning as defined in claim 1;
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

7. A compound according to one or more of claims 1 to 6 selected from the group consisting of
[1] N-(1,2,3,4-tetrahydroisoquinolin-6-yl)naphthalene-1-sulfonamide hydrochloride,
[2] 2,2-dimethyl-6-(N-methylnaphthalene-1-sulfonamido)-1,2,3,4-tetrahydroisoquinolinium iodide,
[3] N-(2-methyl-1,2,3,4-tetrahydroisoquinolin-6-yl)naphthalene-1-sulfonamide hydrochloride,
[4] 5-chloro-3-methyl-N-(1,2,3,4-tetrahydroisoquinolin-6-yl)benzo[b]thiophene-2-sulfonamide hydrochloride,
[5] 5-chloro-3-methyl-N-(2-methyl-1,2,3,4-tetrahydroisoquinolin-6-yl)benzo[b]thiophene-2-sulfonamide hydrochloride,
[6] 4-methyl-N-(1,2,3,4-tetrahydroisoquinolin-6-yl)naphthalene-1-sulfonamide hydrochloride,
[7] 4-methyl-N-(2-methyl-1,2,3,4-tetrahydroisoquinolin-6-yl)naphthalene-1-sulfonamide hydrochloride,
[8] N-(1,2,3,4-tetrahydroisoquinolin-6-yl)naphthalene-2-sulfonamide hydrochloride,
[9] N-(2-methyl-1,2,3,4-tetrahydroisoquinolin-6-yl)naphthalene-2-sulfonamide hydrochloride,
[10] 6-chloro-N-(1,2,3,4-tetrahydroisoquinolin-6-yl)imidazo[2,1-b]thiazole-5-sulfonamide hydrochloride,
[11] 2-methoxy-5-methyl-N-(1,2,3,4-tetrahydroisoquinolin-6-yl)benzenesulfonamide hydrochloride,
[12] N-(1,2,3,4-tetrahydroisoquinolin-6-yl)pyridine-3-sulfonamide dihydrochloride,
[13] 6-(naphthalene-1-sulfonylamino)-3,4-dihydro-1H-isoquinoline-2-carboxylic acid tert-butyl ester,
[14] 6-(5-chloro-3-methyl-benzo[b]thiophene-2- sulfonylamino)-3,4-dihydro-1H-isoquinoline-2-carboxylic acid tert-butyl ester,
[15] 6-(4-methyl-naphthalene-1-sulfonylamino)-3,4 -dihydro-1H-isoquinoline-2-carboxylic acid tert-butyl ester,
[16] 6-(naphthalene-2-sulfonylamino)-3,4-dihydro-1H-isoquinoline-2-carboxylic acid tert-butyl ester,
[17] 6-(2-methoxy-5-methyl-benzenesulfonylamino) -3,4-dihydro-1H-isoquinoline-2-carboxylic acid tert-butyl ester
[18] 6-(pyridine-3-sulfonylamino)-3,4-dihydro-1H-isoquinoline-2carboxylic acid tert-butyl ester;
[19] 6-Chloro-imidazo[2,1-b]thiazole-5-sulfonic acid (2-methyl-1,2,3,4tetrahydro-isoquinolin-6-yl)-amide hydrochloride
[20] 6-(6-Chloro-imidazo[2,1-b]thiazole-5-sulfonylamino)-3,4-dihydro-1-Hisoquinoline-2-carboxylic acid tert-butyl ester
[21] 4-Methyl-3,4-dihydro-2H-benzo[1,4]oxazine-7-sulfonic acid (1,2,3,4-tetrahydro-isoquinolin-6-yl)-amide hydrochloride
[23] Benzo[1,2,5]thiadiazole-4-sulfonic acid (1,2,3,4-tetrahydro-isoquinolin-6-yl)-amide hydrochloride
[24] Benzo[b]thiophene-2-sulfonic acid (1,2,3,4-tetrahydro-isoquinolin-6-yl)-amide hydrochloride
[25] 7-Chloro-benzo[1,2,5] oxadiazole-4-sulfonic acid (1,2,3,4-tetrahydro-isoquinolin -6-yl)-amide hydrochloride
[26] Benzo[1,2,5]oxadiazole-4-sulfonic acid (1,2,3,4-tetrahydro-isoquinolin-6-yl)-amide hydrochloride
[27] 5-Methyl-benzo[1,2,5] thiadiazole-4-sulfonic acid (1,2,3,4-tetrahydro-isoqui- nolin-6-yl)-amide hydrochloride
[28] 7-Methyl-benzo[1,2,5] thiadiazole-4-sulfonic acid (1,2,3,4-tetrahydro-isoqui- nolin-6-yl)-amide hydrochloride
[29] Benzo[b]thiophene-3-sulfonic acid (1,2,3,4- tetrahydro-isoquinolin-6-yl) -amide hydrochloride
[30] 4-Fluoro-naphthalene-1-sulfonic acid (1,2,3,4-tetrahydro-isoquinolin-6-yl)-amide hydrochloride
[31] 4-Chloro-naphthalene-1-sulfonic acid (1,2,3,4-tetrahydro-isoquinolin-6-yl)-amide hydrochloride
[32] 5-Dimethylaminonaphtha- lene-1-sulfonic acid (1,2,3,4 -tetrahydro-isoquinolin -6-yl)-amide hydrochloride
[33] 2-Oxo-4a,8a-dihydro-2H-chromene-6-sulfonic acid (1,2,3,4-tetrahydro-isoqui- nolin-6-yl)-amide hydrochloride
[34] 2-Methyl-benzothiazole-6-sulfonic acid (1,2,3,4- tetrahydro-isoquinolin-6-yl)-amide hydrochloride
[35] 5-Ethyl-2-methoxy-N-(1,2,3,4-tetrahydro- isoquinolin-6-yl)-benzene sulfonamide hydrochloride
[36] 6-Methyl-2,4-dioxo-1,2,3,4-tetrahydro-pyrimidine-5-sulfonic acid (1,2,3,4-tetrahydro-isoquinolin-6-yl)-amide hydrochloride
[37] N-[4-Ethoxy-3-(1,2,3,4-tetrahydro-isoquinolin-6- ylsulfamoyl)-phenyl]-acetamide hydrochloride
[38] 4-Methoxy-3-(1,2,3,4-tetrahydro-isoquinolin-6-yl sulfamoyl)-benzoic acid methyl ester hydrochloride
[39] 2-(2,2,2-Trifluoro-acetyl)-1,2,3,4-tetrahydro-isoquinoline-7-sulfonic acid (1,2,3,4-tetrahydro-isoquinolin-6-yl)-amide hydrochloride
[40] 1,2,3,4-Tetrahydro-isoquino line-7-sulfonic acid (1,2,3,4-tetrahydro-isoquinolin-6- yl)-amide dihydrochloride
[41] 5-Amino-2-ethoxy-N-(1,2,3,4-tetrahydro-isoquinolin-6-yl)-benzenesulfonamide dihydrochloride
[42] 5-Chloro-naphthalene-1-sulfonic acid (1,2,3,4- tetrahydro-isoquinolin-6-yl)-amide hydrochloride
[43] 1,2-Dimethyl-1H-imidazole-4-sulfonic acid (1,2,3,4-tetrahydro-isoquinolin- 6-yl)-amide hydrochloride
[44] N-[4-Methyl-5-(1,2,3,4-tetrahydro-isoquinolin-6- ylsulfamoyl)-thiazol-2-yl] -acetamide hydrochloride
[45] 1,3,5-Trimethyl-1H-pyrazole -4-sulfonic acid (1,2,3,4-tetrahydro-isoquinolin-6-yl) -amide hydrochloride
[46] N-[5-(1,2,3,4-Tetrahydro-isoquinolin-6-ylsulfamoyl) -naphthalen-1-yl]-acetamide hydrochloride
[47] 2-Naphthalen-1-yl-ethanesulfonic acid (1,2,3,4 -tetrahydro-isoquinolin-6-yl) -amide hydrochloride
[48] Dibenzofuran-2-sulfonic acid (1,2,3,4-tetrahydro- isoquinolin-6-yl)-amide hydrochloride
[49] 2,5-Dimethoxy-N-(1,2,3,4-tetrahydro-isoquinolin -6-yl)-benzenesulfonamide hydrochloride
[50] 5-Chloro-2-methoxy-N-(1,2,3,4-tetrahydro-iso quinolin-6-yl)-benzene sulfonamide hydrochloride
[51] 2,5-Dimethyl-N-(1,2,3,4-tetrahydro-isoquinolin -6-yl)-benzenesulfonamide hydrochloride
[52] 2-Fluoro-5-methyl-N-(1,2,3,4-tetrahydro- isoquinolin-6-yl)-benzene sulfonamide hydrochloride
[53] 6-Chloro-imidazo[2,1-b]thiazole-5-sulfonic acid (2-methyl-1,2,3,4-tetrahydro -isoquinolin-7-yl)-amide hydrochloride
[54] 6-Chloro-imidazo[2,1-b]thiazole-5-sulfonic acid (1,2,3,4-tetrahydro-iso- quinolin-7-yl)-amide hydrochloride
[55] 4-Methyl-naphthalene-1-sulfonic acid (1,2,3,4-tetrahydro-isoquinolin-7-yl)-amide hydrochloride
[56] 4-Methyl-naphthalene-1-sulfonic acid (2-methyl-1,2,3,4-tetrahydro-isoquinolin-7-yl)-amide hydrochloride
[57] 5-Chloro-naphthalene-2-sulfonic acid (2-methyl-1,2,3,4-tetrahydro-isoquinolin-7-yl)-amide hydrochloride
[58] 5-Chloro-naphthalene-2-sulfonic acid (1,2,3,4-tetrahydro-isoquinolin-7-yl) -amide hydrochloride
[59] 4-Methyl-3,4-dihydro-2H-benzo[1,4]oxazine-7-sulfonic acid (1,2,3,4-tetrahydro-isoquinolin-7-yl)-amide hydrochloride
[60] 5-Chloro-3-methyl-benzo[b]thiophene-2-sulfonic acid (1,2,3,4-tetrahydro-isoquinolin -7-yl)-amide hydrochloride
[61] Naphthalene-1-sulfonic acid (1,2,3,4-tetrahydro- isoquinolin-7-yl)-amide hydrochloride
[63] 2,3-Dihydro-benzo[1,4] dioxine-6-sulfonic acid (1,2,3,4-tetrahydro-iso- quinolin-7-yl)-amide hydrochloride
[64] 5-Fluoro-3-methyl-benzo[b] thiophene-2-sulfonic acid (1,2,3,4-tetrahydro-isoquinolin -7-yl)-amide hydrochloride
[65] 3-Methyl-quinoline-8-sulfonic acid (1,2,3,4-tetra hydro-isoquinolin-7-yl)-amide hydrochloride
[66] Naphthalene-2-sulfonic acid (1,2,3,4-tetrahydro -isoquinolin-7-yl)-amide hydrochloride
[67] Benzo[1,2,5]thiadiazole-4-sulfonic acid (1,2,3,4-tetrahydro-isoquinolin-7-yl) -amide hydrochloride
[68] 1,4-Dimethyl-2,3-dioxo-1,2,3,4-tetrahydro-quino xaline-6-sulfonic acid (1,2,3,4-tetrahydro-isoquinolin-7-yl)-amide hydrochloride
[69] Benzo[b]thiophene-2-sulfonic acid (1,2,3,4-tetrahydro -isoquinolin-7-yl)-amide hydrochloride
[71] 7-Chloro-benzo[1,2,5] oxadiazole-4-sulfonic acid (1,2,3,4-tetrahydro-isoquinolin-7-yl)-amide hydrochloride
[72] Benzo[1,2,5]oxadiazole-4-sulfonic acid (1,2,3,4-tetrahydro-isoquinolin-7-yl)-amide hydrochloride
[73] 2-Oxo-2,3-dihydro-benzo thiazole-6-sulfonic acid (1,2,3,4-tetrahydro-isoqui- nolin-7-yl)-amide hydro- chloride
[74] 2-Oxo-2,3-dihydro-benzo oxazole-6-sulfonic acid (1,2,3,4-tetrahydro-isoquinolin-7-yl)-amide hydrochloride
[76] 5-Methylbenzo[1,2,5] thiadiazole -4-sulfonic acid (1,2,3,4-tetrahydro-isoquinolin-7-yl)-amide hydrochloride
[77] 7-Methyl-benzo[1,2,5] thiadiazole-4-sulfonic acid (1,2,3,4-tetrahydro-isoquinolin-7-yl)-amide hydrochloride
[78] Benzo[b]thiophene-3-sulfonic acid (1,2,3,4- tetrahydro-isoquinolin-7-yl) -amide hydrochloride
[79] Isoquinoline-5-sulfonic acid (1,2,3,4-tetrahydro-isoquinolin-7-yl)-amide hydrochloride
[80] 4-Fluoro-naphthalene-1-sulfonic acid (1,2,3,4 -tetrahydro-isoquinolin-7-yl) -amide hydrochloride
[81] 4-Chloro-naphthalene-1-sulfonic acid (1,2,3,4 -tetrahydro-isoquinolin-7-yl) -amide hydrochloride
[82] 2,2-Dimethyl-chroman-6-sulfonic acid (1,2,3,4- tetrahydro-isoquinolin-7-yl) -amide hydrochloride
[83] 5-Dimethylamino-naphtha lene-1-sulfonic acid (1,2,3,4-tetrahydro-isoquinolin-7-yl) -amide hydrochloride
[84] 2-Oxo-2H-chromene-6-sulfonic acid (1,2,3,4-tetrahydro-isoquinolin-7-yl)- amide hydrochloride
[85] 2-Methyl-benzothiazole-6-sulfonic acid (1,2,3,4- tetrahydro-isoquinolin-7-yl) -amide hydrochloride
[86] 5-Ethyl-2-methoxy-N-(1,2,3,4-tetrahydro-isoquinolin-7-yl)-benzenesulfonamide hydrochloride
[87] 6-Methyl-2,4-dioxo-1,2,3,4-tetrahydro-pyrimidine- 5-sulfonic acid (1,2,3,4-tetrahydro-isoquinolin-7-yl)-amide hydrochloride
[88] 6-Chloro-imidazo[2,1-b] thiazole-5-sulfonic acid ethyl-(2-ethyl-1,2,3,4-tetrahydro-isoquinolin-5-yl) -amide hydrochloride
[89] 6-Chloro-imidazo[2,1-b] thiazole-5-sulfonic acid (2-ethyl-1,2,3,4-tetrahydro-isoquinolin-5-yl)-amide hydrochloride
[90] 6-Chloro-imidazo[2,1-b] thiazole-5-sulfonic acid (1,2,3,4-tetrahydro-isoquinolin-5-yl)-amide hydrochloride
[91] 5-Methyl-naphthalene-l-sulfonic acid (1,2,3,4- tetrahydro-isoquinolin-5-yl) -amide hydrochloride
[92] Naphthalene-1-sulfonic acid (1,2,3,4-tetrahydro- isoquinolin-5-yl)-amide hydrochloride
[93] 5-Chloro-naphthalene-2-sulfonic acid (1,2,3,4- tetrahydro-isoquinolin-5-yl) -amide hydrochloride
[94] 4-Methyl-3,4-dihydro-2H-benzo[1,4]oxazine-7- sulfonic acid (1,2,3,4-tetrahydro-isoquinolin-5-yl)-amide hydrochloride
[95] 5-Chloro-3-methyl-benzo[b] thiophene-2-sulfonic acid ethyl-(2-ethyl-1,2,3,4-tetrahydro-isoquinolin-5-yl)-amide hydrochloride
[96] 5-Chloro-3-methyl-benzo[b] thiophene-2-sulfonic acid (1,2,3,4-tetrahydro-isoquinolin-5-yl)-amide hydrochloride
[98] 2,3-Dihydro-benzo[1,4] dioxine-6-sulfonic acid (1,2,3,4-tetrahydro-isoquinolin-5-yl)-amide hydrochloride
[99] 5-Fluoro-3-methyl-benzo[b] thiophene-2-sulfonic acid (1,2,3,4-tetrahydro-isoquinolin-5-yl)-amide hydrochloride
[100] 3-Methyl-quinoline-8-sulfonic acid ethyl-(2-ethyl -1,2,3,4-tetrahydro-isoquinolin-5-yl)-amide hydrochloride
[101] 3-Methyl-quinoline-8-sulfonic acid (1,2,3,4- tetrahydro-isoquinolin-5-yl) -amide hydrochloride
[102] Naphthalene-2-sulfonic acid (1,2,3,4-tetrahydro-isoquino lin-5-yl)-amide hydrochloride
[103] Benzo[1,2,5]thiadiazole-4.-sulfonic acid (1,2,3,4- tetrahydro-isoquinolin-5-yl) -amide hydrochloride
[104] Benzo[b]thiophene-2-sulfonic acid (1,2,3,4- tetrahydro-isoquinolin-5-yl) -amide hydrochloride
[105] Benzo[1,2,5]oxadiazole-4-sulfonic acid (1,2,3,4-tetra hydro-isoquinolin-5-yl)- amide hydrochloride
[106] 7-Chlorobenzo[1,2,5]oxa diazole -4-sulfonic acid (1,2,3,4-tetrahydro-isoquinolin-5-yl)-amide hydrochloride
[107] 2-Oxo-2,3-dihydro-benzooxazole-6-sulfonic acid (1,2,3,4-tetrahydro-isoquinolin-5-yl) -amide hydrochloride
[108] 5-Methyl-benzo[1,2,5] thiadiazole-4-sulfonic acid (1,2,3,4-tetrahydro-isoquinolin-5-yl)-amide hydrochloride
[109] 7-Methyl-benzo[1,2,5] thiadiazole-4-sulfonic acid (1,2,3,4-tetrahydro-isoquinolin-5-yl)-amide hydrochloride
[110] Benzo[b]thiophene-3-sulfonic acid (1,2,3,4- tetrahydro-isoquinolin-5-yl) -amide hydrochloride
[111] Isoquinoline-5-sulfonic acid (1,2,3,4-tetrahydro-isoqui nolin-5-yl)-amide dihydrochloride
[112] 4-Fluoro-naphthalene-1-sulfonic acid (1,2,3,4- tetrahydro-isoquinolin-5-yl) -amide hydrochloride
[113] 4-Chloro-naphthalene-1-sulfonic acid (1,2,3,4- tetrahydro-isoquinolin-5-yl) -amide hydrochloride
[114] 2,2-Dimethyl-chroman-6-sulfonic acid (1,2,3,4- tetrahydro-isoquinolin-5-yl) -amide hydrochloride
[115] 5-Dimethylamino-naphthalene-1-sulfonic acid (1,2,3,4-tetrahydro-isoquinolin-5-yl)-amide hydrochloride
[116] 2,2,5,7,8-Pentamethyl-chroman-6-sulfonic acid (1,2,3,4-tetrahydro-isoquino lin-5-yl)-amide hydrochloride
[117] 2-Oxo-2H-chromene-6-sulfonic acid (1,2,3,4-tetrahy dro-isoquinolin-5-yl) -amide hydrochloride
[118] 5-Ethyl-2-methoxy-N(1,2, 3,4-tetrahydro-isoquinolin-5-yl)-benzenesulfonamide hydrochloride
[119] N-[4-Ethoxy-3-(1,2,3,4-tetrahydro-isoquinolin-5-yl sulfamoyl)-phenyl]-acetamide hydrochloride
[120] 2-Methoxy-5-methyl-N-(1,2,3,4-tetrahydro-isoquinolin-5-yl)-benze nesulfonamide hydrocloride
[121] 5-Chloro-naphthalene-1-sulfonic acid (1,2,3,4-tetrahydro-isoquinolin-5-yl) -amide hydrochloride
[122] 2-Methoxy-5-methyl-N-(1,2,3,4-tetrahydro-isoquinolin-5-yl)-benzenesulfonamide hydrocloride
[123] Quinoline-8-sulfonic acid (1,2,3,4-tetrahydro-isoquinolin -5-yl)-amide hydrochloride
[124] Dibenzofuran-2-sulfonic acid (1,2,3,4-tetrahydro- isoquinolin-5-yl)-amide hydrochloride
[125] 6-Chloro-imidazo[2,1-b] thiazole-5-sulfonic acid (1,2,3,4-tetrahydro-isoquinolin-8-yl)-amide hydrochloride
[126] 6-chloro-N-(2-methyl-1,2,3,4-tetrahydroisoquinolin-8-yl)imidazo[2,1-b]thiazote-5-sulfonamide hydrochloride
[127] Benzo[b]thiophene-2-sulfonic acid (1,2,3,4-tetra hydro-isoquinolin-8-yl) -amide hydrochloride
[128] Benzo[b]thiophene-2-sulfonic acid (2-methyl- 1,2,3,4-tetrahydro-isoquinolin-8-yl)-amide hydrochloride
[129] Benzo[b]thiophene-3-sulfonic acid (2-methyl- 1,2,3,4-tetrahydro-isoquinolin-8-yl)-amide hydrochloride
[130] Benzo[b]thiophene-3-sulfonic acid (1,2,3,4-tetra hydro-isoquinolin-8-yl) -amide hydrochloride
[131] 5-Chloro-3-methyl-benzo[b]thiophene-2-sulfonic acid (2-methyl-1,2,3,4-tetrahydro-isoquinolin-8-yl)-amide hydrochloride
[132] 5-Chloro-3-methyl-benzo[b]thiophene-2-sulfonic acid (1,2,3,4-tetrahydro-isoquinolin -8-yl) -amide hydrochloride
[133] Naphthalene-2-sulfonic acid (1,2,3,4-tetrahydro-isoquinolin-8-yl)-amide hydrochloride
[134] Naphthalene-2-sulfonic acid (2-methyl-1,2,3,4-tetrahydro-isoquinolin-8-yl) -amide hydrochloride
[137] Naphthalene-1-sulfonic acid (1,2,3,4-tetrahydro-isoquinolin-8-yl)-amide hydrochloride
[138] Naphthalene-1-sulfonic acid (2-methyl-1,2,3,4-tetrahydro-isoquinolin-8-yl)-amide Hydrochloride
[139] 4-Methyl-naphthalene-1-sulfonic acid (2-methyl- 1,2,3,4-tetrahydro-isoquinolin-8-yl)-amide Hydrochloride
[140] 4-Methyl-naphthalene-1-sulfonic acid (1,2,3,4-tetrahydro-isoquinolin-8-yl)-amide Hydrochloride
[141] 4-Chloro-naphthalene-1-sulfonic acid (1,2,3,4-tetrahydro-isoquinolin-8-yl)- amide Hydrochloride
[142] 4-Chloro-naphthalene-1-sulfonic acid (2-methyl -1,2,3,4-tetrahydro-isoquinolin-8-yl)-amide Hydrochloride
[143] 5-Chloro-naphthalene-1-sulfonic acid (2-methyl -1,2,3,4-tetrahydro-isoquinolin-8-yl)-amide Hydrochloride
[144] 5-Chloro-naphthalene-1-sulfonic acid (1,2,3,4-tetrahydro-isoquinolin-8-yl)- amide Hydrochloride
[145] 5-Chloro-naphthalene-2-sulfonic acid (1,2,3,4-tetrahydro-isoquinolin-8-yl)- amide Hydrochloride
[146] 5-Chloro-naphthalene-2-sulfonic acid (2-methyl -1,2,3,4-tetrahydro-isoquinolin-8-yl)-amide Hydrochloride
[147] 4-Methyl-3,4-dihydro-2H-benzo[1,4]oxazine-7-sulfonic acid (2-methyl-1,2,3,4-tetrahydro-isoquinolin-8-yl)-amide Hydrochloride
[148] 4-Methyl-3,4-dihydro-2H-benzo[1,4]oxazine-7-sulfonic acid (1,2,3,4-tetrahydro-isoquinolin-8-yl)-amide Hydrochloride
[149] 5-Ethyl-2-methoxy-N-(1,2,3,4-tetrahydro-isoquinolin-8-yl)-benzenesulfonamide Hydrochloride
[150] 5-Ethyl-2-methoxy-N-(2-methyl-1,2,3,4-tetrahydro-isoquinolin-8-yl)-benzene sulfonamide Hydrochloride
[151] 5-Methyl-benzo[1,2,5] thiadiazole-4-sulfonic acid (2-methyl-1,2,3,4-tetrahydro-isoquinolin-8-yl)-amide Hydrochloride
[152] 5-Methyl-benzo[1,2,5] thiadiazole-4-sulfonic acid (1,2,3,4-tetrahydro-isoquinolin-8-yl)-amide Hydrochloride
[153] Naphthalene-2-sulfonic acid (1,2,3,4-tetrahydro-isoquinolin-6-yl)-amide and
[154] 5-Chloro-naphthalene-1-sulfonic acid (1,2,3,4- tetrahydro-isoquinolin-7-yl)-amide Hydrochloride
[155] Naphthalene-2-sulfonic acid (2-cyclopropanecarbonyl-1,2,3,4-tetrahydro-isoquinolin-6-yl)-amide
[156] Naphthalene-2-sulfonic acid (2-cyclopropylmethyl-1,2,3,4-tetrahydro-isoqui nolin-6-yl)-amide Hydrochloride
[157] 6-Chloro-imidazo[2, 1-b] thiazole-5-sulfonic acid (2-cyclopropanecarbonyl- 1,2,3,4-tetrahydro-isoquinolin-6-yl)-amide and
[158] 6-Chloro-imidazo[2,1-b] thiazole-5-sulfonic acid (2-cyclopropylmethyl-1,2,3,4- tetrahydro-isoquinolin-6-yl)-amide hydrochloride
optionally in form of one of its stereoisomers, preferably enantiomers or diasteromers, a racemate, or in form of a mixture of at least two of its stereoisomers, preferably enantiomers and/or diastereomers, in any mixing ratio, or a corresponding salt thereof, or a corresponding solvate thereof, or a corresponding base thereof.

8. Process for the preparation of a compound according to one or more of claims 1 to 7, **characterised in that** at least one compound of general formula IVh, 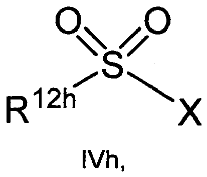 wherein R^{12h} has the meaning according to one or more of claims 1 to 7 and X represents a leaving group, preferably a halogen atom, particularly preferably a chlorine atom, is reacted with at least one compound of general formula VhA, 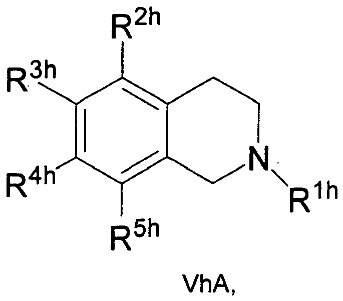 wherein R^{1h} to R^{5h} have the meaning according to one or more of claims 1 to 7, with the proviso that at least one substituent of the group consisting of R^{2h}, R^{3h}, R^{4h} and R^{5h} represents a -N(H)(R^{11h}) moiety, wherein R^{11h} has the meaning according to one or more of claims 1 to 7, or a protected derivative thereof, in a reaction medium, preferably in the presence of at least one base.

9. A medicament comprising at least one substituted tetrahydroisoquinoline compound of general formula Ih according to one or more of claims 1 to 7, optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof and optionally at least one physiologically acceptable auxiliary agent.

10. A medicament according to claim 9 for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

11. Use of at least one substituted tetrahydroisoquinoline compound of general formula Ih according to one or more of claims 1 to 7 for the manufacture of a medicament for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; emesis; vertigo; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; amnesia; autism; sexual dysfunction; gastric motility disorders; circadian rhythm disorders; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

12. A substituted tetrahydroisoquinoline compound of general formula Ig, 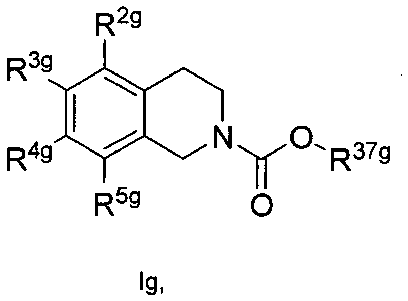 wherein
R^{1g} represents a hydrogen atom; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)₁, _{2 or 3}- group and which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, -C(=O)-CH₃, -C(=O)-C₂H₅, -C(=O)-CH₂-CH₂-CH₃, - C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R^{2g}, R^{3g}, R^{4g} and R^{5g}, independently of one another, each represent a hydrogen atom; F, Cl, Br, I, -NO₂; -NH₂; -SH; -OH; -CN; -C(=O)-OH; - C(=O)-H; -S(=O)₂-OH; -C(=O)-NH₂; -S(=O)₂-NH₂; -OR^{8g}; -SR^{9g}; -N(R^{11g})-S(=O)₂-R^{12g}; a radical selected from the group consisting of methyl, ethyl, n-propyl, isopropyl, n-butyl, sec-butyl, isobutyl, tert-butyl, vinyl, allyl, ethinyl, -CF₃, -CFH₂, -CF₂H, -CH₂-CF₃ and -CF₂-CF₃; or a radical selected from the group consisting of cyclopropyl, cyclobutyl, cyclopentyl and cyclohexyl;
with the proviso that at least one of the substituents R^{2g}, R^{3g}, R^{4g} and R^{5g} represents a -N(R^{11g})-S(=O)₂-R^{12g} moiety;
R^{8g} and R^{9g}, independently of one another, each represent a radical selected from the group consisting of methyl, ethyl, n-propyl, isopropyl, n-butyl, sec-butyl, isobutyl, tert-butyl, -CF₃, -CFH₂, -CF₂H, -CH₂-CF₃ and - CF₂-CF₃; a (hetero)cycloaliphatic radical selected from the group consisting of cyclopropyl, cyclobutyl, cyclopentyl and cyclohexyl; or an aryl or heteroaryl radical selected from the group consisting of phenyl, naphthyl, furyl (furanyl), thienyl (thiophenyl), pyrrolyl, and pyridinyl, which may be bonded via a -(CH₂)₁, _{2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H and - CFH₂;
R^{11g} represents a hydrogen atom, -S(=O)₂-R^{12g} or an alkyl radical selected from the group consisting of methyl, ethyl, n-propyl, isopropyl, n-butyl, sec-butyl, isobutyl and tert-butyl;
R^{12g} represents a phenyl radical of general formula (Ag), 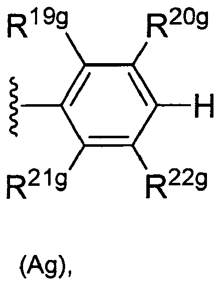 wherein
R^{19g}, R^{20g}, R^{21g} and R^{22g}, independently of one another, each represent a hydrogen atom; F, Cl, Br, I, -NO₂; -NH₂; - SH; -OH; -CN; -C(=O)-OH; -C(=O)-H; -S(=O)₂-OH; -C(=O)-NH₂; -S(=O)₂-NH₂; -C(=O)-R^{23g}; -S(=O)-R^{24g}; -S(=O)₂₋R^{24g}; - OR^{25g}; -SR^{26g}; -C(=O)-OR^{27g}; methyl, ethyl, n-propyl, isopropyl, n-butyl, isobutyl, sec-butyl, tert-butyl, -CF₃, - CF₂H, -CFH₂, -CH₂-CF₃, -CF₂-CF₃, cyclopropyl, cyclobutyl, cyclopentyl and cyclohexyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, - O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, -NO₂, -CHO, -CF₂H and - CFH₂;
R^{23g} and R^{27g}, independently of one another, each represent a radical selected from the group consisting of methyl, ethyl, n-propyl, isopropyl, n-butyl, sec-butyl, isobutyl, tert-butyl, n-pentyl, n-hexyl, -CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be bonded via a -(CH₂)_{1, 2 or 3}-group and which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H and -CFH₂;
R^{24g} and R^{26g}, independently of one another, each represent a radical selected from the group consisting of methyl, ethyl, n-propyl, isopropyl, n-butyl, sec-butyl, isobutyl, tert-butyl, n-pentyl, n-hexyl, vinyl, allyl, ethinyl, - CF₃, -CFH₂, -CF₂H, -CH₂-CF₃ and -CF₂-CF₃; or an aryl or heteroaryl radical selected from the group consisting of phenyl, naphthyl, furyl (furanyl), thienyl (thiophenyl), pyrrolyl, and pyridinyl, which may be unsubstituted or optionally substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, - CF₃, -OCF₃, -SCF₃, -OH, -SH, -NH₂, -NO₂, -CHO, -CF₂H and -CFH₂;
R^{25g} represents a radical selected from the group consisting of methyl, ethyl, n-propyl, isopropyl, n-butyl, sec-butyl, isobutyl, tert-butyl, -CF₃, - CFH₂, -CF₂H, -CH₂-CF₃ and -CF₂-CF₃
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;

13. A compound according to claim 12, **characterized in that**
R¹⁹ represents a hydrogen atom; or a radical selected from the group consisting of methyl, ethyl, n-propyl, n-butyl, n-pentyl and n-hexyl;
R^{2g}, R^{3g}, R^{4g} and R^{5g}, independently of one another, each represent a hydrogen atom or -N(R^{11g})-S(=O)₂-R^{12g}; with the proviso that at least one of the substituents R^{2g}, R^{3g}, R^{4g} and R^{5g} represents a -N(R^{11g})-S(=O)₂-R^{12g} moiety;
R^{11g} represents a hydrogen atom, -S(=O)₂-R^{12g} or an alkyl radical selected from the group consisting of methyl, ethyl and n-propyl;
R^{12g} represents a phenyl radical of general formula (Ag), 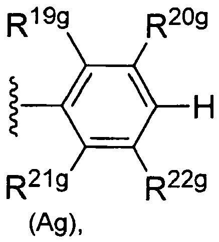 wherein
R^{19g}, R^{20g}, R^{21g} and R^{22g}, independently of one another, each represent a hydrogen atom; F, Cl, Br, I, -O-CH₃; -O-C₂H₅; -O-CF₃; -O-CFH₂ -O=CF₂H; -O-CH₂-CF₃; -O-CF₂-CF₃; methyl, ethyl, n-propyl, isopropyl, n-butyl, isobutyl, sec-butyl and tert-butyl;
with the proviso that at least one of the substituents R^{19g}, R^{20g}, R^{21g} and R^{22g} is unlike hydrogen;
or a naphthyl radical, which may be unsubstituted or optionally substituted with 1, 2, 3, 4 or 5 substituent(s) independently selected from the group consisting of methyl, ethyl, n-propyl, isopropyl, F, Cl and Br;
and R^{37g} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl.

14. A compound according to claim 12 or 13 selected from the group consisting of
[13] 6-(naphthalene-1-sulfonylamino)-3,4-dihydro-1H-isoquinoline-2-carboxylic acid tert-butyl ester,
[15] 6-(4-methyl-naphthalene-1-sulfonylamino)-3,4-dihydro-1H-isoquinoline-2-carboxylic acid tert-butyl ester,
[16] 6-(naphthalene-2-sulfonylamino)-3,4-dihydro-1H-isoquinoline-2-carboxylic acid tert-butyl ester and
[17] 6-(2-methoxy-5-methyl-benzenesulfonylamino)-3,4-dihydro-1H-isoquinoline-2-carboxylic acid tert-butyl ester.

## Patentansprüche

1. Substituierte Tetrahydroisochinolinverbindung der allgemeinen Formel Ih, 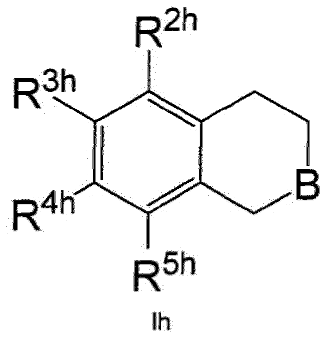 worin
B bedeutet einen Rest, ausgewählt aus der Gruppe bestehend aus 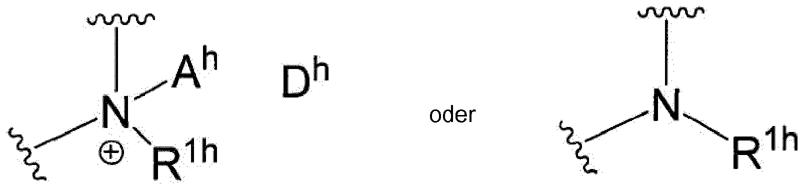 A^{h} bedeutet ein Wasserstoffatom oder einen Rest, ausgewählt aus der Gruppe bestehend aus Methyl, Ethyl, n-Propyl, Isopropyl, n-Butyl, Isobutyl, 2-Butyl und tert-Butyl;
D^{h} bedeutet ein Anion, ausgewählt aus der Gruppe bestehend aus Chlorid, Bromid, Iodid, Fluorid, Hydrogensulfat, Nitrat, Dihydrogenphosphat, Thiocyanat, Cyanat, Acrylat, Methansulfonat, Ethansulfonat, Toluolsulfonat und Benzolsulfonat;
R^{1h} bedeutet einen Rest, ausgewählt aus der Gruppe bestehend aus H, Methyl, Ethyl, -C(=O)-Cyclopropyl, -C(=O)-O-tert-Butyl und -CH₂-Cyclopropyl;
R^{2h}, R^{3h}, R^{4h} und R^{5h} bedeuten jeweils unabhängig voneinander ein Wasserstoffatom oder einen -N(R^{11h})-S(=O)₂-R^{12h}-Rest;
mit der Maßgabe, dass wenigstens einer der Substituenten R^{2h}, R^{3h}, R^{4h} und R^{5h} eine -N(R^{11h})-S(=O)₂-R^{12h} -Einheit bedeutet;
R^{11h} bedeutet einen Rest, ausgewählt aus der Gruppe bestehend aus H, Methyl und Ethyl;
R^{12h} bedeutet einen Rest, ausgewählt aus der Gruppe bestehend aus 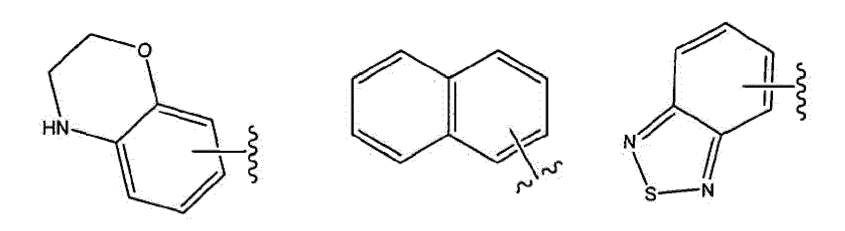 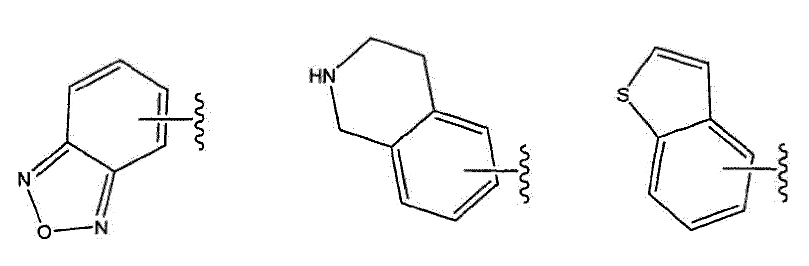 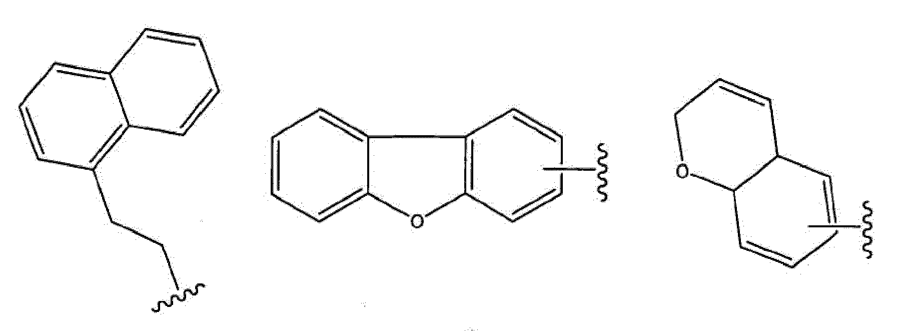 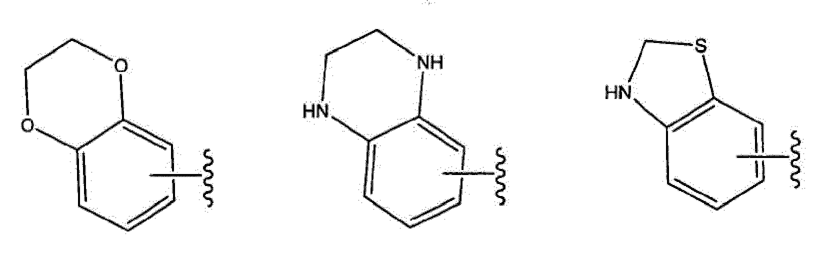 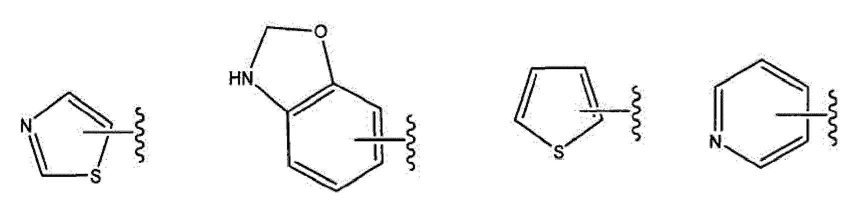 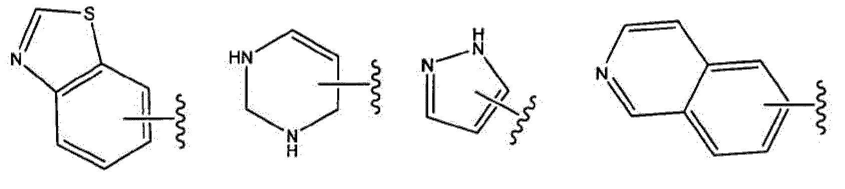 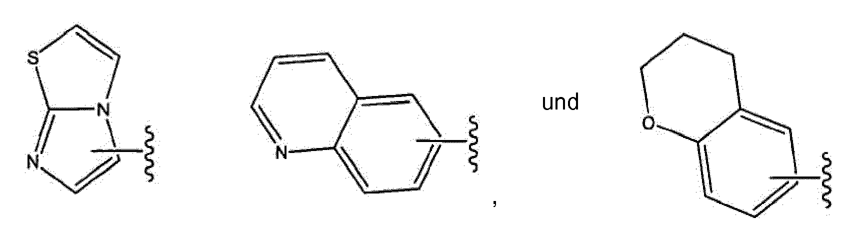 welcher unsubstituiert oder mit 1, 2, 3, 4 oder 5 Substituenten substituiert ist, die unabhängig ausgewählt sind aus der Gruppe bestehend aus H, F, Cl, Methyl, Ethyl, -NH₂, -N(CH₃)₂, Oxo (=O), -O-CH₃, -O-CH₂-CH₃, -NH-(C=O)-CH₃, -C(=O)-O-CH₃, -C(=O)-CF₃; wobei die Substitution in jeder geeigneten Stellung in den vorstehend erwähnten Resten, einschließlich des Heteroatoms bzw. der Heteroatome, stattfinden kann;
oder einen substituierten Phenylrest, ausgewählt aus der Gruppe bestehend aus: 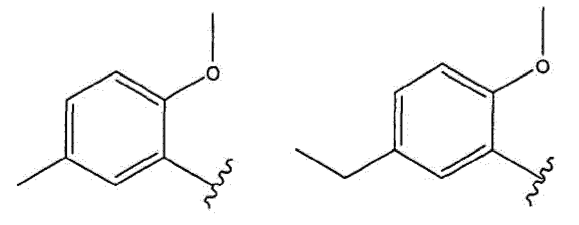 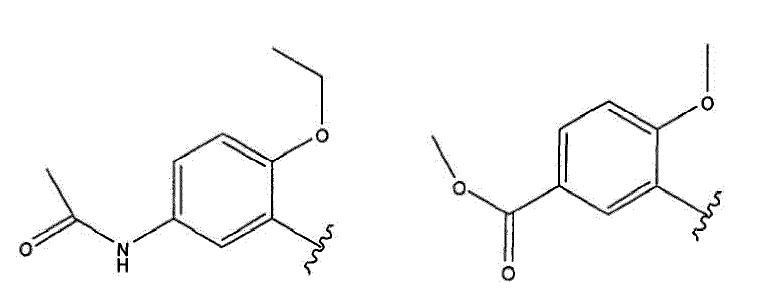 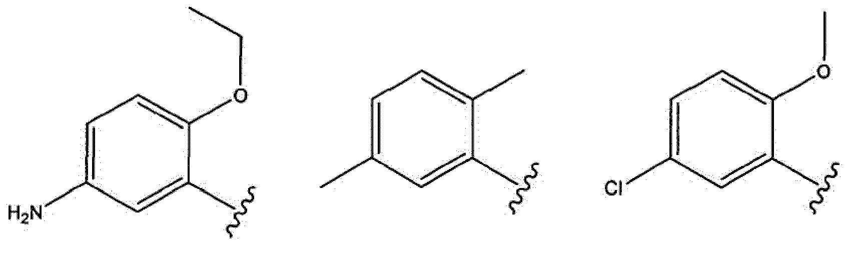 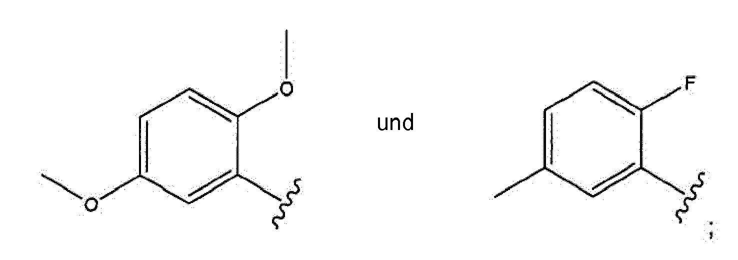 gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

2. Verbindung nach Anspruch 1, **dadurch gekennzeichnet, dass**
B, A^{h}, D^{h}, R^{1h}, R^{2h}, R^{3h}, R^{4h}, R^{5h} und R^{11h} die Bedeutung wie in Anspruch 1 definiert haben;
mit der Maßgabe, dass wenigstens einer von den Substituenten R^{2h}, R^{3h}, R^{4h} und R^{5h} eine -N(R^{11h})-S(=O)₂-R^{12h} -Einheit bedeutet;
und
R¹² einen Rest bedeutet, der ausgewählt ist aus der Gruppe bestehend aus 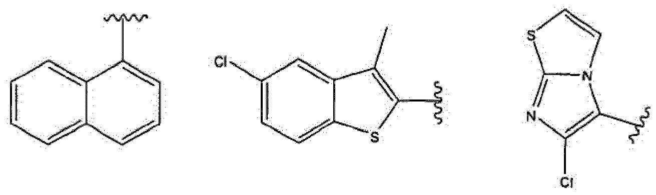 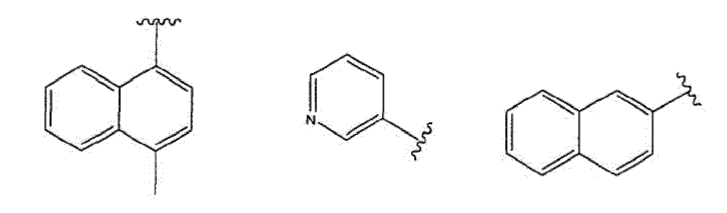 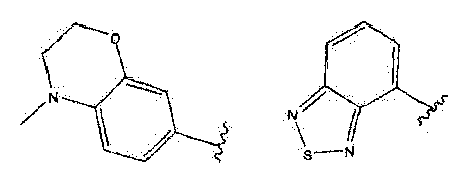 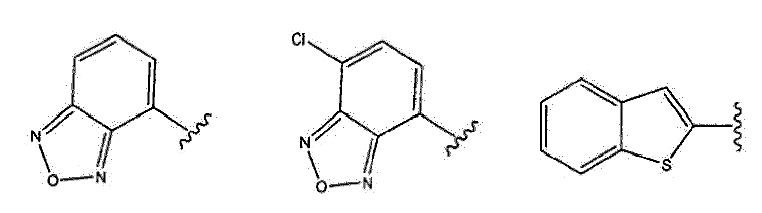 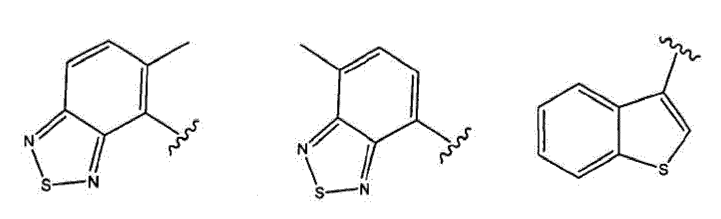 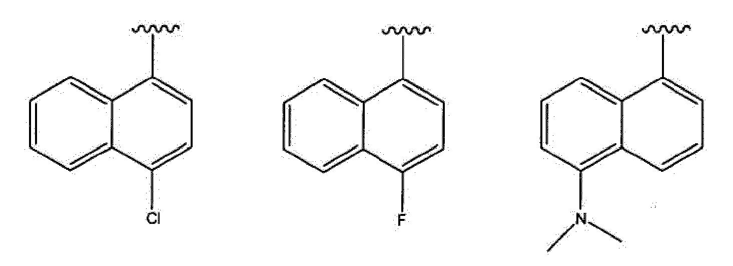 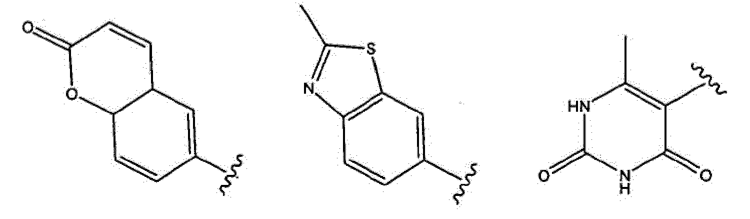 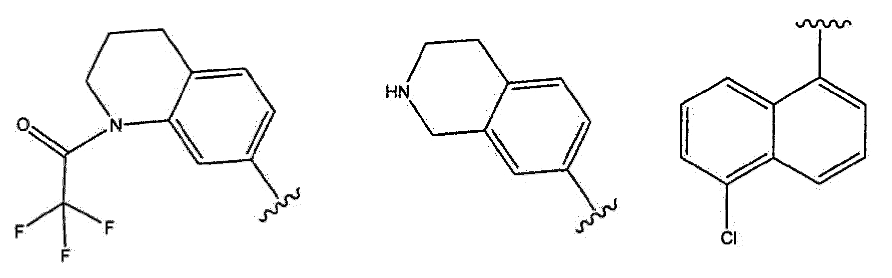 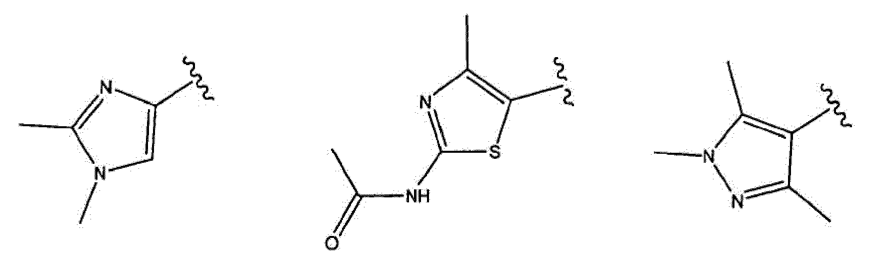 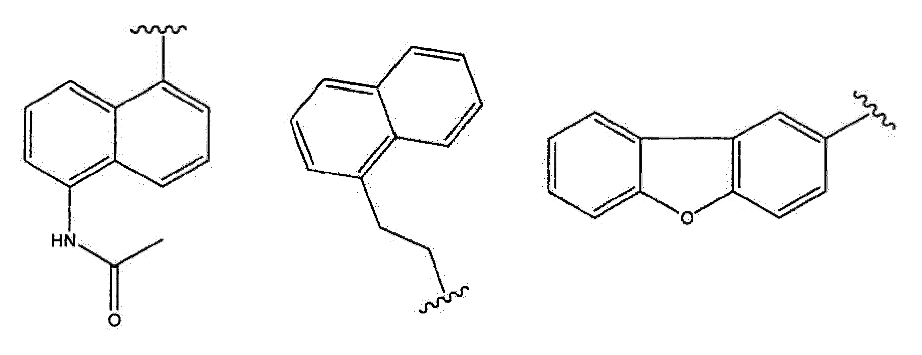 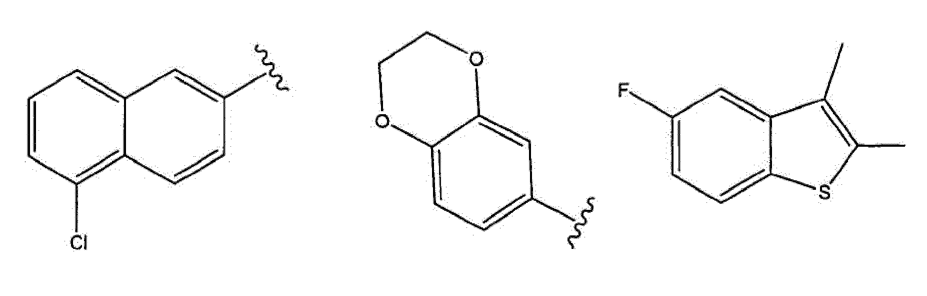 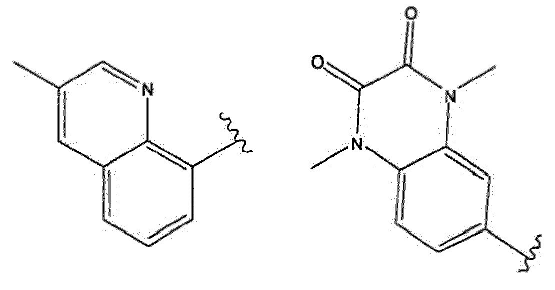 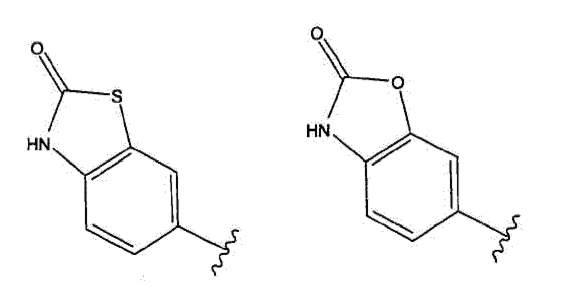 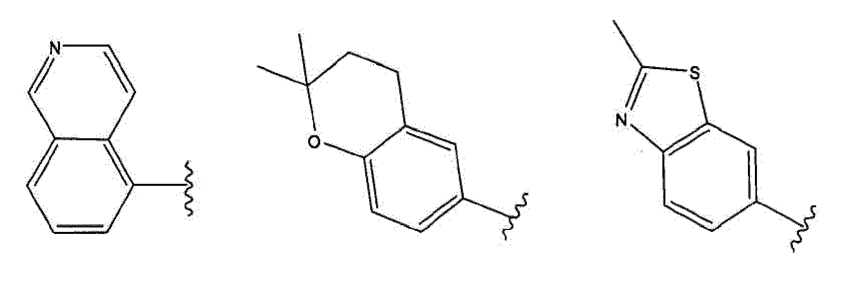 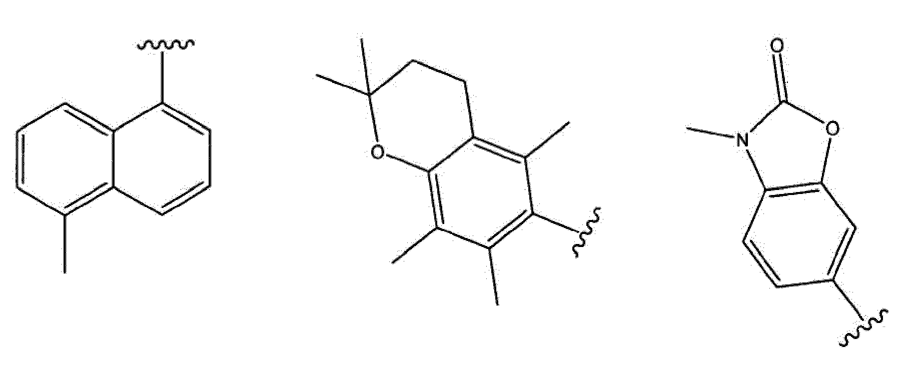 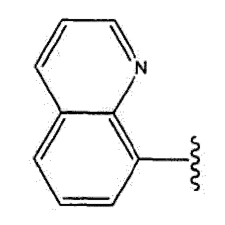 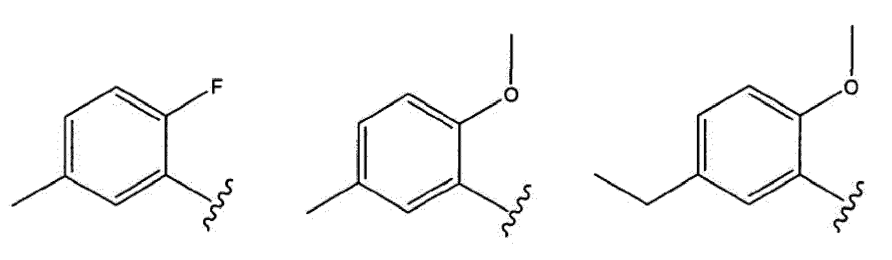 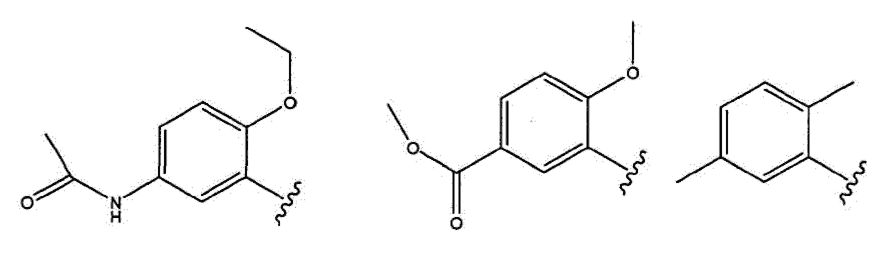 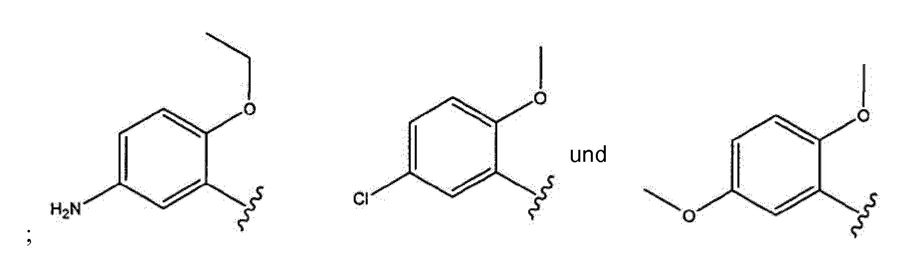 gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

3. Verbindung nach Anspruch 1 oder 2, **dadurch gekennzeichnet, dass**
R^{2h} eine -N(R^{11h})-S(=O)₂-R^{12h} -Einheit bedeutet;
und
B, A^{h}, D^{h}, R^{1h}, R^{3h}, R^{4h}, R^{5h}, R^{11h} und R^{12h} die Bedeutung wie in Anspruch 1 definiert haben; gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

4. Verbindung nach Anspruch 1 oder 2, **dadurch gekennzeichnet, dass**
R^{3h} eine -N(R^{11h})-S(=O)₂₋R¹²h -Einheit bedeutet;
und
B, A^{h}, D^{h}, R^{1h}, R^{2h}, R^{4h}, R^{5h}, R^{11h} und R^{12h} die Bedeutung wie in Anspruch 1 definiert haben; gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

5. Verbindung nach Anspruch 1 oder 2, **dadurch gekennzeichnet, dass**
R^{4h} eine -N(R^{11h})-S(=O)₂R^{12h}-Einheit bedeutet;
und
B, A^{h}, D^{h}, R^{1h}, R^{2h}, R^{3h}, R^{5h}, R^{11h} und R^{12h} die Bedeutung wie in Anspruch 1 definiert haben; gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

6. Verbindung nach Anspruch 1 oder 2, **dadurch gekennzeichnet, dass**
R^{5h} eine -N(R^{11h})-S(=O)₂-R^{12h} -Einheit bedeutet;
und
B, A^{h}, D^{h}, R^{1h}, R^{2h}, R^{3h}, R^{4h}, R^{11h} und R^{12h} die Bedeutung wie in Anspruch 1 definiert haben;
gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

7. Verbindung nach einem oder mehreren der Ansprüche 1 bis 6, ausgewählt aus der Gruppe bestehend aus
[1] N-(1,2,3,4-Tetrahydroisochinolin-6-yl)naphthalin-1-sulfonamid-hydrochlorid,
[2] 2,2-Dimethyl-6-(N-methylnaphthalin-1-sulfonamido)-1,2,3,4-tetrahydroisochinolinium-iodid,
[3] N-(2-Methyl-1,2,3,4-tetrahydroisochinolin-6-yl)naphthalin-1-sulfonamid-hydrochlorid,
[4] 5-Chlor-3-methyl-N-(1,2,3,4-tetrahydroisochinolin-6-yl)benzo[b]thiophen-2-sulfonamid-hydrochlorid,
[5] 5-Chlor-3-methyl-N-(2-methyl-1,2,3,4-tetrahydroisochinolin-6-yl)benzo[b]thiophen-2-sulfonamid-hydrochlorid,
[6] 4-Methyl-N-(1,2,3,4-tetrahydroisochinolin-6-yl)naphthalin-1-sulfonamid-hydrochlorid,
[7] 4-Methyl-N-(2-methyl-1,2,3,4-tetrahydroisochinolin-6-yl)naphthalin-1-sulfonamid-hydrochlorid,
[8] N-(1,2,3,4-Tetrahydroisochinolin-6-yl)naphthalin-2-sulfonamid-hydrochlorid,
[9] N-(2-Methyl-1,2,3,4-tetrahydroisochinolin-6-yl)naphthalin-2-sulfonamid-hydrochlorid,
[10] 6-Chlor-N-(1,2,3,4-tetrahydroisochinolin-6-yl)imidazo[2,1-b]thiazol-5-sulfonamid-hydrochlorid,
[11] 2-Methoxy-5-methyl-N-(1,2,3,4-tetrahydroisochinolin-6-yl)benzolsulfonamid-hydrochlorid,
[12] N-(1,2,3,4-Tetrahydroisochinolin-6-yl)pyridin-3-sulfonamid-dihydrochlorid,
[13] 6-(Naphthalin-1-sulfonylamino)-3,4-dihydro-1H-isochinolin-2-carbonsäure-tert-butylester,
[14] 6-(5-Chlor-3-methyl-benzo[b]thiophen-2-sulfonylamino)-3,4-dihydro-1H-isochinolin-2-carbonsäure-tert-butyl-ester,
[15] 6-(4-Methyl-naphthalin-1-sulfonylamino)-3,4-dihydro-1H-isochinolin-2-carbonsäure-tert-butyl-ester,
[16] 6-(Naphthalin-2-sulfonylamino)-3,4-dihydro-1H-isochinolin-2-carbonsäure-tert-butylester,
[17] 6-(2-Methoxy-5-methyl-benzolsulfonylamino)-3,4-dihydro-1H-isochinolin-2-carbonsäure-tert-butyl-ester
[18] 6-(Pyridin-3-sulfonylamino)-3,4-dihydro-1H-isochinolin-2-carbonsäure-tert-butylester;
[19] 6-Chlor-imidazo[2,1-b]thiazol-5-sulfonsäure-(2-methyl-1,2,3,4-tetrahydro-isochinolin-6-yl)-amid-hydrochlorid
[20] 6-(6-Chlor-imidazo[2,1-b]thiazol-5-sulfonylamino)-3,4-dihydro-1H-isochinolin-2-carbonsäure-tert-butyl-ester
[21] 4-Methyl-3,4-dihydro-2H-benzo[1,4]oxazin-7-sulfonsäure-(1,2,3,4-tetrahydro-isochinolin-6-yl)-amid-hydrochlorid
[23] Benzo[1,2,5]thiadiazol-4-sulfonsäure-(1,2,3,4-tetrahydro-isochinolin-6-yl)-amid-hydrochlorid
[24] Benzo[b]thiophen-2-sulfonsäure-(1,2,3,4-tetrahydro-isochinolin-6-yl)-amid-hydrochlorid
[25] 7-Chlor-benzo[1,2,5]oxadiazol-4-sulfonsäure-(1,2,3,4-tetrahydro-isochinolin-6-yl)-amid-hydrochlorid
[26] Benzo[1,2,5]oxadiazol-4-sulfonsäure-(1,2,3,4-tetrahydro-isochinolin-6-yl)-amid-hydrochlorid
[27] 5-Methyl-benzo[1,2,5]thiadiazol-4-sulfonsäure-(1,2,3,4-tetrahydro-isochinolin-6-yl)-amid-hydrochlorid
[28] 7-Methyl-benzo[1,2,5]thiadiazol-4-sulfonsäure-(1,2,3,4-tetrahydro-isochinolin-6-yl)-amid-hydrochlorid
[29] Benzo[b]thiophen-3-sulfonsäure-(1,2,3,4-tetrahydro-isochinolin-6-yl)-amid-hydrochlorid
[30] 4-Fluor-naphthalin-1-sulfonsäure-(1,2,3,4-tetrahydro-isochinolin-6-yl)-amid-hydrochlorid
[31] 4-Chlor-naphthalin-1-sulfonsäure-(1,2,3,4-tetrahydro-isochinolin-6-yl)-amid-hydrochlorid
[32] 5-Dimethylaminonaphthalin-1-sulfonsäure-(1,2,3,4-tetrahydro-isochinolin-6-yl)-amid-hydrochlorid
[33] 2-Oxo-4a,8a-dihydro-2H-chromen-6-sulfonsäure-(1,2,3,4-tetrahydro-isochinolin-6-yl)-amid-hydrochlorid
[34] 2-Methyl-benzothiazol-6-sulfonsäure-(1,2,3,4-tetrahydro-isochinolin-6-yl)-amid-hydrochlorid
[35] 5-Ethyl-2-methoxy-N-(1,2,3,4-tetrahydro-isochinolin-6-yl)-benzol-sulfonamid-hydrochlorid
[36] 6-Methyl-2,4-dioxo-1,2,3,4-tetrahydro-pyrimidin-5-sulfonsäure-(1,2,3,4-tetrahydro-isochinolin-6-yl)-amid-hydrochlorid
[37] N-[4-Ethoxy-3-(1,2,3,4-tetrahydro-isochinolin-6-ylsulfamoyl)-phenyl]-acetamid-hydrochlorid
[38] 4-Methoxy-3-(1,2,3,4-tetrahydro-isochinolin-6-ylsulfamoyl)-benzoesäure-methylester-hydrochlorid
[39] 2-(2,2,2-Trifluor-acetyl)-1,2,3,4-tetrahydro-isochinolin-7-sulfonsäure-(1,2,3,4-tetrahydro-isochinolin-6-yl)-amid-hydrochlorid
[40] 1,2,3,4-Tetrahydro-isochinolin-7-sulfonsäure-(1,2,3,4-tetrahydro-isochinolin-6-yl)-amid-dihydrochlorid
[41] 5-Amino-2-ethoxy-N-(1,2,3,4-tetrahydro-isochinolin-6-yl)-benzolsulfonamid-dihydrochlorid
[42] 5-Chlor-naphthalin-1-sulfonsäure-(1,2,3,4-tetrahydro-isochinolin-6-yl)-amid-hydrochlorid
[43] 1,2-Dimethyl-1H-imidazol-4-sulfonsäure-(1,2,3,4-tetrahydro-isochinolin-6-yl)-amid-hydrochlorid
[44] N-[4-Methyl-5-(1,2,3,4-tetrahydro-isochinolin-6-ylsulfamoyl)-thiazol-2-yl]-acetamid-hydrochlorid
[45] 1,3,5-Trimethyl-1H-pyrazol-4-sulfonsäure-(1,2,3,4-tetrahydro-isochinolin-6-yl)-amid-hydrochlorid
[46] N-[5-(1,2,3,4-Tetrahydro-isochinolin-6-ylsulfamoyl)-naphthalin-1-yl]-acetamid-hydrochlorid
[47] 2-Naphthalin-1-yl-ethansulfonsäure-(1,2,3,4-tetrahydro-isochinolin-6-yl)-amid-hydrochlorid
[48] Dibenzofuran-2-sulfonsäure-(1,2,3,4-tetrahydro-isochinolin-6-yl)-amid-hydrochlorid
[49] 2,5-Dimethoxy-N-(1,2,3,4-tetrahydro-isochinolin-6-yl)-benzolsulfonamid-hydrochlorid
[50] 5-Chlor-2-methoxy-N-(1,2,3,4-tetrahydro-isochinolin-6-yl)-benzolsulfonamid-hydrochlorid
[51] 2,5-Dimethyl-N-(1,2,3,4-tetrahydro-isochinolin-6-yl)-benzolsulfonamid-hydrochlorid
[52] 2-Fluor-5-methyl-N-(1,2,3,4-tetrahydro-isochinolin-6-yl)-benzolsulfonamid-hydrochlorid
[53] 6-Chlor-imidazo[2,1-b]thiazol-5-sulfonsäure-(2-methyl-1,2,3,4-tetrahydro-isochinolin-7-yl)-amid-hydrochlorid
[54] 6-Chlor-imidazo[2,1-b]thiazol-5-sulfonsäure-(1,2,3,4-tetrahydro-isochinolin-7-yl)-amid-hydrochlorid
[55] 4-Methyl-naphthalin-1-sulfonsäure-(1,2,3,4-tetrahydro-isochinolin-7-yl)-amid-hydrochlorid
[56] 4-Methyl-naphthalin-1-sulfonsäure-(2-methyl-1,2,3,4-tetrahydro-isochinolin-7-yl)-amid-hydrochlorid
[57] 5-Chlor-naphthalin-2-sulfonsäure-(2-methyl-1,2,3,4-tetrahydro-isochinolin-7-yl)-amid-hydrochlorid
[58] 5-Chlor-naphthalin-2-sulfonsäure-(1,2,3,4-tetrahydro-isochinolin-7-yl)-amid-hydrochlorid
[59] 4-Methyl-3,4-dihydro-2H-benzo[1,4]oxazin-7-sulfonsäure-(1,2,3,4-tetrahydro-isochinolin-7-yl)-amid-hydrochlorid
[60] 5-Chlor-3-methyl-benzo[b]thiophen-2-sulfonsäure-(1,2,3,4-tetrahydro-isochinolin-7-yl)-amid-hydrochlorid
[61] Naphthalin-1-sulfonsäure-(1,2,3,4-tetrahydro-isochinolin-7-yl)-amid-hydrochlorid
[63] 2,3-Dihydro-benzo[1,4]dioxin-6-sulfonsäure-(1,2,3,4-tetrahydro-isochinolin-7-yl)-amid-hydrochlorid
[64] 5-Fluor-3-methyl-benzo[b]thiophen-2-sulfonsäure-(1,2,3,4-tetrahydro-isochinolin-7-yl)-amid-hydrochlorid
[65] 3-Methyl-chinolin-8-sulfonsäure-(1,2,3,4-tetrahydro-isochinolin-7-yl)-amid-hydrochlorid
[66] Naphthalin-2-sulfonsäure-(1,2,3,4-tetrahydro-isochinolin-7-yl)-amid-hydrochlorid
[67] Benzo[1,2,5]thiadiazol-4-sulfonsäure-(1,2,3,4-tetrahydro-isochinolin-7-yl)-amid-hydrochlorid
[68] 1,4-Dimethyl-2,3-dioxo-1,2,3,4-tetrahydro-chinoxalin-6-sulfonsäure-(1,2,3,4-tetrahydro-isochinolin-7-yl)-amid-hydrochlorid
[69] Benzo[b]thiophen-2-sulfonsäure-(1,2,3,4-tetrahydro-isochinolin-7-yl)-amid-hydrochlorid
[71] 7-Chlor-benzo[1,2,5]oxadiazol-4-sulfonsäure-(1,2,3,4-tetrahydro-isochinolin-7-yl)-amid-hydrochlorid
[72] Benzo[1,2,5]oxadiazol-4-sulfonsäure-(1,2,3,4-tetrahydro-isochinolin-7-yl)-amid-hydrochlorid
[73] 2-Oxo-2,3-dihydro-benzothiazol-6-sulfonsäure-(1,2,3,4-tetrahydro-isochinolin-7-yl)-amid-hydrochlorid
[74] 2-Oxo-2,3-dihydro-benzooxazol-6-sulfonsäure-(1,2,3,4-tetrahydro-isochinolin-7-yl)-amid-hydrochlorid
[76] 5-Methylbenzo[1,2,5]thiadiazol-4-sulfonsäure-(1,2,3,4-tetrahydro-isochinolin-7-yl)-amid-hydrochlorid
[77] 7-Methyl-benzo[1,2,5]thiadiazol-4-sulfonsäure-(1,2,3,4-tetrahydro-isochinolin-7-yl)-amid-hydrochlorid
[78] Benzo[b]thiophen-3-sulfonsäure-(1,2,3,4-tetrahydro-isochinolin-7-yl)-amid-hydrochlorid
[79] Isochinolin-5-sulfonsäure-(1,2,3,4-tetrahydro-isochinolin-7-yl)-amid-hydrochlorid
[80] 4-Fluor-naphthalin-1-sulfonsäure-(1,2,3,4-tetrahydro-isochinolin-7-yl)-amid-hydrochlorid
[81] 4-Chlor-naphthalin-1-sulfonsäure-(1,2,3,4-tetrahydro-isochinolin-7-yl)-amid-hydrochlorid
[82] 2,2-Dimethyl-chroman-6-sulfonsäure-(1,2,3,4-tetrahydro-isochinolin-7-yl)-amid-hydrochlorid
[83] 5-Dimethylamino-naphthalin-1-sulfonsäure-(1,2,3,4-tetrahydro-isochinolin-7-yl)-amid-hydrochlorid
[84] 2-Oxo-2H-chromen-6-sulfonsäure-(1,2,3,4-tetrahydro-isochinolin-7-yl)-amid-hydrochlorid
[85] 2-Methyl-benzothiazol-6-sulfonsäure-(1,2,3,4-tetrahydro-isochinolin-7-yl)-amid-hydrochlorid
[86] 5-Ethyl-2-methoxy-N-(1,2,3,4-tetrahydro-isochinolin-7-yl)-benzolsulfonamid-hydrochlorid
[87] 6-Methyl-2,4-dioxo-1,2,3,4-tetrahydro-pyrimidin-5-sulfonsäure-(1,2,3,4-tetrahydro-isochinolin-7-yl)-amid-hydrochlorid
[88] 6-Chlor-imidazo[2,1-b]thiazol-5-sulfonsäure-ethyl-(2-ethyl-1,2,3,4-tetrahydro-isochinolin-5-yl)-amid-hydrochlorid
[89] 6-Chlor-imidazo[2,1-b]thiazol-5-sulfonsäure-(2-ethyl-1,2,3,4-tetrahydro-isochinolin-5-yl)-amid-hydrochlorid
[90] 6-Chlor-imidazo[2,1-b]thiazol-5-sulfonsäure-(1,2,3,4-tetrahydro-isochinolin-5-yl)-amid-hydrochlorid
[91] 5-Methyl-naphthalin-1-sulfonsäure-(1,2,3,4-tetrahydro-isochinolin-5-yl)-amid-hydrochlorid
[92] Naphthalin-1-sulfonsäure-(1,2,3,4-tetrahydro-isochinolin-5-yl)-amid-hydrochlorid
[93] 5-Chlor-naphthalin-2-sulfonsäure-(1,2,3,4-tetrahydro-isochinolin-5-yl)-amid-hydrochlorid
[94] 4-Methyl-3,4-dihydro-2H-benzo[1,4]oxazin-7-sulfonsäure-(1,2,3,4-tetrahydro-isochinolin-5-yl)-amid-hydrochlorid
[95] 5-Chlor-3-methyl-benzo[b]thiophen-2-sulfonsäure-ethyl-(2-ethyl-1,2,3,4-tetrahydro-isochinolin-5-yl)-amid-hydrochlorid
[96] 5-Chlor-3-methyl-benzo[b]thiophen-2-sulfonsäure-(1,2,3,4-tetrahydro-isochinolin-5-yl)-amid-hydrochlorid
[98] 2,3-Dihydro-benzo[1,4-]dioxin-6-sulfonsäure-(1,2,3,4-tetrahydro-isochinolin-5-yl)-amid-hydrochlorid
[99] 5-Fluor-3-methyl-benzo[b]thiophen-2-sulfonsäure-(1,2,3,4-tetrahydro-isochinolin-5-yl)-amid-hydrochlorid
[100] 3-Methyl-chinolin-8-sulfonsäure-ethyl-(2-ethyl-1,2,3,4-tetrahydro-isochinolin-5-yl)-amid-hydrochlorid
[101] 3-Methyl-chinolin-8-sulfonsäure-(1,2,3,4-tetrahydro-isochinolin-5-yl)-amid-hydrochlorid
[102] Naphthalin-2-sulfonsäure-(1,2,3,4-tetrahydro-isochinolin-5-yl)-amid-hydrochlorid
[103] Benzo[1,2,5]thiadiazol-4-sulfonsäure-(1,2,3,4-tetrahydro-isochinolin-5-yl)-amid-hydrochlorid
[104] Benzo[b]thiophen-2-sulfonsäure-(1,2,3,4-tetrahydro-isochinolin-5-yl)-amid-hydrochlorid
[105] Benzo[1,2,5]oxadiazol-4-sulfonsäure-(1,2,3,4-tetrahydro-isochinolin-5-yl)-amid-hydrochlorid
[106] 7-Chlorbenzo[1,2,5]oxadiazol-4-sulfonsäure-(1,2,3,4-tetrahydro-isochinolin-5-yl)-amid-hydrochlorid
[107] 2-Oxo-2,3-dihydro-benzooxazol-6-sulfonsäure-(1,2,3,4-tetrahydro-isochinolin-5-yl)-amid-hydrochlorid
[108] 5-Methyl-benzo[1,2,5]thiadiazol-4-sulfonsäure-(1,2,3,4-tetrahydro-isochinolin-5-yl)-amid-hydrochlorid
[109] 7-Methyl-benzo[1,2,5]thiadiazol-4-sulfonsäure-(1,2,3,4-tetrahydro-isochinolin-5-yl)-amid-hydrochlorid
[110] Benzo[b]thiophen-3-sulfonsäure-(1,2,3,4-tetrahydro-isochinolin-5-yl)-amid-hydrochlorid
[111] Isochinolin-5-sulfonsäure-(1,2,3,4-tetrahydro-isochinolin-5-yl)-amid-dihydrochlorid
[112] 4-Fluor-naphthalin-1-sulfonsäure-(1,2,3,4-tetrahydro-isochinolin-5-yl)-amid-hydrochlorid
[113] 4-Chlor-naphthalin-1-sulfonsäure-(1,2,3,4-tetrahydro-isochinolin-5-yl)-amid-hydrochlorid
[114] 2,2-Dimethyl-chroman-6-sulfonsäure-(1,2,3,4-tetrahydro-isochinolin-5-yl)-amid-hydrochlorid
[115] 5-Dimethylamino-naphthalin-1-sulfonsäure-(1,2,3,4-tetrahydro-isochinolin-5-yl)-amid-hydrochlorid
[116] 2,2,5,7,8-Pentamethyl-chroman-6-sulfonsäure-(1,2,3,4-tetrahydro-isochinolin-5-yl)-amid-hydrochlorid
[117] 2-Oxo-2H-chromen-6-sulfonsäure-(1,2,3,4-tetrahydro-isochinolin-5-yl)-amid-hydrochlorid
[118] 5-Ethyl-2-methoxy-N-(1,2,3,4-tetrahydro-isochinolin-5-yl)-benzolsulfonamid-hydrochlorid
[119] N-[4-Ethoxy-3-(1,2,3,4-tetrahydro-isochinolin-5-ylsulfamoyl)-phenyl]-acetamid-hydrochlorid
[120] 2-Methoxy-5-methyl-N-(1,2,3,4-tetrahydro-isochinolin-5-yl)-benzolsulfonamid-hydrochlorid
[121] 5-Chlor-naphthalin-1-sulfonsäure-(1,2,3,4-tetrahydro-isochinolin-5-yl)-amid-hydrochlorid
[122] 2-Methoxy-5-methyl-N-(1,2,3,4-tetrahydro-isochinolin-5-yl)-benzolsulfonamid-hydrochlorid
[123] Chinolin-8-sulfonsäure-(1,2,3,4-tetrahydro-isochinolin-5-yl)-amid-hydrochlorid
[124] Dibenzofuran-2-sulfonsäure-(1,2,3,4-tetrahydro-isochinolin-5-yl)-amid-hydrochlorid
[125] 6-Chlor-imidazo[2,1-b]thiazol-5-sulfonsäure-(1,2,3,4-tetrahydro-isochinolin-8-yl)-amid-hydrochlorid
[126] 6-Chlor-N-(2-methyl-1,2,3,4-tetrahydroisochinolin-8-yl)imidazo[2,1-b]thiazol-5-sulfonamid-hydrochlorid
[127] Benzo[b]thiophen-2-sulfonsäure-(1,2,3,4-tetrahydro-isochinolin-8-yl)-amid-hydrochlorid
[128] Benzo[b]thiophen-2-sulfonsäure-(2-methyl-1,2,3,4-tetrahydro-isochinolin-8-yl)-amid-hydrochlorid
[129] Benzo[b]thiophen-3-sulfonsäure-(2-methyl-1,2,3,4-tetrahydro-isochinolin-8-yl)-amid-hydrochlorid
[130] Benzo[b]thiophen-3-sulfonsäure-(1,2,3,4-tetrahydro-isochinolin-8-yl)-amid-hydrochlorid
[131] 5-Chlor-3-methyl-benzo[b]thiophen-2-sulfonsäure-(2-methyl-1,2,3,4-tetrahydro-isochinolin-8-yl)-amid-hydrochlorid
[132] 5-Chlor-3-methyl-benzo[b]thiophen-2-sulfonsäure-(1,2,3,4-tetrahydro-isochinolin-8-yl)-amid-hydrochlorid
[133] Naphthalin-2-sulfonsäure-(1,2,3,4-tetrahydro-isochinolin-8-yl)-amid-hydrochlorid
[134] Naphthalin-2-sulfonsäure-(2-methyl-1,2,3,4-tetrahydro-isochinolin-8-yl)-amid-hydrochlorid
[137] Naphthalin-1-sulfonsäure-(1,2,3,4-tetrahydro-isochinolin-8-yl)-amid-hydrochlorid
[138] Naphthalin-1-sulfonsäure-(2-methyl-1,2,3,4-tetrahydro-isochinolin-8-yl)-amid-hydrochlorid
[139] 4-Methyl-naphthalin-1-sulfonsäure-(2-methyl-1,2,3,4-tetrahydro-isochinolin-8-yl)-amid-hydrochlorid
[140] 4-Methyl-naphthalin-1-sulfonsäure-(1,2,3,4-tetrahydro-isochinolin-8-yl)-amid-hydrochlorid
[141] 4-Chlor-naphthalin-1-sulfonsäure-(1,2,3,4-tetrahydro-isochinolin-8-yl)-amid-hydrochlorid
[142] 4-Chlor-naphthalin-1-sulfonsäure-(2-methyl-1,2,3,4-tetrahydro-isochinolin-8-yl)-amid-hydrochlorid
[143] 5-Chlor-naphthalin-1-sulfonsäure-(2-methyl-1,2,3,4-tetrahydro-isochinolin-8-yl)-amid-hydrochlorid
[144] 5-Chlor-naphthalin-1-sulfonsäure-(1,2,3,4-tetrahydro-isochinolin-8-yl)-amid-hydrochlorid
[145] 5-Chlor-naphthalin-2-sulfonsäure-(1,2,3,4-tetrahydro-isochinolin-8-yl)-amid-hydrochlorid
[146] 5-Chlor-naphthalin-2-sulfonsäure-(2-methyl-1,2,3,4-tetrahydro-isochinolin-8-yl)-amid-hydrochlorid
[147] 4-Methyl-3,4-dihydro-2H-benzo[1,4]oxazin-7-sulfonsäure-(2-methyl-1,2,3,4-tetrahydro-isochinolin-8-yl)-amid-hydrochlorid
[148] 4-Methyl-3,4-dihydro-2H-benzo[1,4]oxazin-7-sulfonsäure-(1,2,3,4-tetrahydro-isochinolin-8-yl)-amid-hydrochlorid
[149] 5-Ethyl-2-methoxy-N-(1,2,3,4-tetrahydro-isochinolin-8-yl)-benzolsulfonamid-hydrochlorid
[150] 5-Ethyl-2-methoxy-N-(2-methyl-1,2,3,4-tetrahydro-isochinolin-8-yl)-benzolsulfonamid-hydrochlorid
[151] 5-Methyl-benzo[1,2,5]thiadiazol-4-sulfonsäure-(2-methyl-1,2,3,4-tetrahydro-isochinolin-8-yl)-amid-hydrochlorid
[152] 5-Methyl-benzo[1,2,5]thiadiazol-4-sulfonsäure-(1,2,3,4-tetrahydro-isochinolin-8-yl)-amid-hydrochlorid
[153] Naphthalin-2-sulfonsäure-(1,2,3,4-tetrahydro-isochinolin-6-yl)-amid und
[154] 5-Chlor-naphthalin-1-sulfonsäure-(1,2,3,4-tetrahydro-isochinolin-7-yl)-amid-hydrochlorid
[155] Naphthalin-2-sulfonsäure-(2-cyclopropancarbonyl-1,2,3,4-tetrahydro-isochinolin-6-yl)-amid
[156] Naphthalin-2-sulfonsäure-(2-cyclopropylmethyl-1,2,3,4-tetrahydro-isochinolin-6-yl)-amid-hydrochlorid
[157] 6-Chlor-imidazo[2,1-b]thiazol-5-sulfonsäure-(2-cyclopropancarbonyl-1,2,3,4-tetrahydro-isochinolin-6-yl)-amid und
[158] 6-Chlor-imidazo[2,1-b]thiazol-5-sulfonsäure-(2-cyclopropylmethyl-1,2,3,4-tetrahydro-isochinolin-6-yl)-amid-hydrochlorid
gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein entsprechendes Salz davon, oder ein entsprechendes Solvat davon, oder eine entsprechende Base davon.

8. Verfahren zur Herstellung einer Verbindung nach einem oder mehreren der Ansprüche 1 bis 7, **dadurch gekennzeichnet, dass** wenigstens eine Verbindung der allgemeinen Formel IVh 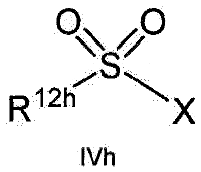 worin R^{12h} die Bedeutung gemäß einem oder mehreren der Ansprüche 1 bis 7 hat und X eine Abgangsgruppe, vorzugsweise ein Halogenatom, besonders bevorzugt ein Chloratom, bedeutet, mit wenigstens einer Verbindung der allgemeinen Formel VhA 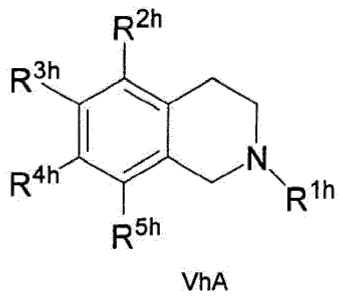 worin R^{1h} bis R^{5h} die Bedeutung gemäß einem oder mehreren der Ansprüche 1 bis 7 haben, mit der Maßgabe, dass wenigstens ein Substituent der Gruppe bestehend aus R^{2h}, R^{3h}, R^{4h} und R^{5h} eine -N(H)(R^{11h}) -Einheit bedeutet, worin R^{11h} die Bedeutung gemäß einem oder mehreren der Ansprüche 1 bis 7 hat, oder einem geschützten Derivat davon, in einem Reaktionsmedium, vorzugsweise in Gegenwart von wenigstens einer Base, umgesetzt wird.

9. Medikament, umfassend wenigstens eine substituierte Tetrahydroisochinolinverbindung der allgemeinen Formel Ih gemäß einem oder mehreren der Ansprüche 1 bis 7, gegebenenfalls in Form von einem von ihren Stereoisomeren, vorzugsweise Enantiomeren oder Diastereomeren, einem Racemat, oder in Form von einer Mischung von wenigstens zwei von ihren Stereoisomeren, vorzugsweise Enantiomeren und/oder Diastereomeren, in einem beliebigen Mischungsverhältnis, oder ein Salz davon, oder ein entsprechendes Solvat davon und gegebenenfalls wenigstens ein physiologisch verträgliches Hilfsmittel.

10. Medikament nach Anspruch 9 für die Prophylaxe und/oder Behandlung einer Störung oder Krankheit, die mit der Nahrungsmittelaufnahme zusammenhängt, vorzugsweise für die Regulierung des Appetits, für die Beibehaltung, Erhöhung oder Verringerung des Körpergewichts, für die Prophylaxe und/oder Behandlung von Fettleibigkeit, Bulimie, Anorexie, Kachexie, Typ II-Diabetes (nicht-insulinabhängiger Diabetes mellitus), vorzugsweise Typ II-Diabetes, der durch Fettleibigkeit verursacht ist; für die Prophylaxe und/oder Behandlung von einem Schlaganfall; Migräne, einem Kopftrauma; Epilepsie; dem Reizkolonsyndrom, dem Reizdarmsyndrom; Emesis; Vertigo; Störungen des zentralen Nervensystems; Angst; Panikattacken; einer Depression; bipolaren Störungen; einer Zwangsstörung; kognitiven Störungen; einer kognitiven Dysfunktion in Zusammenhang mit psychiatrischen Erkrankungen; Gedächtnisstörungen; seniler Demenz; affektiven Psychosen; Schlafstörungen; einer Psychose; neurodegenerativen Störungen, vorzugsweise ausgewählt aus der Gruppe bestehend aus Alzheimer-Krankheit, Parkinson-Krankheit, Chorea Huntington und multipler Sklerose; Schizophrenie; Amnesie; Autismus; sexueller Dysfunktion; gastrischen Motilitätsstörungen; Störungen des zirkadianen Rhythmus; chronisch intermittierender Hypoxie; Krämpfen; oder einer Hyperaktivitätsstörung (ADHD, Aufmerksamkeitsdefizit/Hyperaktivitätsstörung); für eine Verbesserung des Erkennungsvermögens (kognitive Verbesserung) oder des kognitiven Gedächtnisses (Verbesserung des kognitiven Gedächtnisses); für die Prophylaxe und/oder Behandlung von Drogenabhängigkeit und/oder -entzug; für die Prophylaxe und/oder Behandlung von Alkoholabhängigkeit und/oder -entzug, für die Prophylaxe und/oder Behandlung von Nikotinabhängigkeit und/oder -entzug.

11. Verwendung von wenigstens einer substituierten Tetrahydroisochinolinverbindung der allgemeinen Formel Ih gemäß einem oder mehreren der Ansprüche 1 bis 7 für die Herstellung eines Medikaments für die Prophylaxe und/oder Behandlung einer Störung oder Krankheit, die mit der Nahrungsmittelaufnahme zusammenhängt, vorzugsweise für die Regulierung des Appetits, für die Beibehaltung, Erhöhung oder Verringerung des Körpergewichts, für die Prophylaxe und/oder Behandlung von Fettleibigkeit, Bulimie, Anorexie, Kachexie, Typ II-Diabetes (nicht-insulinabhängiger Diabetes mellitus), vorzugsweise Typ II-Diabetes, der durch Fettleibigkeit verursacht ist; für die Prophylaxe und/oder Behandlung von einem Schlaganfall; Migräne, einem Kopftrauma; Epilepsie; dem Reizkolonsyndrom, dem Reizdarmsyndrom; Emesis; Vertigo; Störungen des zentralen Nervensystems; Angst; Panikattacken; einer Depression; bipolaren Störungen; einer Zwangsstörung; kognitiven Störungen; einer kognitiven Dysfunktion in Zusammenhang mit psychiatrischen Erkrankungen; Gedächtnisstörungen; seniler Demenz; affektiven Psychosen; Schlafstörungen; einer Psychose; neurodegenerativen Störungen, vorzugsweise ausgewählt aus der Gruppe bestehend aus Alzheimer-Krankheit, Parkinson-Krankheit, Chorea Huntington und multipler Sklerose; Schizophrenie, Amnesie; Autismus; sexueller Dysfunktion; gastrischen Motilitätsstörungen; Störungen des zirkadianen Rhythmus; chronisch intermittierender Hypoxie; Krämpfen; oder einer Hyperaktivitätsstörung (ADHD, Aufmerksamkeitsdefizit/Hyperaktivitätsstörung); für eine Verbesserung des Erkennungsvermögens (kognitive Verbesserung) oder des kognitiven Gedächtnisses (Verbesserung des kognitiven Gedächtnisses); für die Prophylaxe und/oder Behandlung von Drogenabhängigkeit und/oder -entzug; für die Prophylaxe und/oder Behandlung von Alkoholabhängigkeit und/oder -entzug, für die Prophylaxe und/oder Behandlung von Nikotinabhängigkeit und/oder -entzug.

12. Substituierte Tetrahydroisochinolinverbindung der allgemeinen Formel Ig 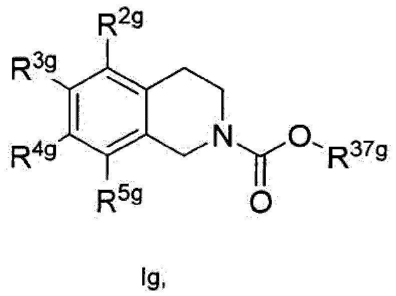 worin
R^{1g} bedeutet ein Wasserstoffatom; einen Rest, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ und -CH₂-CH₂-CH₂-NH-C₂H₅; oder einen (hetero)cycloaliphatischen Rest, ausgewählt aus der Gruppe bestehend aus Imidazolidinyl, Aziridinyl, Azetidinyl, Pyrrolidinyl, Piperidinyl, Morpholinyl, Thiomorpholinyl, Piperazinyl, Pyrazolidinyl und Azepanyl, welcher über eine -(CH₂)_{1, 2 oder 3}- Gruppe gebunden sein kann und welcher unsubstituiert oder gegebenenfalls mit 1, 2, 3, 4 oder 5 Substituenten substituiert sein kann, die unabhängig ausgewählt sind aus der Gruppe bestehend aus Oxo (=O), Thioxo (=S), Methyl, Ethyl, n-Propyl, Isopropyl, n-Butyl, tert-Butyl, sec-Butyl, Isobutyl, -C(=O)-CH₃, -C(=O)-C₂H₅, -C(=O)-CH₂-CH₂-CH₃, -C(=O)-CH(CH₃)₂, -C(=O)-C(CH₃)₃, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃;
R^{2g}, R^{3g}, R^{4g} und R^{5g} bedeuten jeweils unabhängig voneinander ein Wasserstoffatom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -OR^{8g}; -SR^{9g}; -N(R^{11g})-S(=O)₂-R^{12g}; einen Rest, ausgewählt aus der Gruppe bestehend aus Methyl, Ethyl, n-Propyl, Isopropyl, n-Butyl, sec-Butyl, Isobutyl, tert-Butyl, Vinyl, Allyl, Ethinyl, -CF₃, -CFH₂, -CF₂H, -CH₂-CF₃ und -CF₂-CF₃; oder einen Rest, ausgewählt aus der Gruppe bestehend aus Cyclopropyl, Cyclobutyl, Cyclopentyl und Cyclohexyl;
mit der Maßgabe, dass wenigstens einer von den Substituenten R^{2g}, R^{3g}, R^{4g} und R^{5g} eine -N(R^{11g})-S(=O)₂-R^{12g}-Einheit bedeutet;
R^{8g} und R^{9g} bedeuten jeweils unabhängig voneinander einen Rest, ausgewählt aus der Gruppe bestehend aus Methyl, Ethyl, n-Propyl, Isopropyl, n-Butyl, sec-Butyl, Isobutyl, tert-Butyl, -CF₃, -CFH₂, -CF₂H, -CH₂-CF₃ und -CF₂-CF₃; einen (hetero)cycloaliphatischen Rest, ausgewählt aus der Gruppe bestehend aus Cyclopropyl, Cyclobutyl, Cyclopentyl und Cyclohexyl; oder einen Aryl- oder Heteroarylrest, ausgewählt aus der Gruppe bestehend aus Phenyl, Naphthyl, Furyl (Furanyl), Thienyl (Thiophenyl), Pyrrolyl und Pyridinyl, welcher über eine -(CH₂)₁, _{2 oder 3}- Gruppe gebunden sein kann und welcher unsubstituiert oder gegebenenfalls mit 1, 2 oder 3 Substituenten substituiert sein kann, die unabhängig ausgewählt sind aus der Gruppe bestehend aus Methyl, Ethyl, n-Propyl, Isopropyl, n-Butyl, tert-Butyl, sec-Butyl, Isobutyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF₃, -OH, -SH, -NH₂, -NO₂, -CHO, -CF₂H und -CFH₂;
R^{11g} bedeutet ein Wasserstoffatom, -S(=O)₂-R^{12g} oder einen Alkylrest, ausgewählt aus der Gruppe bestehend aus Methyl, Ethyl, n-Propyl, Isopropyl, n-Butyl, sec-Butyl, Isobutyl und tert-Butyl;
R^{12g} bedeutet einen Phenylrest der allgemeinen Formel (Ag)
worin 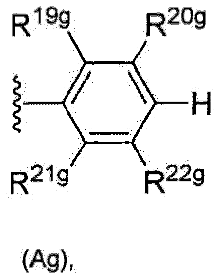 R^{19g}, R^{20g}, R^{21g} und R^{22g} bedeuten jeweils unabhängig voneinander ein Wasserstoffatom; F, Cl, Br, I, -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{23g}; -S(=O)-R^{24g}; -S(=O)₂-R^{24g}; -OR^{25g}; -SR^{26g}; -C(=O)-OR^{27g}; Methyl, Ethyl, n-Propyl, Isopropyl, n-Butyl, Isobutyl, sec-Butyl, tert-Butyl, -CF₃, -CF₂H, -CFH₂, -CH₂-CF₃, -CF₂-CF₃, Cyclopropyl, Cyclobutyl, Cyclopentyl und Cyclohexyl;
mit der Maßgabe, dass wenigstens einer von den Substituenten R^{19g}, R^{20g}, R^{21g} und R^{22g} kein Wasserstoff ist;
oder einen Naphthylrest, welcher unsubstituiert und gegebenenfalls mit 1, 2, 3, 4 oder 5 Substituenten substituiert sein kann, die unabhängig ausgewählt sind aus der Gruppe bestehend aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, -NO₂, -CHO, -CF₂H und -CFH₂;
R^{23g} und R^{27g} bedeuten jeweils unabhängig voneinander einen Rest, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, -CF₃, -CFH₂, -CF₂H, -CH₂-CF₃ und -CF₂-CF₃; oder einen Aryl- oder Heteroarylrest, ausgewählt aus der Gruppe bestehend aus Phenyl, Naphthyl, Furyl (Furanyl), Thienyl (Thiophenyl), Pyrrolyl und Pyridinyl, welcher über eine -(CH₂)_{1, 2 oder 3}-Gruppe gebunden sein kann und welcher unsubstituiert oder gegebenenfalls mit 1, 2 oder 3 Substituenten substituiert sein kann, die unabhängig ausgewählt sind aus der Gruppe bestehend aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, F, Cl, Br, I, -CN, -CF₃, -OCF₃, -SCF₃, -OH, -SH, -NH₂-NO₂, -CHO, -CF₂H und -CFH₂;
R^{24g} und R^{26g} bedeuten jeweils unabhängig voneinander einen Rest, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, Vinyl, Allyl, Ethinyl, -CF₃, -CFH₂, -CF₂H, -CH₂-CF₃ und -CF₂-CF₃; oder einen Aryl- oder Heteroarylrest, ausgewählt aus der Gruppe bestehend aus Phenyl, Naphthyl, Furyl (Furanyl), Thienyl (Thiophenyl), Pyrrolyl und Pyridinyl, welcher unsubstituiert oder gegebenenfalls mit 1, 2 oder 3 Substituenten substituiert sein kann, die unabhängig ausgewählt sind aus der Gruppe bestehend aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, F, Cl, Br, I, -CN, -CF₃, -OCF₃, -SCF₃, -OH, -SH, -NH₂, -NO₂, -CHO, -CF₂H und -CFH₂;
R^{25g} bedeutet einen Rest, ausgewählt aus der Gruppe bestehend aus Methyl, Ethyl, n-Propyl, Isopropyl, n-Butyl, sec-Butyl, Isobutyl, tert-Butyl, -CF₃, -CFH₂, -CF₂H, -CH₂-CF₃ und -CF₂-CF₃
und R^{37g} bedeutet einen Rest, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, Fluorenyl, Fluorenylmethyl, Phenyl, Benzyl und Naphthyl.

13. Verbindung nach Anspruch 12, **dadurch gekennzeichnet, dass**
R^{1g} ein Wasserstoffatom; oder einen Rest, ausgewählt aus der Gruppe bestehend aus Methyl, Ethyl, n-Propyl, n-Butyl, n-Pentyl und n-Hexyl, bedeutet;
R^{2g}, R^{3g}, R^{4g} und R^{5g} jeweils unabhängig voneinander ein Wasserstoffatom oder -N(R^{11g})-S(=O)₂-R^{12g} bedeuten;
mit der Maßgabe, dass wenigstens einer von den Substituenten R^{2g}, R^{3g}, R^{4g} und R^{5g} eine -N(R^{11g})-S(=O)₂-R^{12g}-Einheit bedeutet;
R^{11g} ein Wasserstoffatom, -S(=O)₂-R^{12g} oder einen Alkylrest, ausgewählt aus der Gruppe bestehend aus Methyl, Ethyl und n-Propyl, bedeutet;
R^{12g} einen Phenylrest der allgemeinen Formel (Ag) 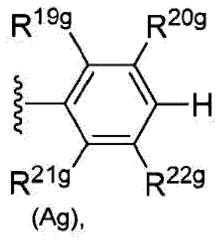 worin
R^{19g}, R^{20g}, R^{21g} und R^{22g} jeweils unabhängig voneinander ein Wasserstoffatom;
F, Cl, Br, I, -O-CH₃; -O-C₂H₅; -O-CF₃; -O-CFH₂; -O-CF₂H; -O-CH₂-CF₃; -O-CF₂-CF₃; Methyl, Ethyl, n-Propyl, Isopropyl, n-Butyl, Isobutyl, sec-Butyl und tert-Butyl bedeuten;
mit der Maßgabe, dass wenigstens einer von den Substituenten R^{19g}, R^{20g}, R^{21g} und R^{22g} kein Wasserstoff ist;
oder einen Naphthylrest bedeutet, welcher unsubstituiert oder gegebenenfalls mit 1, 2, 3, 4 oder 5 Substituenten, unabhängig ausgewählt aus der Gruppe bestehend aus Methyl, Ethyl, n-Propyl, Isopropyl, F, Cl und Br, substituiert sein kann;
und R^{37g} einen Rest, ausgewählt aus der Gruppe bestehend aus Methyl, Ethyl, n-Propyl, Isopropyl, n-Butyl, sec-Butyl, Isobutyl, tert-Butyl, n-Pentyl, n-Hexyl, Fluorenyl, Fluorenylmethyl, Phenyl, Benzyl und Naphthyl, bedeutet.

14. Verbindung nach Anspruch 12 oder 13, ausgewählt aus der Gruppe bestehend aus [13] 6-(Naphthalin-1-sulfonylamino)-3,4-dihydro-1H-isochinolin-2-carbonsäure-tert-butylester,
[15] 6-(4-Methyl-naphthalin-1-sulfonylamino)-3,4-dihydro-1H-isochinolin-2-carbonsäure-tert-butyl-ester,
[16] 6-(Naphthalin-2-sulfonylamino)-3,4-dihydro-1H-isochinolin-2-carbonsäure-tert-butylester und
[17] 6-(2-Methoxy-5-methyl-benzolsulfonylamino)-3,4-dihydro-1H-isochinolin-2-carbonsäure-tert-butyl-ester.

## Revendications

1. Composé de tétrahydroisoquinoléine substitué de la formule générale Ih, 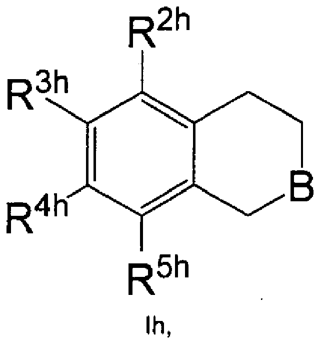 où
B représente un radical sélectionné dans le groupe consistant en 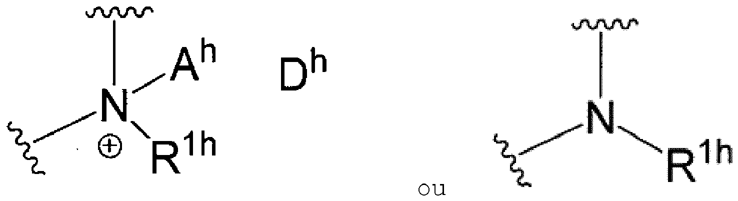
A^{h} représente un atome d'hydrogène ou un radical sélectionné dans le groupe consistant en méthyle, éthyle, n-propyle, isopropyle, n-butyle, isobutyle, 2-butyle et tert-butyle;
D^{h} représente un anion sélectionné dans le groupe consistant en chlorure, bromure, iodure, fluorure, hydrogènesulfate, nitrate, dihydrogènephosphate, thiocyanate, cyanate, acrylate, méthanesulfonate, éthanesulfonate, toluènesulfonate et benzènesulfonate;
R^{1h} représente un radical sélectionné dans le groupe consistant en H, méthyle, éthyle, -C(=O)-cyclopropyle, -C(=O)-O-tert-butyle et -CH₂-cyclopropyle;
R^{2h}, R^{3h}, R^{4h} et R^{5h}, indépendamment les uns des autres, représentent chacun un atome d'hydrogène ou un radical -N(R^{11h})-S(=O)₂-R^{12h};
à condition qu'au moins un des substituants R^{2h}, R^{3h}, R^{4h} et R^{5h} représente un fragment -N(R^{11h})-S(=O)₂-R^{12h};
R^{11h} représente un radical sélectionné dans le groupe consistant en H, méthyle et éthyle;
R^{12h} représente un radical sélectionné dans le groupe consistant en 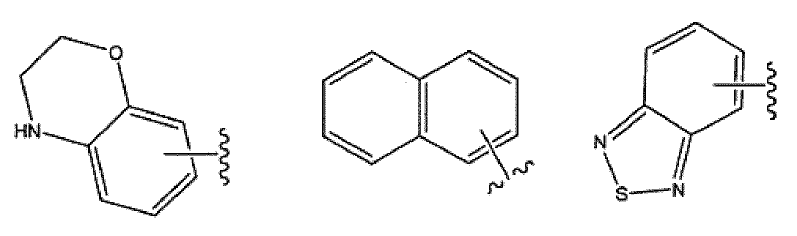 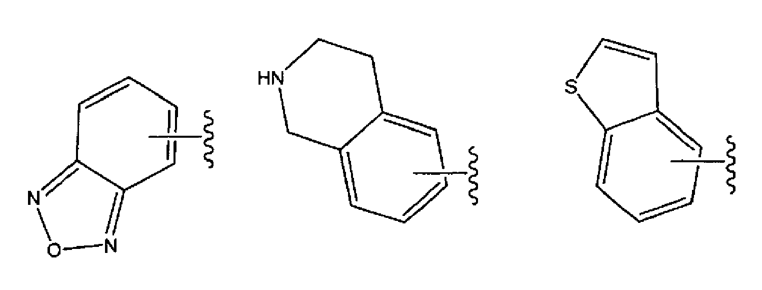 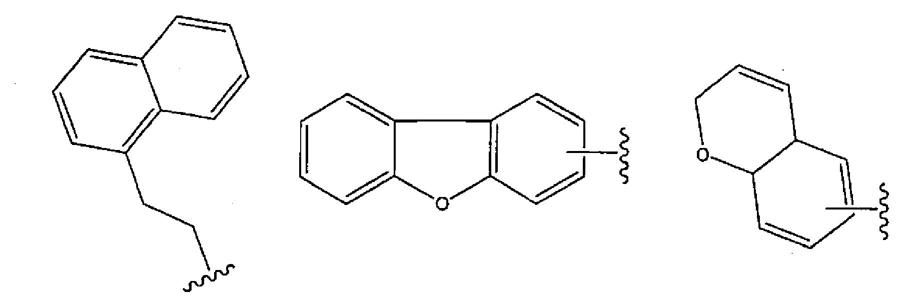 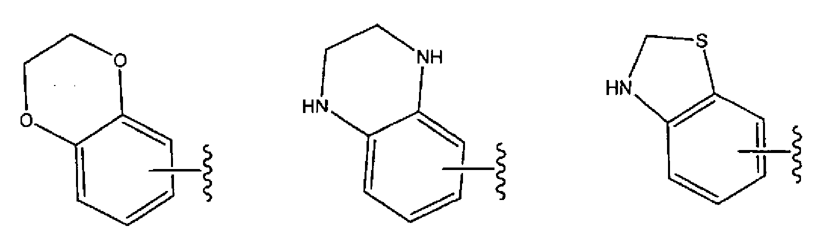 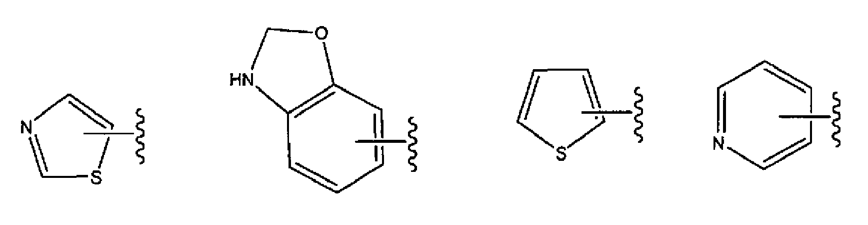 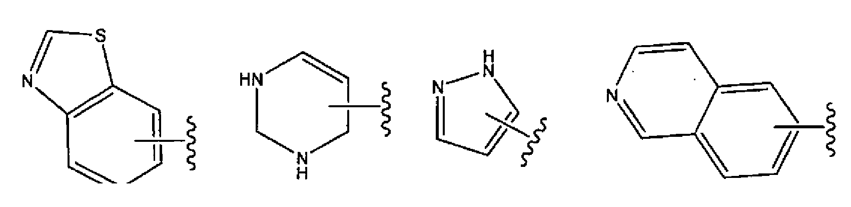 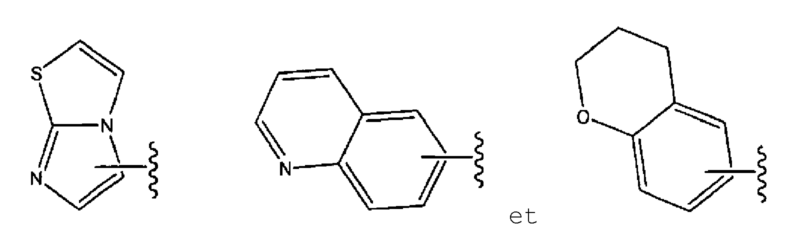
qui est non substitué ou substitué avec 1,2,3,4 ou 5 substituants indépendamment sélectionnés dans le groupe consistant en H, F, Cl, méthyle, éthyle, NH₂, -N(CH₃)₂, oxo(=O), -O-CH₃, -O-CH₂-CH₃, -NH-(C=O)-CH₃, C(=O)-O-CH₃, - C(=O)-CF₃; où la substitution peut avoir lieu sur n'importe quelle position appropriée dans les radicaux mentionnés avant, incluant le ou les hétéroatomes;
ou un radical phényle substitué sélectionné dans le groupe consistant en: 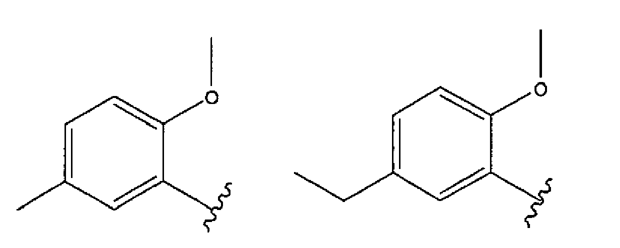 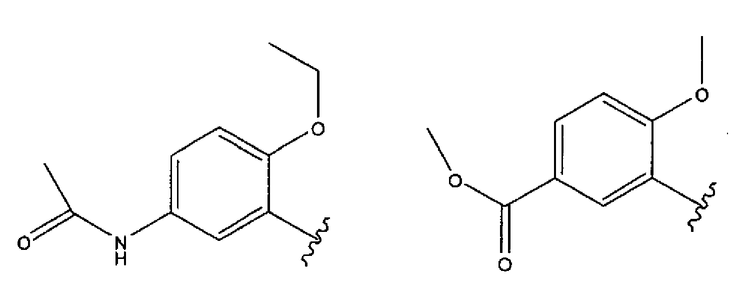 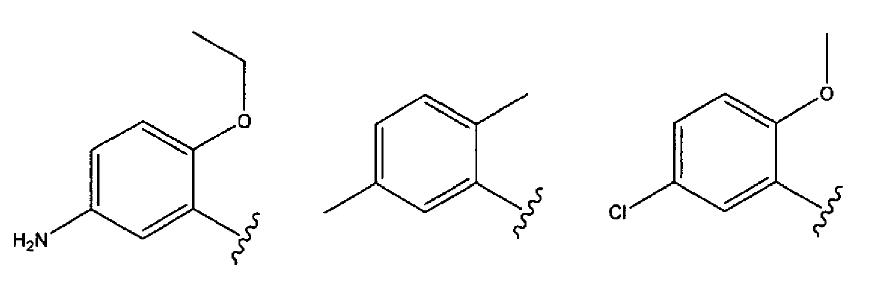 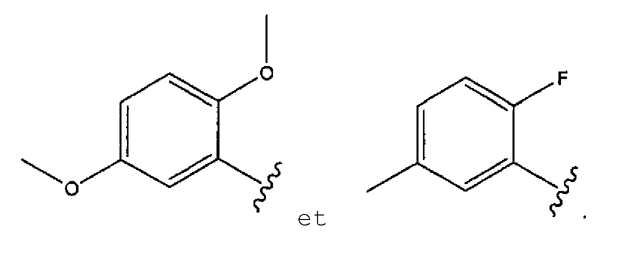 optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

2. Composé selon la revendication 1, **caractérisé en ce que**
B, A^{h}, D^{h}, R^{1h}, R^{2h}, R^{3h}, R^{4h}, R^{5h} et R^{11h} ont la signification telle que définie dans la revendication 1;
à condition qu'au moins un des substituants R^{2h}, R^{3h}, R^{4h} et R^{5h} représentent un fragment -N(R^{11h})-S(=O)₂-R^{12h};
et
R^{12h} représente un radical sélectionné dans le groupe consistant en 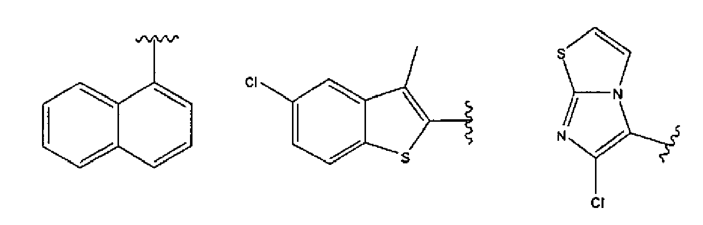 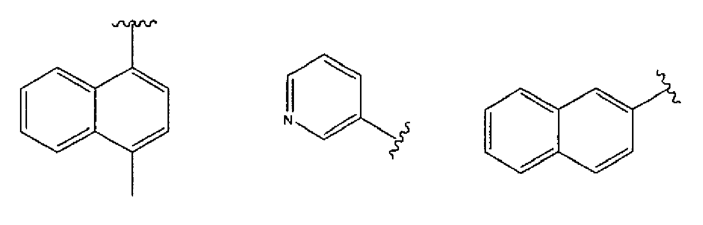 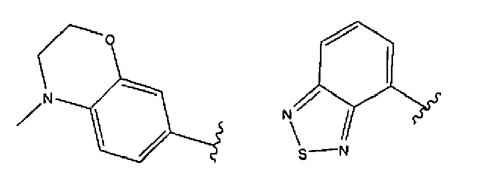 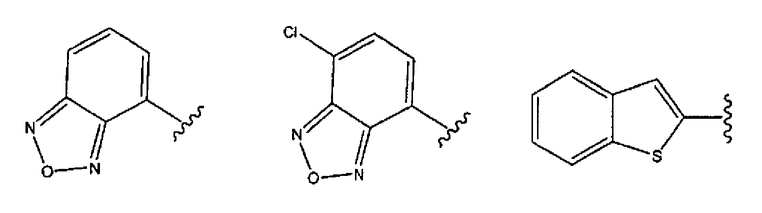 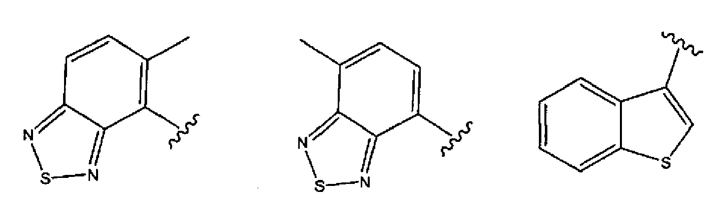 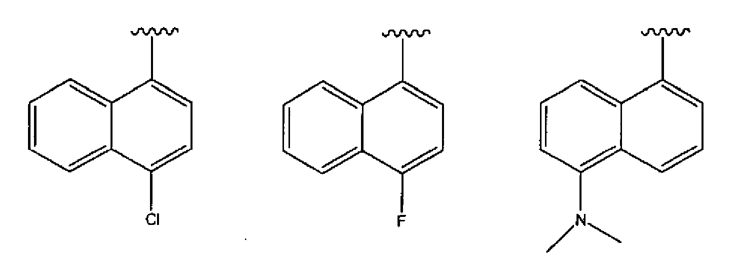 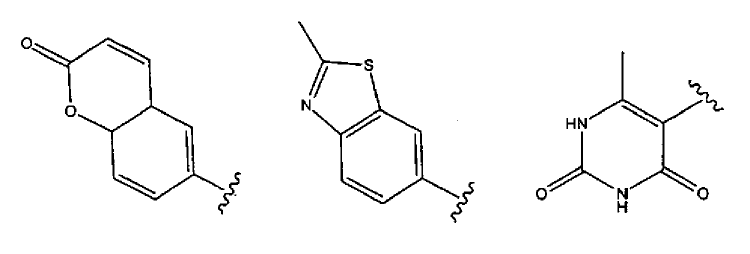 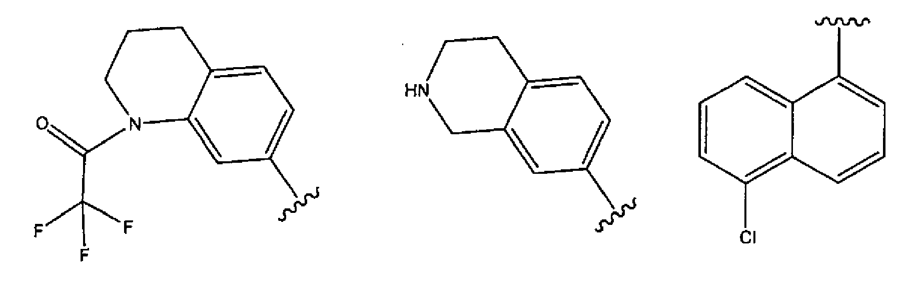 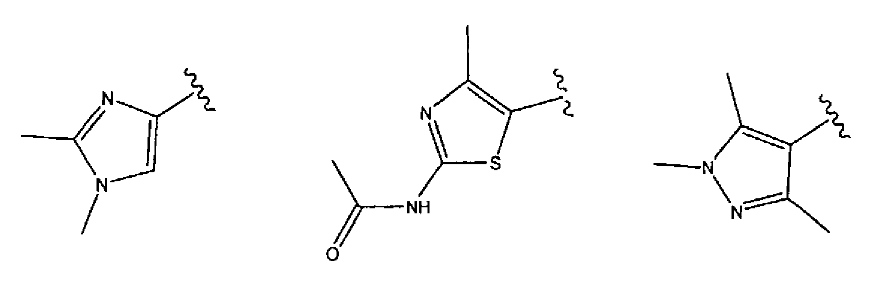 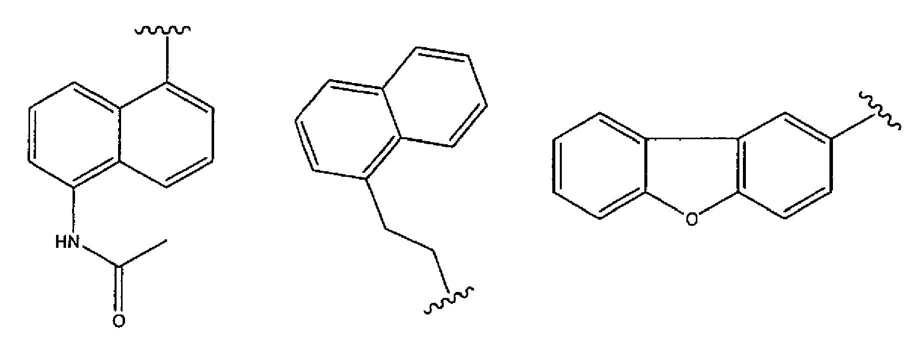 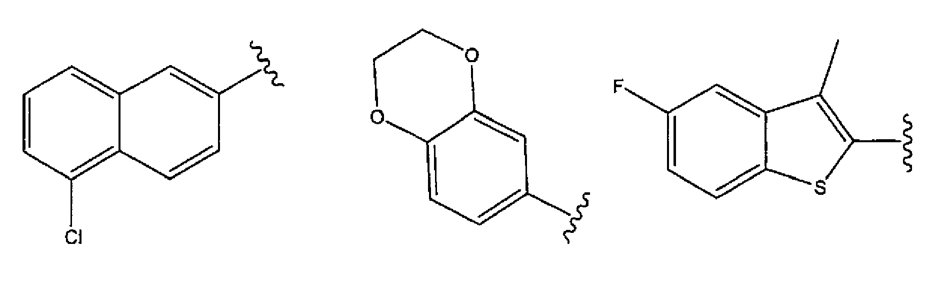 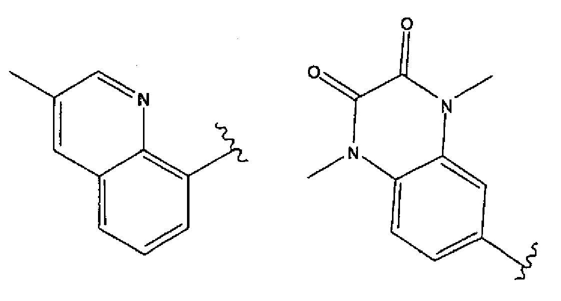 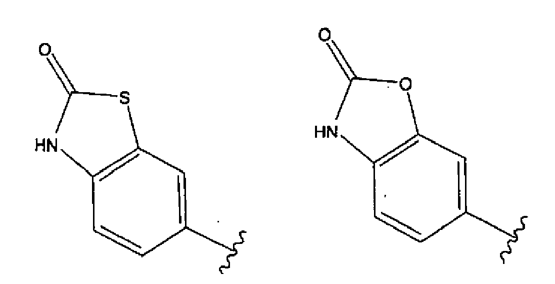 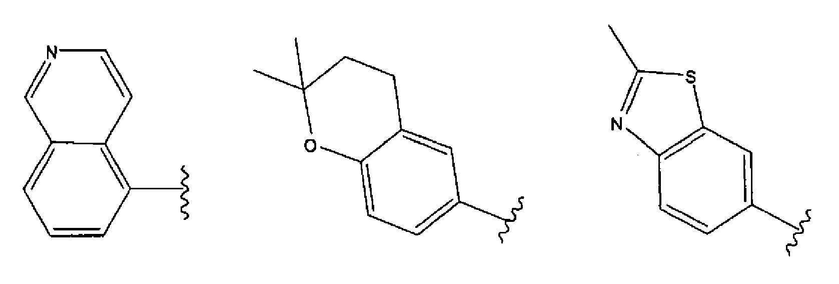 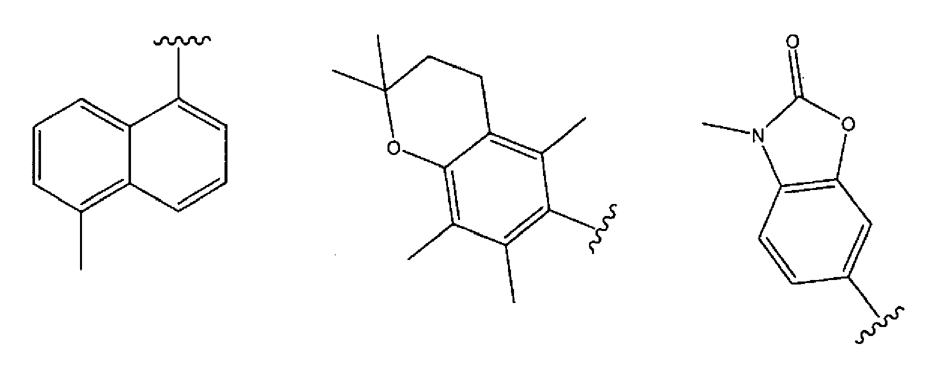 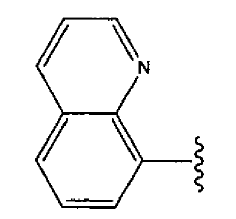 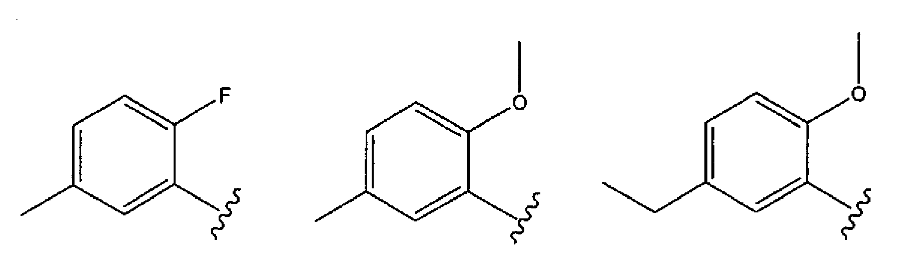 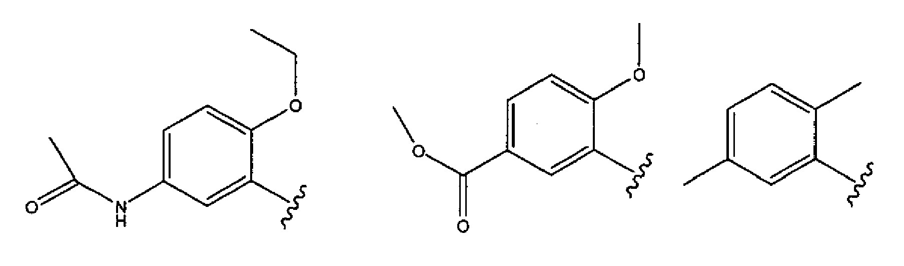 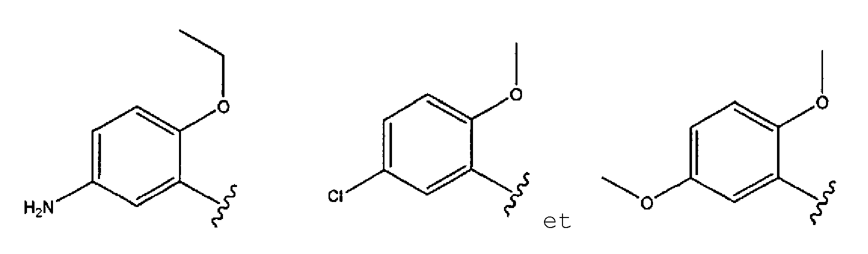 optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

3. Composé selon la revendication 1 ou 2, **caractérisé en ce que** R^{2h} représente un fragment -N(R^{11h})-S (=O)₂-R^{12h};
et
B, A^{h}, D^{h}, R^{1h}, R^{3h}, R^{4h}, R^{5h}, R^{11h} et R^{12h} ont la signification telle que définie dans la revendication 1;
optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

4. Composé selon la revendication 1 ou 2, **caractérisé en ce que**
R^{3h} représente un fragment -N(R^{11h})-S(=O)₂-R^{12h};
et
B, A^{h}, D^{h}, R^{1h}, R^{2h}, R^{4h}, R^{5h}, R^{11h} et R^{12h} ont la signification telle que définie dans la revendication 1;
optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

5. Composé selon la revendication 1 ou 2, **caractérisé en ce que**
R^{4h} représente un fragment -N(R^{11h})-S(=O)₂-R^{12h};
et
B, A^{h}, D^{h}, R^{1h}, R^{2h}, R^{3h}, R^{5h}, R^{11h} et R^{12h} ont la signification telle que définie dans la revendication 1;
optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

6. Composé selon la revendication 1 ou 2, **caractérisé en ce que**
R^{5h} représente un fragment -N(R^{11h})-S(=O)₂-R¹²h;
et
B, A^{h}, D^{h}, R^{1h}, R^{2h}, R^{3h}, R^{4h}, R^{11h} et R^{12h} ont la signification telle que définie dans la revendication 1;
optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

7. Composé selon l'une ou plusieurs des revendications 1 à 6, sélectionné dans le groupe consistant en
[1] N-(1,2,3,4-tétrahydroisoquinolin-6-yl)naphthalène-1-sulfonamide chlorhydrate,
[2] 2,2-diméthyl-6-(N-méthylnaphthalène-1-sulfonamido)-1,2,3,4-tétrahydroisoquinolinium iodure,
[3] N-(2-méthyl-1,2,3,4-tétrahydroisoquinolin-6-yl)naphthalène-1-sulfonamide chlorhydrate,
[4] 5-chloro-3-méthyl-N-(1,2,3,4-tétrahydroisoquinolin-6-yl)benzo[b]thiophène-2-sulfonamide chlorhydrate,
[5] 5-chloro-3-méthyl-N-(2-méthyl-1,2,3,4-tétrahydroisoquinolin-6-yl)benzo[b]thiophène-2-sulfonamide chlorhydrate,
[6] 4-méthyl-N-(1,2,3,4-tétrahydroisoquinolin-6-yl)naphthalène-1-sulfonamide chlorhydrate,
[7] 4-méthyl-N-(2-méthyl-1,2,3,4-tétrahydroisoquinolin-6-yl)naphthalène-1-sulfonamide chlorhydrate,
[8] N-(1,2,3,4-tetrahydroisoquinolin-6-yl)naphthalene-2-sulfonamide chlorhydrate,
[9] N-(2-methyl-1,2,3,4-tetrahydroisoquinolin-6-yl)naphthalène-2-sulfonamide chlorhydrate,
[10] 6-chloro-N-(1,2,3,4-tétrahydroisoquinolin-6-yl)imidazo[2,1-b]thiazole-5-sulfonamide chlorhydrate,
[11] 2-méthoxy-5-méthyl-N-(1,2,3,4-tétrahydroisoquinolin-6-yl)benzènesulfonamide chlorhydrate,
[12] N-(1,2,3,4-tétrahydroisoquinolin-6-yl)pyridine-3-sulfonamide dichlorhydrate,
[13] tert-butyl ester de l'acide 6-(naphthalène-1-sulfonylamino)-3,4-dihydro-1H-isoquinoline-2-carboxylique,
[14] tert-butyl ester de l'acide 6-(5-chloro-3-méthyl-benzo[b]thiophène-2-sulfonylamino)-3,4-dihydro-1H-isoquinoline-2-carboxylique,
[15] tert-butyl ester de l'acide 6-(4-méthyl-naphthalène-1-sulfonylamino)-3,4-dihydro-1H-isoquinoline-2-carboxylique;
[16] tert-butyl ester de l'acide 6-(naphthalène-2-sulfonylamino)-3,4-dihydro-1H-isoquinoline-2-carboxylique,
[17] tert-butyl ester de l'acide 6-(2-méthoxy-5-méthyl-benzènesulfonylamino)-3,4-dihydro-1H-isoquinoline-2-carboxylique,
[18] tert-butyl ester de l'acide 6-(pyridine-3-sulfonylamino)-3,4-dihydro-1H-isoquinoline-2carboxylique;
[19] (2-méthyl-1,2,3,4tétrahydro-isoquinolin-6-yl)-amide chlorhydrate de l'acide 6-chloro-imidazo[2,1-b] thiazole-5-sulfonique
[20] tert-butyl ester de l'acide 6-(6-chloro-imidazo [2,1-b]thiazole-5-sulfonylamino)-3,4-dihydro-1hisoquinoline-2-carboxylique
[21] (1,2,3,4-tetrahydro-isoquinolin-6-yl)-amide chlorhydrate de l'acide 4-méthyl-3,4-dihydro-2H-benzo[1,4]oxazine-7-sulfonique
[23] (1,2,3,4-tétrahydro-isoquinolin-6-yl)-amide chlorhydrate de l'acide benzo[1,2,5]thiadiazole-4-sulfonique
[24] (1,2,3,4-tetrahydro-isoquinolin- 6-yl)-amide chlorhydrate de l'acide benzo[b]thiophène-2-sulfonique
[25] (1,2,3,4- tetrahydro-isoquinolin-6-yl)-amide chlorhydrate de l'acide 7-chloro-benzo[1,2,5] oxadiazole-4-sulfonique
[26] (1,2,3,4-tétrahydro-isoquinolin-6-yl)-amide chlorhydrate de l'acide benzo[1,2,5]oxadiazole-4-sulfonique
[27] (1,2,3,4-tétrahydro-isoquinolin-6-yl)-amide chlorhydrate de l'acide 5-méthyl-benzo[1,2,5] thiadiazole-4-sulfonique
[28] (1,2,3,4-tétrahydro-isoquinolin-6-yl)-amide chlorhydrate de l'acide 7-méthyl-benzo[1,2,5]thiadiazole-4-sulfonique
[29] (1,2,3,4-tétrahydro-isoquinolin- 6-yl)-amide chlorhydrate de l'acide nenzo[b]thiophène-3-sulfonique
[30] (1,2,3,4-tétrahydro-isoquinolin-6-yl)-amide chlorhydrate de l'acide 4-fluoro-naphthalène-1-sulfonique
[31] (1,2,3,4-tétrahydro-isoquinolin-6-yl)-amide chlorhydrate de l'acide 4-chloro-naphthalène-1-sulfonique
[32] (1,2,3,4-tétrahydro-isoquinolin -6-yl)-amide chlorhydrate de l'acide 5-diméthylaminonaphthalène-1-sulfonique
[33] (1,2,3,4-tétrahydro-isoquinolin-6-yl)-amide chlorhydrate de l'acide 2-oxo-4a,8a-dihydro-2H-chromène-6-sulfonique
[34] (1,2,3,4-tétrahydro-isoquinolin-6-yl)-amide chlorhydrate de l'acide 2-méthyl-benzothiazole-6-sulfonique
[35] 5-éthyl-2-méthoxy-N-(1,2,3,4-tétrahydro-isoquinolin-6-yl)-benzène sulfonamide chlorhydrate
[36] (1,2,3,4-tétrahydro-isoquinolin-6-yl)-amide chlorhydrate de l'acide 6-méthyl-2,4-dioxo-1,2,3,4-tétrahydro-pyrimidine-5-sulfonique
[37] N-[4-éthoxy-3-(1,2,3,4-tétrahydro-isoquinolin-6-ylsulfamoyl)-phényl]-acétamide chlorhydrate
[38] méthyl ester chlorhydrate de l'acide 4-méthoxy-3-(1,2,3,4-tétrahydro-isoquinolin-6-yl sulfamoyl)-benzoique
[39] (1,2,3,4-tétrahydro-isoquinolin-6-yl)-amide chlorhydrate de l'acide 2-(2,2,2-trifluoro-acétyl)-1,2,3,4-tétrahydro-isoquinoline-7-sulfonique
[40] (1,2,3,4-tétrahydro-isoquinolin-6-yl)-amide dichlorhydrate de l'acide 1,2,3,4-tétrahydro-isoquinoline-7-sulfonique
[41] 5-Amino-2-éthoxy-N-(1,2,3,4-tétrahydro-isoquinolin-6-yl)-benzènesulfonamide dichlorhydrate
[42] (1,2,3,4-tétrahydro-isoquinolin-6-yl)-amide chlorhydrate de l'acide 5-chloro-naphthalène-1-sulfonique
[43] (1,2,3,4-tétrahydro-isoquinolin-6-yl)-amide chlorhydrate de l'acide 1,2-diméthyl-1H-imidazole-4-sulfonique
[44] N-[4-Méthyl-5-(1,2,3,4-tétrahydro-isoquinolin-6-ylsulfamoyl)-thiazol-2-yl]-acétamide chlorhydrate
[45] (1,2,3,4-tétrahydro-isoquinolin-6-yl)-amide chlorhydrate de l'acide 1,3,5-triméthyl-1H-pyrazole-4-sulfonique
[46] N-[5-(1,2,3,4-tétrahydro-isoquinolin-6-ylsulfamoyl) -naphthalène-1-yl]-acétamide chlorhydrate
[47] (1,2,3,4-tétrahydro-isoquinolin-6-yl) -amide chlorhydrate de l'acide 2-naphthalène-1-yl-éthanesulfonique
[48] (1,2,3,4-tétrahydro-isoquinolin-6-yl)-amide chlorhydrate de l'acide dibenzofuran-2-sulfonique
[49] 2,5-diméthoxy-N-(1,2,3,4-tétrahydro-isoquinolin-6-yl)-benzènesulfonamide chlorhydrate
[50] 5-chloro-2-méthoxy-N-(1,2,3,4-tétrahydro-isoquinolin-6-yl)-benzène sulfonamide chlorhydrate
[51] 2,5-diméthyl-N-(1,2,3,4-tétrahydro-isoquinolin-6-yl)-benzènesulfonamide chlorhydrate
[52] 2-fluoro-5-méthyl-N-(1,2,3,4-tétrahydro-isoquinolin-6-yl)-benzène sulfonamide chlorhydrate
[53] (2-méthyl-1,2,3,4-tétrahydro-isoquinolin-7-yl)-amide chlorhydrate de l'acide 6-chloro-imidazo[2,1-b]thiazole-5-sulfonique
[54] (1,2,3,4-tétrahydro-isoquinolin-7-yl)-amide chlorhydrate de l'acide 6-chloro-imidazo[2,1-b]thiazole-5-sulfonique
[55] (1,2,3,4-tétrahydro-isoquinolin-7-yl)-amide chlorhydrate de l'acide 4-méthyl-naphthalène-1-sulfonique
[56] (2-méthyl-1,2,3,4-tétrahydro isoquinolin-7-yl)-amide chlorhydrate de l'acide 4-méthyl-naphthalène-1-sulfonique
[57] (2-méthyl-1,2,3,4-tétrahydro isoquinolin-7-yl)-amide chlorhydrate de l'acide 5-chloro-naphthalène-2-sulfonique
[58] (1,2,3,4-tétrahydro-isoquinolin-7-yl)-amide chlorhydrate de l'acide 5-chloro-naphthalène-2-sulfonique
[59] (1,2,3,4-tétrahydro-isoquinolin-7-yl)-amide chlorhydrate de l'acide 4-méthyl-3,4-dihydro-2H-benzo[1,4]oxazine-7-sulfonique,
[60] (1,2,3,4-tétrahydro-isoquinolin-7-yl)-amide chlorhydrate 5-Chloro-3-méthyl-benzo[b]thiophène-2-sulfonique
[61] (1,2,3,4-tétrahydro-isoquinolin-7-yl)-amide chlorhydrate de l'acide naphthalène-1-sulfonique
[63] (1,2,3,4-tétrahydro-isoquinolin-7-yl)-amide chlorhydrate de l'acide 2,3-dihydro-benzo[1,4]dioxine-6-sulfonique
[64] (1,2,3,4-tétrahydro-isoquinolin-7-yl)-amide chlorhydrate de l'acide 5-fluoro-3-méthyl-benzo[b]thiophène-2-sulfonique
[65] (1,2,3,4-tétra hydro-isoquinolin-7-yl)-amide chlorhydrate de l'acide 3-méthyl-quinoline-8-sulfonique
[66] (1,2,3,4-tétrahydro-isoquinolin-7-yl)- amide chlorhydrate de l'acide naphthalène-2-sulfonique
[67] (1,2,3,4-tétrahydro-isoquinolin-7-yl)-amide chlorhydrate de l'acide Benzo[1,2,5]thiadiazole-4-sulfonique
[68] (1,2,3,4-tétrahydro-isoquinolin-7-yl)-amide chlorhydrate de l'acide 1,4-diméthyl-2,3-dioxo-1,2,3,4-tétrahydro-quino xaline-6-sulfonique
[69] (1,2,3,4-tétrahydro-isoquinolin-7-yl)-amide chlorhydrate de l'acide benzo[b]thiophène-2-sulfonique
[71] (1,2,3,4-tétrahydro-isoquinolin-7-yl)-amide chlorhydrate de l'acide 7-chloro-benzo[1,2,5] oxadiazole-4-sulfonique
[72] (1,2,3,4-tétrahydro-isoquinolin-7-yl)-amide chlorhydrate de l'acide benzo[1,2,5]oxadiazole-4-sulfonique
[73] (1,2,3,4-tétrahydro-isoquinolin-7-yl)-amide chlorhydrate de l'acide 2-oxo-2,3-dihydro-benzothiazole-6-sulfonique
[74] (1,2,3,4-tétrahydro-isoquinolin-7-yl)-amide chlorhydrate de l'acide 2-oxo-2,3-dihydro-benzo oxazole-6-sulfonique
[76] (1,2,3,4-tétrahydro-isoquinolin-7-yl)-amide chlorhydrate de l'acide 5-méthylbenzo [1,2,5]thiadiazole-4-sulfonique
[77] (1,2,3,4-tétrahydro-isoquinolin-7-yl)-amide chlorhydrate de l'acide 7-méthyl-benzo[1,2,5] thiadiazole-4-sulfonique
[78] (1,2,3,4-tétrahydro-isoquinolin- 7-yl)-amide chlorhydrate de l'acide benzo[b]thiophène-3-sulfonique
[79] (1,2,3,4-tétrahydro-isoquinolin-7-yl)- amide chlorhydrate de l'acide isoquinoline-5-sulfonique
[80] (1,2,3,4-tétrahydro-isoquinolin-7-yl)-amide chlorhydrate de l'acide 4-fluoro-naphthalène-1-sulfonique
[81] (1,2,3,4-tétrahydro-isoquinolin-7-yl) -amide chlorhydrate de l'acide 4-chloro-naphthalène-1-sulfonique
[82] (1,2,3,4-tétrahydro-isoquinolin-7-yl)-amide chlorhydrate de l'acide 2,2-diméthyl-chroman-6-sulfonique
[83] (1,2,3,4-tétrahydro-isoquinolin-7-yl)-amide chlorhydrate de l'acide 5-diméthylamino-naphthalène-1-sulfonique
[84] (1,2,3,4-tétrahydro-isoquinolin-7-yl)- amide chlorhydrate de l'acide 2-oxo-2H-chromène-6-sulfonique
[85] (1,2,3,4-tétrahydro-isoquinolin-7-yl)-amide chlorhydrate de l'acide 2-méthyl-benzothiazole-6-sulfonique
[86] 5-éthyl-2-méthoxy-N-(1,2,3,4-tétrahydro-isoquinolin-7-yl)-benzènesulfonamide chlorhydrate
[87] (1,2,3,4-tétrahydro-isoquinolin-7-yl)-amide chlorhydrate de l'acide 6-méthyl-2,4-dioxo-1,2,3,4-tétrahydro-pyrimidine-5-sulfonique
[88] éthyl-(2-éthyl-1,2,3,4-tétrahydro-isoquinolin-5-yl) amide chlorhydrate de l'acide 6-chloro-imidazo[2,1-b]thiazole-5-sulfonique
[89] (2-éthyl-1,2,3,4-tétrahydro-isoquinolin-5-yl)-amide chlorhydrate de l'acide 6-Chloro-imidazo[2,1-b]thiazole-5-sulfonique
[90] (1,2,3,4-tétrahydro-isoquinolin-5-yl)-amide chlorhydrate de l'acide 6-chloro-imidazo[2,1-b]thiazole-5-sulfonique
[91] (1,2,3,4-tétrahydro-isoquinolin-5-yl) -amide chlorhydrate de l'acide 5-méthyl-naphthalène-1-sulfonique
[92] (1,2,3,4-tétrahydro-isoquinolin-5-yl)-amide chlorhydrate de l'acide naphthalène-1-sulfonique
[93] (1,2,3,4-tétrahydro-isoquinolin-5-yl)-amide chlorhydrate de l'acide 5-chloro-naphthalène-2-sulfonique
[94] (1,2,3,4-tétrahydro-isoquinolin-5-yl)-amide chlorhydrate de l'acide 4-méthyl-3,4-dihydro-2H-benzo[1,4]oxazine-7- sulfonique
[95] éthyl-(2-éthyl-1,2,3,4-tétrahydro-isoquinolin-5-yl)-amide chlorhydrate de l'acide 5-chloro-3-méthyl-benzo[b] thiophène-2-sulfonique
[96] (1,2,3,4-tétrahydro-isoquinolin-5-yl)-amide chlorhydrate de l'acide 5-chloro-3-méthyl-benzo[b]thiophène-2-sulfonique
[98] (1,2,3,4-tétrahydro- isoquinolin-5-yl)-amide chlorhydrate de l'acide 2,3-dihydro-benzo[1,4] dioxine-6-sulfonique
[99] (1,2,3,4-tétrahydro-isoquinolin-5-yl)-amide chlorhydrate de l'acide 5-fluoro-3-méthyl-benzo[b] thiophène-2-sulfonique
[100] éthyl-(2-éthyl-1,2,3,4-tétrahydro-isoquinolin-5-yl)-amide chlorhydrate de l'acide 3-méthyl-quinoline-8-sulfonique
[101] (1,2,3,4-tétrahydro-isoquinolin-5-yl) -amide chlorhydrate de l'acide 3-méthyl-quinoline-8-sulfonique
[102] (1,2,3,4-tétrahydro-isoquinolin-5-yl)- amide chlorhydrate de l'acide naphtalène-2-sulfonique
[103] (1,2,3,4-tétrahydro-isoquinolin-5-yl) -amide chlorhydrate de l'acide benzo[1,2,5]thiadiazole-4-sulfonique
[104] (1,2,3,4-tétrahydro-isoquinolin- 5-yl) -amide chlorhydrate de l'acide benzo[b]thiophène-2-sulfonique
[105] (1,2,3,4-tétrahydro- isoquinolin-5-yl)- amide chlorhydrate de l'acide benzo[1,2,5]oxadiazole-4-sulfonique
[106] (1,2,3,4-tétrahydro-isoquinolin-5-yl)-amide chlorhydrate de l'acide 7-chlorobenzo[1,2,5] oxadiazole -4-sulfonique
[107] (1,2,3,4-tétrahydro-isoquinolin-5-yl) -amide chlorhydrate de l'acide 2-oxo-2,3-dihydro-benzooxazole-6-sulfonique
[108] (1,2,3,4-tétrahydro-isoquinolin-5-yl)-amide chlorhydrate de l'acide 5-méthyl-benzo[1,2,5] thiadiazole-4-sulfonique
[109] (1,2,3,4-tétrahydro-isoquinolin-5-yl)-amide chlorhydrate de l'acide 7-méthyl-benzo[1,2,5]thiadiazole-4-sulfonique
[110] (1,2,3,4-tétrahydro-isoquinolin-5-yl)-amide chlorhydrate de l'acide benzo[b]thiophène-3-sulfonique
[111] (1,2,3,4-tétrahydro-isoquinolin-5-yl)- amide dichlorhydrate de l'acide isoquinoline-5-sulfonique
[112] (1,2,3,4-tétrahydro-isoquinolin-5-yl) -amide chlorhydrate de l'acide 4-fluoro-naphthalène-1-sulfonique
[113] (1,2,3,4-tétrahydro-isoquinolin-5-yl)-amide chlorhydrate de l'acide 4-chloro-naphthalène-1-sulfonique
[114] (1,2,3,4-tétrahydro-isoquinolin-5-yl) -amide chlorhydrate de l'acide 2,2-diméthyl-chroman-6-sulfonique
[115] (1,2,3,4-tétrahydro-isoquinolin-5-yl)-amide chlorhydrate de l'acide 5-diméthylamino-naphthalène-1-sulfonique
[116] (1,2,3,4-tétrahydro-isoquinolin-5-yl)-amide chlorhydrate de l'acide 2,2,5,7,8-pentaméthyl-chroman-6-sulfonique
[117] (1,2,3,4-tétrahydro-isoquinolin-5-yl)-amide chlorhydrate de l'acide 2-oxo-2H-chromène-6-sulfonique
[118] 5-éthyl-2-méthoxy-N(1,2,3,4-tétrahydro-isoquinolin-5-yl)-benzènesulfonamide chlorhydrate
[119] N-[4-éthoxy-3-(1,2,3,4-tétrahydro-isoquinolin-5-yl sulfamoyl)-phényl]-acétamide chlorhydrate
[120] 2-méthoxy-5-méthyl-N-(1,2,3,4-tétrahydro-isoquinolin-5-yl)-benzènesulfonamide chlorhydrate
[121] (1,2,3,4-tétrahydro-isoquinolin-5-yl)-amide chlorhydrate de l'acide 5-chloro-naphthalène-1-sulfonique
[122] 2-méthoxy-5-méthyl-N-(1,2,3,4-tétrahydro-isoquinolin-5-yl)-benzènesulfonamide chlorhydrate
[123] (1,2,3,4-tétrahydro-isoquinolin-5-yl)-amide chlorhydrate de l'acide quinoline-8-sulfonique
[124] (1,2,3,4-tétrahydro-isoquinolin-5-yl)-amide chlorhydrate de l'acide dibenzofuran-2-sulfonique
[125] (1,2,3,4-tétrahydro-isoquinolin-8-yl)-amide chlorhydrate de l'acide 6-chloro-imidazo[2,1-b] thiazole-5-sulfonique
[126] 6-chloro-N-(2-méthyl-1,2,3,4-tétrahydroisoquinolin-8-yl)imidazo[2,1-b]thiazole-5-sulfonamide chlorhydrate
[127] (1,2,3,4-tétrahydro-isoquinolin-8-yl)-amide chlorhydrate de l'acide benzo[b]thiophène-2-sulfonique
[128] (2-méthyl-1,2,3,4-tétrahydro-isoquinolin-8-yl)-amide chlorhydrate de l'acide benzo[b]thiophène-2-sulfonique
[129] (2-méthyl-1,2,3,4-tétrahydro-isoquinolin-8-yl)-amide chlorhydrate de l'acide benzo[b]thiophène-3-sulfonique
[130] (1,2,3,4-tétrahydro-isoquinolin-8-yl) -amide chlorhydrate de l'acide benzo[b]thiophène-3-sulfonique
[131] (2-méthyl-1,2,3,4-tétrahydro-isoquinolin-8-yl)-amide chlorhydrate de l'acide 5-chloro-3-méthyl-benzo[b]thiophène-2-sulfonique
[132] (1,2,3,4-tétrahydro-isoquinolin-8-yl) -amide chlorhydrate 5-chloro-3-méthyl-benzo[b]thiophène-2-sulfonique
[133] (1,2,3,4-tétrahydro-isoquinolin-8-yl)- amide chlorhydrate de l'acide naphthalène-2-sulfonique
[134] (2-méthyl-1,2,3,4-tétrahydro-isoquinolin-8-yl)-amide chlorhydrate de l'acide naphthalène-2-sulfonique
[137] (1,2,3,4-tétrahydro-isoquinolin-8-yl)-amide chlorhydrate de l'acide naphthalène-1-sulfonique
[138] (2-méthyl-1,2,3,4-tétrahydro-isoquinolin-8-yl)-amide chlorhydrate de l'acide naphthalène-1-sulfonique
[139] (2-méthyl-1,2,3,4-tétrahydro-isoquinolin-8-yl)-amide chlorhydrate de l'acide 4-méthyl-naphthalène-1-sulfonique
[140] (1,2,3,4-tétrahydro-isoquinolin-8-yl)-amide chlorhydrate de l'acide 4-méthyl-naphthalène-1-sulfonique
[141] (1,2,3,4-tétrahydro-isoquinolin-8-yl)-amide chlorhydrate de l'acide 4-chloro-naphthalène-1-sulfonique
[142] (2-méthyl-1,2,3,4-tétrahydro-isoquinolin-8-yl)-amide chlorhydrate de l'acide 4-chloro-naphthalène-1-sulfonique
[143] (2-méthyl-1,2,3,4-tétrahydro-isoquinolin-8-yl)-amide chlorhydrate de l'acide 5-chloro-naphthalène-1-sulfonique
[144] (1,2,3,4-tétrahydro-isoquinolin-8-yl)-amide chlorhydrate de l'acide 5-chloro-naphthalène-1-sulfonique
[145] (1,2,3,4-tétrahydro-isoquinolin-8-yl)-amide chlorhydrate de l'acide 5-chloro-naphthalène-2-sulfonique
[146] (2-méthyl-1,2,3,4-tétrahydro-isoquinolin-8-yl)-amide chlorhydrate de l'acide 5-chloro-naphthalène-2-sulfonique
[147] (2-méthyl-1,2,3,4-tétrahydro-isoquinolin-8-yl)-amide chlorhydrate de l'acide 4-méthyl-3,4-dihydro-2H-benzo[1,4]oxazine-7-sulfonique
[148] (1,2,3,4-tétrahydro-isoquinolin-8-yl)-amide chlorhydrate de l'acide 4-méthyl-3,4-dihydro-2H-benzo[1,4]oxazine-7-sulfonique
[149] 5-éthyl-2-méthoxy-N-(1,2,3,4-tétrahydro-isoquinolin-8-yl)-benzènesulfonamide chlorhydrate
[150] 5-éthyl-2-méthoxy-N-(2-méthyl-1,2,3,4-tétrahydro-isoquinolin-8-yl)-benzène sulfonamide chlorhydrate
[151] (2-méthyl-1,2,3,4-tétrahydro-isoquinolin-8-yl)-amide chlorhydrate de l'acide 5-méthyl-benzo[1,2,5] thiadiazole-4-sulfonique
[152] (1,2,3,4-tétrahydro-isoquinolin-8-yl)-amide chlorhydrate de l'acide 5-méthyl-benzo[1,2,5] thiadiazole-4-sulfonique
[153] (1,2,3,4-tétrahydro-isoquinolin-6-yl)- amide et de l'acide naphthalène-2-sulfonique
[154] (1,2,3,4-tétrahydro-isoquinolin-7-yl)-amide chlorhydrate de l'acide 5-chloro-naphthalène-1-sulfonique
[155] (2-cyclopropanecarbonyl-1,2,3,4-tétrahydro-isoquinolin-6-yl)-amide de l'acide naphthalène-2-sulfonique
[156] (2-cyclopropylméthyl-1,2,3,4-tétrahydro-isoquinolin-6-yl)-amide chlorhydrate de l'acide naphthalène-2-sulfonique
[157] (2-cyclopropanecarbonyl-1,2,3,4-tétrahydro-isoquinolin-6-yl)-amide de l'acide 6-chloro-imidazo[2,1-b]thiazole-5-sulfonique et
[158] (2-cyclopropylméthyl-1,2,3,4-tétrahydro-isoquinolin-6-yl)-amide chlorhydrate de l'acide 6-chloro-imidazo[2,1-b] thiazole-5-sulfonique
optionnellement sous la forme d'un de ses stéréoisomères, de préférence énantiomères ou diastéréomères, un racémate, ou sous la forme d'un mélange d'au moins deux de ses stéréoisomères, de préférence énantiomères et/ou diastéréomères, selon n'importe quel rapport de mélange, ou un sel correspondant de celui-ci, ou un solvate correspondant de celui-ci ou une base correspondante de celui-ci.

8. Procédé de préparation d'un composé selon l'une ou plusieurs des revendications 1 à 7, **caractérisé en ce qu'**au moins un composé de la formule générale IVh 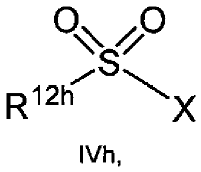 où R^{12h} a la signification selon l'une ou plusieurs des revendications 1 à 7, et X représente un groupe mobile, de préférence un atome d'halogène, particulièrement de préférence un atome de chlore, est amené à réagir avec au moins un composé de la formule générale VhA, 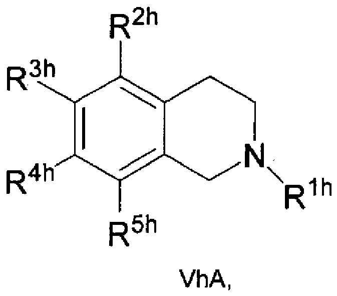 où R^{1h} à R^{5h} ont la signification selon l'une ou plusieurs des revendications 1 à 7, à condition qu'au moins un substituant du groupe consistant en R²ⁿ, R^{3h}, R^{4h} et R^{5h} représente un fragment -N(H)(R^{11h}), où R^{11h} a la signification selon l'une ou plusieurs des revendications 1 à 7, ou un dérivé protégé de celui-ci, dans un milieu de réaction, de préférence en présence d'au moins une base.

9. Médicament comprenant au moins un composé de tétrahydroisoquinoléine substitué de la formule générale 1h selon une ou plusieurs des revendications 1 à 7, optionnellement sous forme d'un de ses stéréoisomères, de préférence énantiomères ou déastéréomères, un racémate ou sous forme d'un mélange d'au moins deux de ses stéréoisomères, de préférence énantiomères et/ou diastéréomères, selon n'importe quel rapport de mélange, ou un sel de celui-ci ou un solvate correspondant de celui-ci et optionnellement au moins un agent auxiliaire physiologiquement acceptable.

10. Médicament selon la revendication 9 pour la prophylaxie et/ou le traitement d'un trouble ou d'une maladie liée à l'ingestion d'aliments, de préférence pour la régulation de l'appétit, pour la conservation, l'augmentation ou la réduction du poids corporel, pour la prophylaxie et/ou le traitement de l'obésité, boulimie, anorexie, cachexie, diabètes de type II (diabètes sucré non insulino-dépendant), de préférence le diabète de type II qui est provoqué par l'obésité; pour la prophylaxie et/ou le traitement d'accidents cérébraux vasculaires, migraine, traumatisme cranien; épilepsie, syndrome du côlon irritable, syndrome du côlon irritable; vomissements; vertigo; troubles du système central nerveux; anxiété; attaques de panique; dépression; troubles bipolaires; trouble obsessionnel-compulsif; troubles cognitifs; dysfonctionnement cognitif associé à des maladies psychiatriques; troubles de la mémoire; démence sénile; troubles de l'humeur; troubles du sommeil; psychose; troubles neurodégénératifs, de préférence sélectionnés dans le groupe consistant en Morbus Alzheimer, Morbus Parkinson, Morbus Huntington et Sclérose Multiple; schizophrénie; amnésie; autisme; dysfonctionnement sexuel; troubles de motilité gastrique; troubles du rythme circadien; hypoxie chronique intermittente; convulsions; ou trouble d'hyperactivité (ADHD, trouble de déficit d'attention/hyperactivité); pour l'amélioration de la cognition (amélioration cognitive) ou mémoire cognitive (amélioration de la mémoire cognitive); pour la prophylaxie et/ou le traitement de la toxicomanie et/ou du sevrage de la drogue; pour la prophylaxie et/ou le traitement de la dépendance à l'alcool et/ou le sevrage, pour la prophylaxie et/ou le traitement de la dépendance à la nicotine et/ou le sevrage.

11. Utilisation d'au moins un d'un composé de tétrahydroisoquinoléine substitué de la formule générale Ih selon l'une ou plusieurs des revendications 1 à 7 pour la fabrication d'un médicament pour la prophylaxie et/ou le traitement d'un trouble ou d'une maladie liée à l'ingestion d'aliments, de préférence pour la régulation de l'appétit, pour la conservation, l'augmentation ou la réduction du poids corporel, pour la prophylaxie et/ou le traitement de l'obésité, boulimie, anorexie, cachexie, diabètes de type II (diabètes sucré non insulino-dépendant), de préférence le diabète de type II qui est provoqué par l'obésité; pour la prophylaxie et/ou le traitement d'accidents cérébraux vasculaires, migraine, traumatisme cranien; épilepsie, syndrome de côlon irritable, syndrome du côlon irritable; vomissements; vertigo; troubles du système nerveux central; anxiété; attaques de panique; dépression; troubles bipolaires; troubles obsessionnels-compulsifs; troubles cognitifs; dysfonctionnement cognitif associé à des maladies psychiatriques; troubles de la mémoire; démence sénile; troubles de l'humeur; troubles du sommeil; psychose; troubles neurodégénératifs, de préférence sélectionnés dans le groupe consistant en Morbus Alzheimer, Morbus Parkinson, Morbus Huntington et Sclérose Multiple; schizophrénie; amnésie; autisme; dysfonctionnement sexuel; troubles de motilité gastrique; troubles du rythme circadien; hypoxie chronique intermittente; convulsions; ou trouble d'hyperactivité (ADHD, trouble de déficit d'attention/hyperactivité); pour l'amélioration de la cognition (amélioration cognitive) ou mémoire cognitive (amélioration de la mémoire cognitive); pour la prophylaxie et/ou le traitement de la toxicomanie et/ou du sevrage de la drogue; pour la prophylaxie et/ou le traitement de la dépendance à l'alcool et/ou le sevrage, pour la prophylaxie et/ou le traitement de la dépendance à la nicotine et/ou le sevrage.

12. Composé de tétrahydroisoquinoléine substitué de la formule générale Ig. 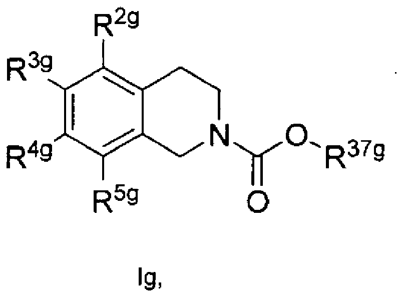 où
R^{1g} représente un atome d'hydrogène; un radical sélectionné dans le groupe consistant en méthyle, éthyle, n-propyle, isopropyle, n-butyle, sec-butyle, isobutyle, tert-butyle, n-pentyle, n-hexyle, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂, -CH₂-CH₂-N(C₂H₅)₂, -CH₂-CH₂-NH-C₂H₅, -CH₂-CH₂-CH₂-NH-CH₃₎, -CH₂-CH₂-CH₂-N (CH₃)₂, -CH₂-CH₂-CH₂-N (C₂H₅)₂ et -CH₂-CH₂-CH₂-NH-C₂H₅; ou un radical (hétéro)cycloaliphatique sélectionné dans le groupe consistant en imidazolidinyle, aziridinyle, azétidinyle, pyrrolidinyle, pipéridinyle, morpholinyle, thiomorpholinyle, pipérazinyle, pyrazolidinyle et azépanyle, qui peut être lié par un groupe via a -(CH₂)₁, ₂ ou ₃⁻ et qui peut être non substitué ou optionnellement substitué par 1, 2, 3, 4 or 5 substituant(s) indépendamment sélectionnés dans le groupe consistant en oxo (=0), thioxo (=S), méthyle, éthyle, n-propyle, isopropyle, n-butyle, tert-butyle, sec-butyle, isobutyle, -C(=O)-CH₃, -C(=O)-C₂H₅, -C(=O)-CH₂-CH₂-CH₃, -C (=O) -CH (CH₃)₂, -C (=O) -C (CH₃)₃, -C (=O) -NH₂, -C(=O)-NH-CH₃, -C(=O) - NH-C₂H₅, -C(=O)-N(CH₃)₂, -C(=O)-N(C₂H₅)₂ et -S(=O)₂-CH₃;
R^{2g}, R^{3g}, R^{4g} et R^{5g}, indépendamment les uns des autres, représentent chacun un atome d'hydrogène; F, Cl, Br, I, -NO₂; -NH₂; -SH; -OH; -CN; -C(=O)-OH; - C(=O)-H; -S(=O)₂-OH; -C(=O)-NH₂; -S(=O)₂-NH₂; -OR⁸⁹; -SR^{9g}; -N(R^{11g})-S(=O)₂-R^{12g}; un radical sélectionné dans le groupe consistant en méthyle, éthyle, n-propyle, isopropyle, n-butyle, sec-butyle, isobutyle, tert-butyle, vinyle, allyle, éthinyle, -CF₃, -CFH₂, -CF₂H, -CH₂-CF₃ and -CF₂-CF₃; ou un radical sélectionné dans le groupe consistant en cyclopropyle, cyclobutyle, cyclopentyle et cyclohexyle;
à condition qu'au moins un des substituants R^{2g}, R^{3g}, R^{4g} et R^{5g} représente un fragment -N(R^{11g})-S(=O)₂-R^{12g};
R^{8g} et R^{9g}, indépendamment l'un de l'autre, représentent chacun un radical sélectionné dans le groupe consistant en méthyle, éthyle, n-propyle, isopropyle, n-butyle, sec-butyle, isobutyle, tert-butyle, CF₃, -CFH₂, -CF₂H, -CH₂-CF₃ et -CF₂-CF₃; un radical (hétéro)cycloaliphatique sélectionné dans le groupe consistant en cyclopropyle, cyclobutyle, cyclopentyle et cyclohexyle; ou un radical aryle ou hétéroaryle sélectionné dans le groupe consistant en phényle, naphtyle, furyl(furanyl), thiényl(thiophényle), pyrrolyle et pyridinyle, qui peut être lié par un groupe -(CH₂)₁, _{2 ou 3} et qui peut être non substitué ou optionnellement substitué par 1, 2 ou 3 substituants indépendamment sélectionnés dans le groupe consistant en méthyle, éthyle, n-propyle, isopropyle, -butyle, tert-butyle, sec-butyle, isobutyle, -OCH₃, -OC₂H₅, -OCH₂-CH₂-CH₃, -O-CH(CH₃)₂, -O-C(CH₃)₃, -S-CH₃, -S-C₂H₅, -S-CH₂-CH₂-CH₃, F, Cl, Br, I, -CN, -CF₃, -OCF₃, -SCF_{3I} -OH, -SH, -NH₂, -NO₂, -CHO, -CF₂H et - CFH₂;
R^{11g} représente un atome d'hydrogène, -S(=O)₂-R^{12g} ou un radical alkyle sélectionné dans le groupe consistant en méthyle, éthyle, n-propyle, isopropyle, n-butyle, sec-butyle, isobutyle et tert-butyle;
R^{12g} représente un radical phényle de la formule générale (Ag), 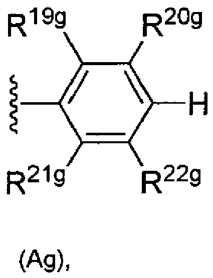 où R^{19g}, R^{20g}, R^{21g} et R^{22g}, indépendamment les uns des autres, représentent chacun un atome d'hydrogène;
F, Cl, Br, I, -NO₂; -NH₂; -SH; -OH; -CN; -C(=O)-OH; -C(=O)-H; -S(=O)₂-OH; -C(=O) - NH₂; -S(=O)₂-NH₂; -C(=O)-R^{23g}; -S(=O)-R^{24g}*;* -S(=O)₂-R^{24g}; -OR^{25g}; -SR^{26g}; -C(=O)-OR R^{27g}; méthyle, éthyle, n-propyle, isopropyle, n-butyle, isobutyle, sec-butyle, tert-butyle, -CF₃, -CF₂H, -CFH₂, -CH₂-CF₃, -CF₂-CF₃, cyclopropyle, cyclobutyle, cyclopentyle et cyclohexyle;
à condition qu'au moins un des substituant R^{19g}, R^{20g}, R^{21g} et R^{22g} soient différents de l'hydrogène;
ou un radical naphtyle, qui peut être non substitué ou optionnellement substitué par 1, 2, 3, 4 ou 5 substituant(s) indépendamment sélectionnés dans le groupe consistant en méthyle, éthyle, n-propyle, isopropyle, n-butyle, tert-butyle, sec-butyle, isobutyle, n-pentyle, -0-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, -NO₂, -CHO, -CF₂H et - CFH₂;
R^{23g} et R^{27g}, indépendamment l'un de l'autre, représentent chacun un radical sélectionné dans le groupe consistant en méthyle, éthyle, n-propyle, isopropyle, n-butyle, sec-butyle, isobutyle, tert-butyle, n-pentyle, n-hexyle, -CF₃, -CFH₂, -CF₂H, -CH₂-CF₃ et -CF₂-CF₃; ou un radical aryle ou hétéroaryle sélectionné dans le groupe consistant en phényle, naphthyle, furyle (furanyle), thiényle (thiophényle), pyrrolyle et pyridinyle, qui peut être lié par un groupe -(CH₂)₁, _{2 ou 3} et qui peut être non substitué ou optionnellement substitué par 1, 2 ou 3 substituants indépendamment sélectionnés dans le groupe consistant en méthyle, éthyle, n-propyle, isopropyle, n-butyle, tert-butyle, sec-butyle, isobutyle, n-pentyle, -0-CH₃, -O-C₂H₅, -O-CH₂-CH₂-CH₃, F, Cl, Br, I, -CN, -CF₃, -OCF₃, -SCF₃, -OH, -SH, -NH₂-NO0₂, -CHO, -CF₂H et -CFH₂;
R^{24g} et R^{26g}, indépendamment l'un de l'autre, représentent chacun un radical sélectionné dans le groupe consistant en méthyle, éthyle, n-propyle, isopropyle, n-butyle, sec-butyle, isobutyle, tert-butyle, n-pentyle, n-hexyle, vinyle, allyle, éthinyle, -CF₃, -CFH₂, -CF₂H, -CH₂CF₃ et -CF₂-CF₃; ou un radical aryle ou hétéroaryle sélectionné dans le groupe consistant en phényle, naphtyle, furyle (furanyle), thiényle (thiophényle), pyrrolyle et pyridinyle, qui peut être non substitué ou optionnellement substitué par 1, 2 ou 3 substituants indépendamment sélectionnés dans le groupe consistant en méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -0-CH(CH₃)₂, -O-C(CH₃)₃=, F, Cl, Br, I, -CN, -CF₃, -OCF₃, -SCF₃, -OH, -SH, -NH₂, -NO₂, -CHO, -CF₂H et -CFH₂;
R^{25g} représente un radical sélectionné dans le groupe consistant en méthyle, éthyle, n-propyle, isopropyle, n-butyle, sec-butyle, isobutyle, tert-butyle, -CF₃, -CFH₂, -CF₂H, -CH₂-CF₃ et -CF₂-CF₃
et R^{37g} représente un radical sélectionné dans le groupe consistant en méthyle, éthyle, n-propyle, isopropyle, n-butyle, sec-butyle, isobutyle, tert-butyle, n-pentyle, n-hexyle, fluorényle, fluorénylméthyle, phényle, benzyle et naphthyle.

13. Composé selon la revendication 12, **caractérisé en ce que**
R^{1g} représente un atome d'hydrogène; ou un radical sélectionné dans le groupe consistant en méthyle, éthyle, n-propyle, n-butyle, n-pentyle et n-hexyle;
R^{2g}, R^{3g}, R^{4g} et R^{5g}, indépendamment les uns des autres, représentent chacun un atome d'hydrogène ou -N(R^{11g})-S(=O)₂-R^{12g}; R^{12g}; à condition qu'au moins un des substituants R^{2g}, R^{3g},
R^{4g} et R^{5g} représentent un fragment -N(R^{11g})-S(=O)₂-R^{12g};
R^{11g} représente un atome d'hydrogène, -S(O)₂-R^{12g} ou un radical alkyle sélectionné dans le groupe consistant méthyle, éthyle et n-propyle;
R^{12g} représente un radical phényle de la formule générale (Ag), 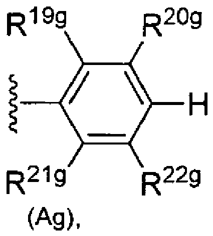 où
R^{19g}, R^{20g}, R^{21g} et R^{22g}, indépendamment les uns des autres, représentent chacun un atome d'hydrogène;
F, Cl, Br, I, -O-CH₃; -O-C₂H₅; -O-CF₃; -O-CFH₂; -O-CF₂H; -O-CH₂-CF₃; -O-CF₂-CF₃; méthyle, éthyle, n-propyle, isopropyle, n-butyle, isobutyle, sec butyle et tert-butyle;
à condition qu'au moins un des substituants R^{19g}, R^{20g}, R^{21g} et R^{22g} soit différent de l'hydrogène;
ou un radical naphtyle qui peut être non substitué ou optionnellement substitué par 1, 2, 3, 4 ou 5 substituants indépendamment sélectionnés dans le groupe consistant en méthyle, éthyle, n-propyle, isopropyle, F, Cl et Br;
et R^{37g} représente un radical sélectionné dans le groupe consistant en méthyle, éthyle, n-propyle, isopropyle, n-butyle, sec-butyle, isobutyle, tert-butyle, n-pentyle, n-hexyle, fluorényle, fluorénylméthyle, phényle, benzyle et naphthyle.

14. Composé selon la revendication 12 ou 13, sélectionné dans le groupe consistant en
[13] tert-butyl ester de l'acide 6-(naphthalène-1-sulfonylamino)-3,4-dihydro-1H-isoquinoline-2- carboxylique,
[15] tert-butyl ester de l'acide 6-(4-méthyl-naphthalène-1-sulfonylamino)-3,4-dihydro-1H- isoquinoline-2-carboxylique,
[16] tert-butyl ester de l'acide 6-(naphthalène-2-sulfonylamino)-3,4-dihydro-1H-isoquinoline-2-carboxylique,
[17] tert-butyl ester de l'acide 6-(2-méthoxy-5-méthyl-benzènesulfonylamino)-3,4-dihydro-1H-isoquinoline-2-carboxylique.
